# EUROPEAN PATENT SPECIFICATION

(11) **EP 4 103 245 B1**
(45) Date of publication and mention of the grant of the patent: **01.04.2026**
(21) Application number: 21708937.4
(22) Date of filing: 10.02.2021
(51) Int. Cl.: A61L 31/16, A61L 29/16

(54) **ADMINISTRATION OF CALCIUM CHANNEL TRPC6 INHIBITORS USING BALLOONS, STENTS OR OTHER MEDICAL DEVICES**
VERABREICHUNG VON CALCIUMKANAL-TRPC6-INHIBITOREN UNTER VERWENDUNG VON BALLONS, STENTS ODER ANDEREN MEDIZINISCHEN VORRICHTUNGEN
ADMINISTRATION D'INHIBITEURS DE TRPC6 DE CANAL DE CALCIUM À L'AIDE DE BALLONNETS, D'ENDOPROTHÈSES OU D'AUTRES DISPOSITIFS MÉDICAUX

(30) Priority: 11.02.2020 EP 20156526
(43) Date of publication of application: 21.12.2022
(73) Proprietor: Klinikum der Technischen Universität München (TUM Klinikum), 81675 München (DE)
(72) Inventor: SCHUNKERT, Heribert, 81925 Munich (DE); KASTRATI, Adnan, 81925 Munich (DE); KESSLER, Thorsten, 80803 Munich (DE)
(74) Representative: Schiweck Weinzierl Koch Patentanwälte Partnerschaft mbB
(86) International application number: PCT/EP2021/053116
(87) International publication number: WO 2021/160625

(56) References cited:
- WO-A1-02/087545
- WO-A1-2019/081637
- WO-A2-2006/076735

## Description

The present invention relates to a medical device suitable for being inserted into the lumen of an anatomic structure of a subject, said medical device comprising means for administration of TRPC6 inhibitor, wherein said means comprises the TRPC6 inhibitor. The present invention further concerns a method of manufacturing a medical device suitable for being inserted into the lumen of an anatomic structure of a subject. Also, the invention relates to a TRPC6 inhibitor for use in the treatment or prevention of a disease associated with neointimal hyperplasia associated with the migration of smooth muscular cells. The invention also relates to a method of treating or preventing a disease associated with neointimal hyperplasia, said method comprising administering a TRPC6 inhibitor to a subject in need thereof. Also envisaged is a method of inhibiting migration of smooth muscle cells. The invention additionally relates to an *in vitro* test kit comprising: (a) smooth muscle cells (SMCs); (b) a surface suitable for SMC cultivation coated with a TRPC6 inhibitor. Also concerned is a pharmaceutical composition comprising a TRPC6 inhibitor and a pharmaceutically acceptable carrier.

### BACKGROUND

Cardiovascular diseases are the leading cause of death worldwide (P. Joseph et al., Reducing the Global Burden of Cardiovascular Disease, Part 1: The Epidemiology and Risk Factors. Circ Res 121, 677-694 2017) and often require interventional treatment including coronary angioplasty and stenting. Such operations frequently lead to sub endothelial scar formation, which in the vascular system is known as neointima (A. Kastrati et al., Restenosis after coronary placement of various stent types. Am J Cardiol 87, 34-39 2001; A. K. Mitra, D. M. Gangahar, D. K. Agrawal, Cellular, molecular and immunological mechanisms in the pathophysiology of vein graft intimal hyperplasia. Immunol Cell Biol 84, 115-124 2006). Neointima thus describes the phenomenon that a new or thickened layer of arterial intima is formed after injury, such as cardiovascular disease. Yet, neointima may also be formed on a prosthesis or in atherosclerosis by migration and proliferation of cells from the media. When the neointima results in the thickened layer of arterial intima, this is also called neointimal hyperplasia.

Neointimal hyperplasia can result in restenosis, which is a re-narrowing of a previously treated vascular lesion, and it is a crucial problem in interventional cardiovascular medicine. Neointima hyperplasia can become critical, when the blood flow is massively reduced in the context of a restenosis. The incidence rate of developing such restenosis ranges between 10% and 40% depending on the type of intervention. Therefore, therapeutic strategies to prevent neointima hyperplasia are of major clinical importance.

Neointima hyperplasia is characterized by an accumulation of inflammatory cells, migration and proliferation of vascular smooth muscle cells (SMCs), and the synthesis of extracellular matrix (ECM) components resulting in neointimal hyperplasia (A. C. Newby, A. B. Zaltsman, Molecular mechanisms in intimal hyperplasia. The Journal of pathology 190, 300-309 (2000). Activation of SMCs involve signaling cascades including the mTOR pathway, which controls cell cycle progression by phosphorylating ribosomal S6 protein kinase (R. J. Shaw, L. C. Cantley, Ras, PI(3)K and mTOR signalling controls tumour cell growth. Nature 441, 424-430 2006).

To reduce the risk of neointima formation after stenting, mTOR inhibitor (e.g. rapamycine) coated stents are regularly used to locally inhibit SMC proliferation (A. Kalra et al., New-Generation Coronary Stents: Current Data and Future Directions. Curr Atheroscler Rep 19, 14 2017). WO 02/087545 A1 discloses the treatment of neointima hyperplasia by administering an anti-proliferative agent, i.e. paclitaxel, using a catheter, balloon or stent.

Therefore, there still exists a need in the art for alternative therapies for treating neointimal hyperplasia.

The solution of the present invention is described in the following, exemplified in the examples, illustrated in the figures and reflected in the claims.

### SUMMARY

The present invention relates to a medical device suitable for being inserted into the lumen of an anatomic structure of a subject, said medical device comprising means for administration of a TRPC6 inhibitor, wherein said means comprises the TRPC6 inhibitor as defined by claims 1-6.

Further, the present invention concerns a method of manufacturing a medical device, as defined by the claims 1-6, suitable for being inserted into the lumen of an anatomic structure of a subject, wherein the method comprises contacting the surface of the medical device with a coating composition comprising a TRPC6 inhibitor.

The present invention also relates to a medical device obtainable or being obtained by a method as disclosed herein.

The present invention also relates to a TRPC6 inhibitor for use in the treatment or prevention of a disease associated with neointimal hyperplasia, wherein the neointimal hyperplasia is associated with the migration of smooth muscular cells, as defined by claim 8.

The present invention also relates to an in-vitro method of inhibiting migration of smooth muscle cells, said method comprising contacting the smooth muscle cells with a TRPC6 inhibitor and determining the migration of the smooth muscle cells compared to the migration of smooth muscle cells in the absence or before the contacting with the TRPC6 inhibitor.

The invention relates to a kit comprising the medical device as disclosed herein.

The present invention also relates to an *in vitro* test kit comprising: (a) smooth muscle cells (SMCs); and (b) a surface suitable for SMC cultivation coated with a TRPC6 inhibitor.

### BRIEF DESCRIPTION OF THE DRAWINGS

The Figures show:

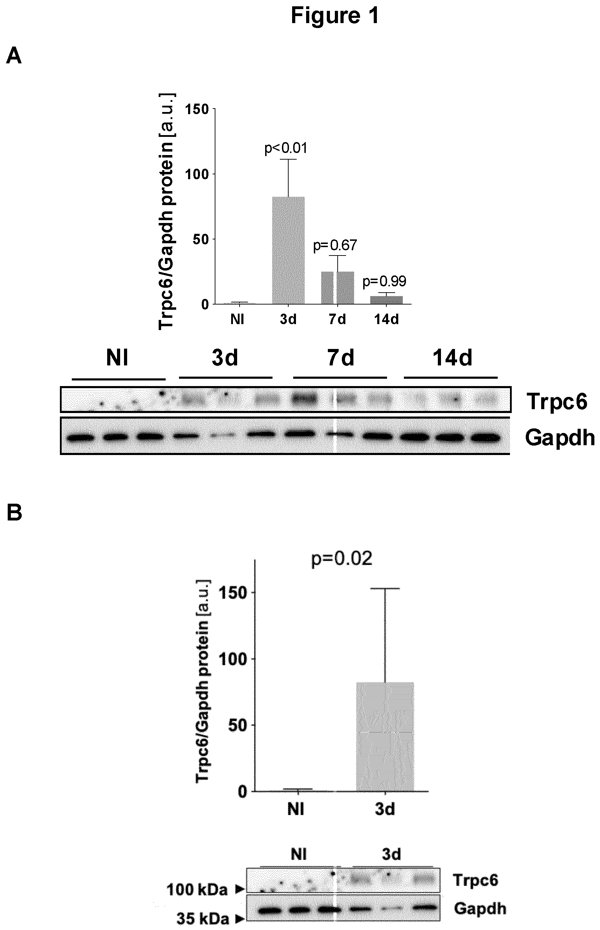

**Figure 1A****:** Expression of Trpc6 in the femoral artery of the mouse after vascular damage. NI, non-injured (undamaged vessel), 3d/7d/14d, 3/7/14 days after vessel damage; a.u., arbitrary units; Gapdh, loading control. **Fig. 1B****:** Expression of Trpc6 in the femoral artery of the mouse after vascular damage according to a second data set. NI, non-injured (undamaged vessel), 3d, 3 days after vessel damage; a.u., arbitrary units; Gapdh, loading control.

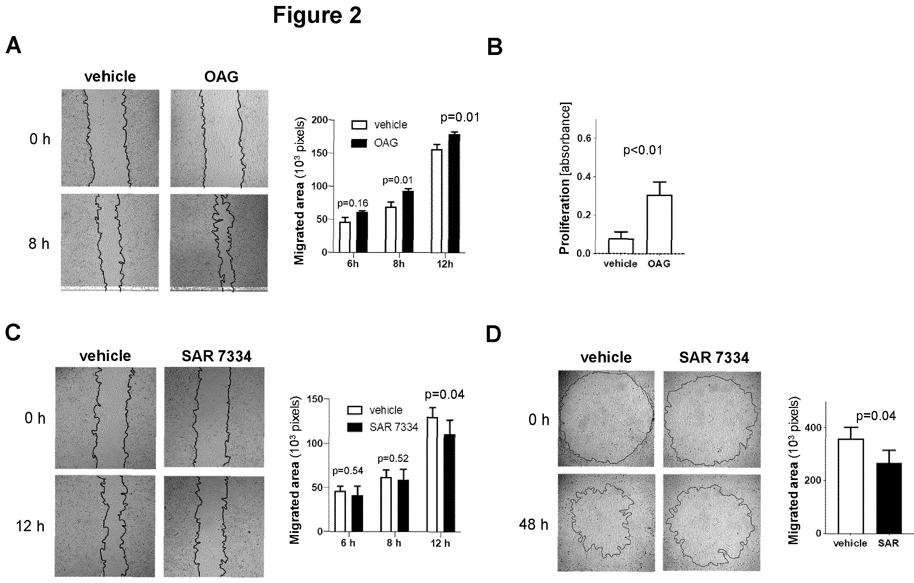

**Figure 2****:** Trpc6 as a novel target in vascular remodeling. **Fig. 2A****:** Scratch wound assay of human aortic SMCs after stimulation with 100 µM 1-oleoyl-2-acetyl-sn-glycerol (OAG), analyzed after six, eight and 12 hours (paired t-test). **Fig. 2B****:** Assessment of human aortic smooth muscle cells (SMC) proliferation using BrdU ELISA in the presence of 100 µM OAG or vehicle (paired t-test). **Fig. 2C****:** Scratch wound assay of human aortic SMCs after treatment with 100 nM SAR 7334, analyzed after six, eight and 12 hours (paired t-test). **Fig. 2D****:** Radius migration assay of human aortic SMC after coating with 100 nM SAR7335 (paired t-test). Data are mean and SEM. *a.u., OAG,* 1-oleoyl-2-acetyl-sn-glycerol.

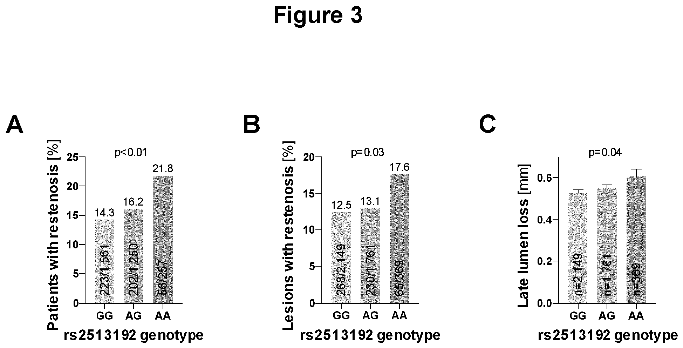

**Figure 3****:** The rs2513192 genotype associated with the expression of TRPC6 in vessels is also associated with the development of restenosis (≥50% lumen reduction) at the patient (A) and lesion (B) levels. Furthermore, the rs2513192 genotype is dose-dependently associated with late lumen loss.

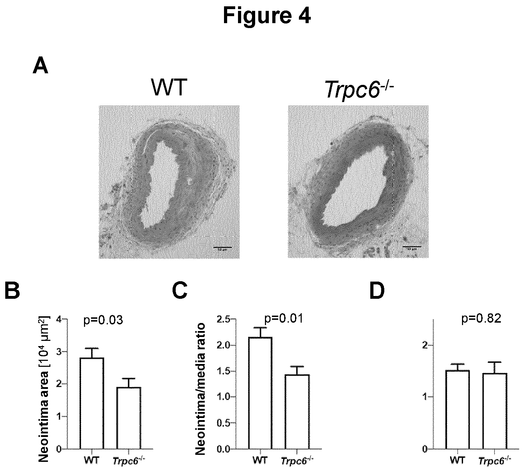

**Figure 4****:** Neointima formation in *Trpc6*^{*-*/*-*} mice. **Fig. 4A****:** Hematoxylin and eosin stain of femoral arteries 28 days after wire-induced vascular injury. **Fig. 4B****-D:** Quantification of neointima area (B), neointima/media ratio (C), and media area (D) in *Trpc6*^{*-*/*-*} and wildtype mice 28 days after vascular injury (unpaired t-test). Data are mean and SEM.

### DETAILED DESCRIPTION

The inventors of the present invention show that migration of vascular smooth muscle cells (VSMCs) is a hallmark of neointima formation. Further, the inventors found that increased Trpc6 (Transient receptor potential cation channel, subfamily C, member 6) protein level and, subsequently, activity is a molecular correlate of the initiation of SMC migration/proliferation and neointima (hyperplasia) formation.

Specifically, the inventors surprisingly found that Trpc6-/- mice display a reduced neointima area (1.90 ± 0.27 vs. 2.82 ± 0.28, p = 0.03) and neointima/media ratio (1.44 ± 0.15 vs. 2.16 ± 0.18, p = 0.01) compared to wildtype mice upon wire injury (Fig. 4). These data indicate that the risk for neointimal hyperplasia/stenosis and/or restenosis is reduced in Trpc6 -/- mice compared to wildtype mice (Fig. 4).

To further confirm data, human aortic vascular smooth muscle cells (VSMC) were cultured in a culture dish coated with SAR7334 - an TRPC6 inhibitor. Afterwards, the local migration was analyzed in comparison to a control cell culture not contacted with the TRPC6 inhibitor. The culture in contact with the TRPC6 inhibitor has a reduced local migration than the control culture. This again indicates that TRPC6 inhibitors reduce migration of VSMCs (Fig. 2).

In a further experiment the inventors performed a scratch wound assay in which a confluent layer of human aortic VSMCs is "injured" by a scratch. The cells were either treated with OAG - an activator of TRPC6 or SAR 7334 - an inhibitor of TRPC6. The treatment with the TRPC6 activator (OAG) leads to an increase in wound reclosure secondary to vascular injury compared to vehicle treatment after eight and 12 hours (vehicle 69.05 ± 7.03 vs. OAG 93.09 ± 3.67 [10³ pixels], p = 0.01 and vehicle 156.93 ± 7.06 vs. OAG 179.14 ± 3.61 [10³ pixels], p = 0.01, respectively; Fig. 2). Further, OAG also enhances proliferation of human VSMCs (vehicle 0.08 ± 0.03 vs. OAG 0.31 ± 0.07 [absorbance units], p < 0.01; Fig. 2).

On the other hand, the TRPC6 inhibitor SAR7334 reduces VSMC migration after 12 hours (111.4 ± 15.5 vs. 130.0 ± 10.6 [10³ pixels], p = 0.04; Fig. 2). The migration 6 and 8 hours after injury is comparable to control levels (Fig. 2).

Therefore, the administration of a TRPC6 inhibitor to previously injured VSMCs keeps the migration capacity of VSMCs at control levels (6 and 8 hours after injury) or even reduces it (12 hours after injury). Thus, this experiment shows that TRPC6 inhibitors promote normal neointimal formation after injury, while reducing the risk of neointimal hyperplasia, which ultimately can lead to a stenosis or restenosis.

That TRPC6 is indeed associated with an increased risk of restenosis after coronary stenting is shown in Fig. 3. Here, carriers of a genetic variant associated with increased TRPC6 gene expression (rs2513192 single nucleotide polymorphism) were analyzed for the occurrence of restenosis by quantitative coronary analysis in 4,279 lesions and 3,068 individuals which underwent coronary stenting (Fig. 3). In this example, restenosis was defined as a diameter reduction of more than 50 percent. Restenosis occurred in 65 of 369 AA lesions compared to 498 of 3,910 GG/AG lesions (17.6 vs. 12.7%, p=0.01; Odds Ratio (OR) 1.46 [95% Confidence Interval (CI) 1.08 - 1.95], p = 0.01; Fig. 7B ) with a gene-dosage effect (GG 12.5 vs. AG 13.1 vs. AA 17.6%, p = 0.03). The rs2513192 genotype was also associated with increased TRPC6 expression and SMC migration (data not shown). Therefore, patients with an increased gene expression of TRPC6 have a higher risk to develop restenosis than patients with normal TRPC6 gene expression.

Furthermore, immunoblotting of proteins secondary to wire injury confirmed highest Trpc6 protein levels at three days after vascular injury with a subsequent reduction at seven and 14 days. Therefore, Trpc6 expression is restricted to the early phase after vascular damage. In undamaged vessels and at later points in time after damage, Trpc6 expression is below the detection limit (Fig.1). Thus, by using TRPC6 inhibitors the inventors thus expect - without wishing to be bound to theory - that side effects on undamaged vessels/cells are negligible in TRPC6 inhibitor therapy.

In summary, the inventors have found that by inhibiting TRPC6 the migration of SMCs can be controlled/decreased. A TRPC6 inhibitor can thus be used in reduction of the occurrence or risk of neointimal hyperplasia/stenosis and/or restenosis.

To the knowledge of the authors to reduce the risk of neointima formation after stenting, mTOR inhibitor (e.g. rapamycine) coated stents are regularly used to locally inhibit SMC proliferation (A. Kalra et al., New-Generation Coronary Stents: Current Data and Future Directions. Curr Atheroscler Rep 19, 14 2017). However, mTOR inhibition is not specific for proliferating SMCs of the neointima, but affects all cells in the surrounding. In addition, current drug-eluting stents reveal certain safety concerns, as due to delayed healing they are linked to an increased risk of thrombosis E. Camenzind, P. G. Steg, W. Wijns, Stent thrombosis late after implantation of first-generation drug-eluting stents: a cause for concern. Circulation 115, 1440-1455; discussion 1455 2007; A. V. Finn et al., Vascular responses to drug eluting stents: importance of delayed healing. Arterioscler Thromb Vasc Biol 27, 1500-1510 2007; T. F. Luscher et al., Drug-eluting stent and coronary thrombosis: biological mechanisms and clinical implications. Circulation 115, 1051-1058 2007). If thrombosis develops it further needs to be counteracted by the prolonged administration of platelet inhibitors.

Compared to the use of mTOR inhibitors when utilizing TRPC6 inhibitors for treating or preventing neointimal hyperplasia/stenosis and/or restenosis a) a different aspect of neointimal hyperplasia/stenosis and/or restenosis formation is targeted (migration of SMCs vs. proliferation of SMCs) and b) lower side effects are to be expected.

Accordingly, the present invention relates to a medical device suitable for being inserted into the lumen of an anatomic structure of a subject, said medical device comprising means for administration of a TRPC6 inhibitor, wherein said means comprises the TRPC6 inhibitor. The term medical device, as used herein is any device intended to be used for medical purposes. The medical device is suitable for being inserted into the lumen of an anatomic structure. In particular, the medical device according to the invention can be *inter alia* be inserted into canals, vessels, passageways, or body cavities. The medical device can for example be a catheter, stent, a balloon, a microcatheter, or a bioabsorbable scaffold.

It is thus envisioned that the medical device can be a catheter.

The term catheter, as used herein, refers to any suitable catheter. Catheters are known to the skilled person. A catheter may be a tubular medical device suitable for insertion into the lumen of an anatomical structure, e.g. esophagus, trachea, urethra, or a blood vessel. Preferably, the catheter is suitable for insertion into the lumen of a blood vessel, more preferably the lumen of an artery. Also, the catheter may be suitable for insertion into the lumen of the esophagus. The catheter may be suitable for insertion into the lumen of the trachea. The catheter may be suitable for insertion into the lumen of the urethra. A catheter may permit injection or withdrawal of fluids, or may only be used in order to keep the lumen of an anatomical structure open.

It is also contemplated that the medical device can be a stent or a balloon.

The term balloon, as used herein refers to any suitable balloon. Balloons are known to the skilled person and *inter alia* described by Li et al. (2019) "Drug-coated balloon versus drug-eluting stent in de novo small coronary vessel disease: A systematic review and meta-analysis." Medicine (Baltimore) ;98(21):e15622. A balloon may be a small bag that can be inflated with e.g. air or gas. The balloon can be inserted into the lumen of an anatomic structure, such as oesophagus, trachea, urethra, blood vessel, and then be inflated with e.g. air or gas in order to keep the lumen open. Preferably, the balloon can be inserted into the lumen of a blood vessel, more preferably the lumen of an artery. Also, the balloon can be inserted into the lumen of the esophagus. The balloon can be inserted into the lumen of the trachea. The balloon can be inserted into the lumen of the urethra. The balloon can also be made of plastic.

The medical device can be a stent.

The term stent, as used herein, refers to any suitable stent. Stents are known to the skilled person and *inter alia* described by Htay and Liu (2005) "Drug eluting stent: a review and update" Vasc Health Risk Manag. 2005;1(4):263-76. Also stents for e.g. the oesophagus are known and are *inter alia* described in Kang (2019) "A Review of Self-Expanding Esophageal Stents for the Palliation Therapy of Inoperable Esophageal Malignancies" BioMed Research International volume 2019, Article ID 9265017, 11 pages. Stents for the trachea and laropharyinx are known and *inter alia* described by Dipak et al. (2005) "Tracheal stent in the treatment of tracheal stenosis" The Medical Journal of Malaysia 60(4):498-501 and stents for the urethra are also known and *inter alia* described by Eisenberg et al. (2007) "Preservation of Lower Urinary Tract Function in Posterior Urethral Stenosis: Selection of Appropriate Patients for Urethral Stents" Journal of Urology, volume 178, issue 6, page: 2456-2461.

The stent may be a short narrow tube. The stent may be of any suitable material. For example, the stent may be of metal (e.g. stainless steel, cobalt, chromium, nickel-titanium shape memory alloy (nitinol), and titanium or alloys comprising or consisting of these; examples of such alloys are titanium- or cobalt/chromium-based alloys) or plastic. The stent may be a microporous stent, e.g. a microporous coronary stent, e.g. made of metal. A stent can also be in the form of a mesh. Stents can be inserted into the lumen of an anatomical structure such as esophagus, trachea, urethra, blood vessel, and can be used to keep a previously blocked lumen open. Preferably, the stent can be inserted into the lumen of a blood vessel, more preferably the lumen of an artery. Also, the stent can be inserted into the lumen of the esophagus. The stent can be inserted into the lumen of the trachea. The stent can be inserted into the lumen of the urethra.

A stent can have virtually any structural pattern that is compatible with a bodily lumen in which it is implanted. For example, a stent can be composed of a pattern or network of circumferential and longitudinally extending interconnecting structural elements or struts. In general, the struts are arranged in patterns, which are designed to contact the lumen walls of a vessel and to maintain vascular patency.

The medical device may also be a biodegradable scaffold. The biodegradable scaffold may be any suitable biodegradable scaffold. Biodegradable scaffolds are temporary scaffold (implants) that may reabsorb over time. Biodegradable scaffolds are known to the skilled person and *inter alia* described in Omar and Kumbhani (2019) "The Current Literature on Bioabsorbable Stents: a Review" Current Atherosclerosis Reports volume 21, Article number: 54. They are indicated for example for improving coronary luminal diameter in patients with ischemic heart disease due to de novo native coronary artery lesions. An example for such biodegradable scaffold is Magmaris, from Biotronik. The biodegradable scaffold can be inserted into the lumen of an anatomical structure such as esophagus, trachea, urethra, blood vessel. Preferably, the biodegradable scaffold can be inserted into the lumen of a blood vessel, more preferably the lumen of an artery. Also, the biodegradable scaffold can be inserted into the lumen of the esophagus. The biodegradable scaffold can be inserted into the lumen of the trachea. The biodegradable scaffold can be inserted into the lumen of the urethra.

The medical device may also be a microcatheter. A microcatheter as used herein may be any suitable microcatheter. Microcatheter are known to the skilled person and *inter alia* described by Karalis et al. (2017) "Microcatheters: A valuable tool in the presence of a challenging coronary anatomy in the setting of acute coronary interventions. Case report and mini review" Cardiovascular Revascularization Medicine; volume 18, issue 6, supplement 1, pages 48-51 and Vemmou et al. (2019) "Recent advances in microcatheter technology for the treatment of chronic total occlusions" Expert Review of Medical Devices , volume 16, 2019, issue 4, pages 267-273. The microcatheter can be inserted into the lumen of an anatomical structure such as esophagus, trachea, urethra, blood vessel. Preferably, the microcatheter can be inserted into the lumen of a blood vessel, more preferably the lumen of an artery. Also, the microcatheter can be inserted into the lumen of the esophagus. The microcatheter can be inserted into the lumen of the trachea. The microcatheter can be inserted into the lumen of the urethra.

Therefore, according to the invention, the medical device as defined herein allows, upon its insertion into the lumen of an anatomical structure, to hold the lumen of said anatomical structure open. In particular, the medical device may allow for passage of air or liquid through the lumen of the anatomical structure.

The term lumen, as used herein, refers to any suitable lumen of an anatomical structure of a subject. For example, the lumen may be the inside space of a tubular anatomical structure. The lumen may be a canal that allows for liquid, food or gas/air passage. In particular, the lumen of the anatomic structure may be the lumen of a blood vessel, the trachea, the esophagus or urethra. Preferably, the lumen is the lumen of a blood vessel, more preferably the lumen of an artery. Also, the lumen may be the lumen of the esophagus. The lumen may be the lumen of the trachea. The lumen may be the lumen of the urethra.

An anatomical structure as used herein may be any anatomical structure. For example, the anatomical structure may be the esophagus, trachea, urethra or blood vessels. Preferably, the anatomic structure is a blood vessel. More preferably, the blood vessel may be an artery. Also, the anatomical structure may be the esophagus. The anatomical structure may be the trachea. The anatomical structure may be the urethra.

The invention envisages that the medical device is inserted in the lumen of an anatomical structure of a subject. A "subject" as used herein may be any suitable subject. Preferably, the term "subject" as used herein refers to a mammal. The subject may be a dog, cat, horse, sheep, goat, cattle or a human subject, preferably a human subject.

Restenosis is the recurrence of stenosis after a procedure. As used herein stenosis may refer to any stenosis.

Stenosis is an abnormal narrowing in a blood vessel or other tubular organs or anatomic structures of a subject. Restenosis is the re-occurrence of stenosis.

Accordingly, the stenosis and/or restenosis may be a stenosis and/or restenosis of blood vessels, esophagus, trachea or urethra. Preferably, the stenosis and/or restenosis is a stenosis and/or restenosis of a blood vessel, more preferably of an artery. Also, the stenosis and/or restenosis may be a stenosis and/or restenosis of the esophagus. The stenosis and/or restenosis may be a stenosis and/or restenosis of the trachea. The stenosis and/or restenosis may be a stenosis and/or restenosis of the urethra. Therefore, the subject may also be a subject that has been afflicted with stenosis and/or restenosis. More specifically, the subject may also be a subject has been or is affected by or at risk of stenosis and/or restenosis of blood vessels, esophagus, trachea or urethra. Preferably, the subject is a subject that has been or is affected by or at risk of stenosis and/or restenosis of a blood vessel, more preferably of an artery. In a merely illustrative and non-limiting example, stenosis and/or restenosis may refer to a lumen reduction of ≥50% of a blood vessel, other tubular organ or anatomic structure (e.g. esophagus, trachea, or urethra) compared to the lumen of the of said blood vessel, other tubular organ or anatomic structure (e.g. esophagus, trachea, or urethra) not affected by stenosis and/or restenosis. The stenosis and/or restenosis may also be a blood vessel stenosis and/or restenosis such as a vascular stenotic lesion or a stenosis and/or restenosis in heart valves. Thus, the subject may also have been or may be afflicted with or be at risk of a vascular stenotic/restenotic lesion or a stenosis and/or restenosis in heart valves.

The vascular stenotic/restenotic lesion can be any vascular stenotic/restenotic lesion. Non-limiting examples include peripheral artery stenosis/restensois, coronary artery stenosis and/or restenosis, carotid artery stenosis and/or restenosis which may predispose to (strokes and transient ischemic episodes) or renal artery stenosis and/or restenosis. Thus, the subject may also be have been or may be afflicted with or be at risk of peripheral artery stenosis and/or restenosis, coronary artery stenosis and/or restenosis or renal artery stenosis and/or restenosis.

The stenosis and/or restenosis may also be a stenosis and/or restenosis in heart valves. The stenosis in heart valves can be any stenosis and/or restenosis in heart valves. Examples of a stenosis in heart valves includes the pulmonary valve stenosis and/or restenosis (thickening of the pulmonary valve, therefore causing narrowing), mitral valve stenosis and/or restenosis (thickening of the mitral valve (of the left heart), therefore causing narrowing), tricuspid valve stenosis and/or restenosis (thickening of the tricuspid valve (of the right heart), therefore causing narrowing), aortic valve stenosis and/or restenosis (thickening of the aortic valve, therefore causing narrowing).

An aortic stenosis and/or restenosis is a narrowing (stenosis) of the aortic valve, the valve between the left ventricle of the heart and the aorta. This narrowing impedes the delivery of blood to the body through the aorta and makes the heart work harder.

Thus, the subject may also have been or may be afflicted with or be at risk of a stenosis of heart valves such as pulmonary valve stenosis and/or restenosis, mitral valve stenosis and/or restenosis, tricuspid valve stenosis and/or restenosis, aortic valve stenosis and/or restenosis, preferably the subject may also be afflicted with or be at risk of aortic valve stenosis and/or restenosis.

Stenosis may also be a stenosis and/or restenosis of the esophagus. Smooth muscle cells also seem to play a role in esophageal stenosis (Jeng et al. (2003) "Restenosis following balloon dilation of benign esophageal stenosis" World J Gastroenterol. 2003 Nov 15; 9(11): 2605-2608 and Oue and Puri (1999) "Smooth Muscle Cell Hypertrophy versus Hyperplasia in Infantile Hypertrophic Pyloric Stenosis" Pediatric Research, volume 45, pages 853-857). Thus, it is plausible that TRPC6 signaling is also involved in SMC migration in esophageal stenosis and restenosis. Esophagal stenosis and/or restenosis is known to the skilled person. Non-limiting examples include pyloric stenosis and/or restenosis or esophageal stricture.

Thus, the subject may also have been or may be afflicted with or be at risk of pyloric stenosis and/or restenosis or esophageal stricture.

As used herein a pyloric stenosis is a narrowing of the opening from the stomach to the first part of the small intestine (the pylorus). Symptoms include projectile vomiting without the presence of bile.

Stenosis and/or restenosis may also be a stenosis and/or restenosis of the trachea. Smooth muscle cells also seem to play a role in tracheal stenosis (Wang et al. (2016) "Paclitaxel Drug-eluting Tracheal Stent Could Reduce Granulation Tissue Formation in a Canine Model" ;129(22): 2708-2713). Thus, it is plausible that TRPC6 signaling is also involved in SMC migration in tracheal stenosis and restenosis. Such stenosis and/or restenosis are known to the skilled person. Non-limiting examples include subglottic stenosis or larygotracheal stenosis and/or restenosis.

Thus, the subject may also have been or may be afflicted with or be at risk of subglottic stenosis or larygotracheal stenosis and/or restenosis. As used herein a subglottic stenosis and/or restenosis is a congenital or acquired narrowing of the subglottic airway. A laryngotracheal stenosis and/or restenosis refers to abnormal narrowing of the central air passageways. This can occur at the level of the larynx, trachea, carina or main bronchi.

Stenosis and/or restenosis may also be a stenosis and/or restenosis of the urethra. Smooth muscle cells also seem to play a role in urethral stenosis (Will et al. (2011) "Paclitaxel Inhibits Ureteral Smooth Muscle Cell Proliferation and Collagen Production in the Absence of Cell Toxicity", pages 335-340. Thus, it is plausible that TRPC6 signaling is also involved in SMC migration in urethral stenosis and restenosis. Urethral stenosis and/or restenosis is known to the skilled person. Non-limiting examples include urethral meatal stenosis and/or restenosis.

As used herein urethral meatal stenosis and/or restenosis may be any urethral meatal stenosis and/or restenosis. It refers to a narrowing (stenosis) of the opening of the urethra at the external meatus thus constricting the opening through which urine leaves the body from the urinary bladder.

Thus, the subject may also have been or be afflicted with or be at risk of urethral meatal stenosis and/or restenosis.

Restenosis can *inter alia* pertain to an artery or other large blood vessel, but also uretha, trachea and esophagus that has become narrowed (suffered from stenosis), received treatment to clear the stenosis e.g. insertion of a suitable stent and subsequently become re-narrowed (restenosis). This is usually restenosis of an artery, or other blood vessel, or possibly a vessel within an organ. Accordingly, restenosis preferably refers to restenosis of a blood vessel, more preferably an artery. Restenosis may also refer to restenosis of the esophagus. Restenosis may also refer to restenosis of the trachea. Restenosis may also refer to restenosis of the urethra. Preferably, the subject is a subject have been or may be afflicted with or at risk of restenosis. The subject may also be a subject that has undergone angioplasty.

In particular, in the context of the invention the restenosis can be caused by neointima hyperplasia following therapeutic procedures such e.g. inserting a medical device such as a stent in the lumen of the anatomical structure affected by stenosis. Procedures frequently used to treat the vascular damage from narrowing and renarrowing (stenosis) of blood vessels are known to the skilled person and include vascular surgery, cardiac surgery, and angioplasty (Corriere et al. (2009) "Restenosis after renal artery angioplasty and stenting: Incidence and risk factors" J Vasc Surg. 50(4): 813-819.e1.

For example, restenosis may occur after angioplasty. In case restenosis occurs after balloon angioplasty it is called a post-angioplasty restenosis (PARS). Thus, the subject may also be a subject at risk of developing or afflicted with PARS.

Thus, restenosis may be an in-stent restenosis or ISR, occurring after vascular, tracheal, eosophagal or urethreal surgery, wherein a medical device such as a stent is employed, and where restenosis occurs afterwards. Thus, the subject may be a subject that has undergone stent therapy.

The presence of restenosis can be measured by methods known to the skilled artisan, for example follow-up imaging such as angiography or duplex ultrasound. These techniques allow identifying proliferating and/or migrating cells forming neointima or visualize the lumen of an anatomic structure. Angiography techniques allow visualizing the lumen of blood vessels or other anatomical structures. This is traditionally done by injecting a radio-opaque contrast agent into the blood vessel and imaging using X-ray based techniques such as fluoroscopy.

Duplex ultrasound employs the Doppler effect to generate imaging of the movement of tissues and body fluids (usually blood), and their relative velocity. This technique can allow therefore to evaluate, for example, whether the lumen of a blood vessel is undergoing restenosis based on the movement and velocity of blood flowing through it. Restenosis can also be individuated or diagnosed based on symptoms, such as recurrent chest pain (angina) or major heart attack (myocardial infarction).

Accordingly, the subject in the context of the invention may be a s subject at risk of or afflicted with vascular injury. Vascular injuries are known to the skilled person and *inter alia* described by Wani et al. (2012) "Vascular Injuries: Trends in Management" Trauma Mon.;17(2):266-9. Vascular injuries may be divided into following groups: spasm, thrombosis, contusion/Intimal flap, laceration/transection, A-V (arteriovenous) fistula, aneurysm and pseudoaneurysm. In particular, in the context of the present invention the vascular injury is an injury causing neointima hyperplasia leading to restenosis, as defined herein.

Accordingly, the subject may be at risk of or is afflicted with neointimal hyperplasia.

The term neointima hyperplasia as used herein is known to the skilled person and inter alia described by Zain et al. (2019) "Neointimal Hyperplasia." StatPearls Publishing; Available from: https://www.ncbi.nlm.nih.gov/books/NBK499893/ and Subbotin (2007) "Analysis of arterial intimal hyperplasia: review and hypothesis" Theoretical Biology and Medical Modelling, 4:41.

Neointimal hyperplasia can result in restenosis and/or stenosis, which is a (re-)narrowing of a previously treated vascular lesion, and it is a crucial problem in interventional cardiovascular medicine. Neointima hyperplasia can become critical, when the blood flow is massively reduced in the context of a restenosis.

Neointima hyperplasia is characterized by an accumulation of inflammatory cells, migration and proliferation of vascular smooth muscle cells (SMCs), and the synthesis of extracellular matrix (ECM) components resulting in neointimal hyperplasia (A. C. Newby, A. B. Zaltsman, Molecular mechanisms in intimal hyperplasia. The Journal of pathology 190, 300-309 (2000). Activation of SMCs involve signaling cascades including the mTOR pathway, which controls cell cycle progression by phosphorylating ribosomal S6 protein kinase (R. J. Shaw, L. C. Cantley, Ras, PI(3)K and mTOR signalling controls tumour cell growth. Nature 441, 424-430 2006).

Neointima can form as a result of vascular surgery such as angioplasty or stent placement. It is discussed to be due to proliferation of smooth muscle cells in the media giving rise to appearance of fused intima and media. Neointimal hyperplasia may result in an increase in the thickness of the lining of a blood vessel e.g. in response to injury or vascular reconstruction such as stent-therapy compared to the thickness of the lining of the blood vessel before injury. Thus, the neointimal hyperplasia may be a thickened layer of intima of the blood vessel than the intima before neointima hyperplasia.

Neointimal hyperplasia may be arterial neointimal hyperplasia secondary to arterial manipulation in endarterectomy or angioplasty or a venous graft associated neointimal hyperplasia secondary to coronary artery bypass grafting or arteriovenous grafting/ fistula formation.

The subject may be a subject having the rs2513192 single nucleotide polymorphism (NCBI dbSNP number rs2513192 *[Homo sapiens]* released July 9, 2019). In particular, the authors found that the A allele was linked to both increased TRPC6 expression and SMC migration. Furthermore, the A allele was also associated with development of restenosis (Fig. 3). As is known to the skilled person in the art A single-nucleotide polymorphism (SNP, pronounced snip) is a DNA sequence variation occurring when a single nucleotide adenine (A), thymine (T), cytosine (C), or guanine (G]) in the genome (or other shared sequence) differs between members of a species or paired chromosomes in an individual. For example, two sequenced DNA fragments from different individuals, AAGCCTA to AAGCTTA, contain a difference in a single nucleotide. In this case we say that there are two alleles: C and T. Almost all common SNPs have only two alleles. In preferred embodiments, the subject carries the A allele.

According to the invention, the medical device comprises means for an administration of a TRPC6 inhibitor.

The mean as used herein may be any suitable means. Such means are also known to the skilled person. The TRPC6 inhibitor can be released from the means at a target site, e.g. a site affected by stenosis and/or restenosis, to administer the TRPC6 inhibitor. For example, the means may be a coating of the medical device. The skilled person knows how coatings can be applied to medical devices, in particular to a surface of the medical device, and also knows how such coatings are prepared. The medical device may be a catheter. The medical device may be a stent. The medical device may be a balloon. The medical device may be a microcatheter. The medical device may be a bioabsorbable scaffold. Preferably, the medical device is a stent or a balloon. In this regard, coating of a medical device such as a stent is *inter alia* described by US 8679520. The coating may be localized at least at the surface of the medical device, which is in contact with the layer of the anatomical structure which is closest to the lumen of the anatomical structure. In this context the layer of the anatomical structure which is closest to the lumen may be the tunica intima, which is is the layer which is closest to the lumen of a blood vessel. In addition or alternatively, the coating may be localized in pores of the medical device.

The coating as used herein may comprise the TRPC6 inhibitor. The TRPC6 inhibitor can be released from the coating at a target site, e.g. a site affected by stenosis and/or restenosis, to administer the TRPC6 inhibitor.

The coating may further comprise a polymer. Representative examples of polymers that can be used to form the coating include ethylene vinyl alcohol copolymer (commonly known by the generic name EVOH or by the trade name EVAL); poly(hydroxyvalerate); poly(L-lactic acid); polycaprolactone; poly(lactide-co-glycolide); poly(hydroxybutyrate); poly(hydroxybutyrate-co-valerate); polydioxanone; polyorthoester; polyanhydride; poly(glycolic acid); poly(D,L-lactic acid); poly(glycolic acid-co-trimethylene carbonate); polyphosphoester; polyphosphoester urethane; poly(amino acids); cyanoacrylates; poly(trimethylene carbonate); poly(iminocarbonate); copoly(ether-esters) (e.g., PEO/PLA); polyalkylene oxalates; polyphosphazenes; biomolecules, such as fibrin, fibrinogen, cellulose, starch, collagen and hyaluronic acid; polyurethanes; silicones; polyesters; polyolefins; polyisobutylene and ethylene-alphaolefin copolymers; acrylic polymers and copolymers; vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile; polyvinyl ketones; polyvinyl aromatics, such as polystyrene; polyvinyl esters, such as polyvinyl acetate; copolymers of vinyl monomers with each other and olefins, such as ethylene-methyl methacrylate copolymers, acrylonitrile-styrene copolymers, ABS resins, and ethylene-vinyl acetate copolymers; polyamides, such as Nylon 66 and polycaprolactam; alkyd resins; polycarbonates; polyoxymethylenes; polyimides; polyethers; epoxy resins; polyurethanes; rayon; rayon-triacetate; cellulose; cellulose acetate; cellulose butyrate; cellulose acetate butyrate; cellophane; cellulose nitrate; cellulose propionate; cellulose ethers; and carboxymethyl cellulose. Furthermore, various combinations of polymers can be employed. The appropriate mixture of polymers can be coordinated with biologically active materials of interest to produce desired effects when coated on a medical device in accordance with the invention.

Alternatively, the coating may be a polymer-free coating.

Additionally or alternatively, the coating may comprise a solvent. Therefore, the coating composition applied on the surface of the medical device may comprise a TRPC6 inhibitor, a polymer, and/or a solvent. In this context, representative examples of solvents can include N,N-dimethylacetamide (DMAC) having the formula CH₃-CO-N(CH₃)₂, N,N-dimethylformamide (DMFA) having the formula H-CO-N(CH₃)₂, tetrahydrofuran (THF) having the formula C₄H₈O, dimethylsulfoxide (DMSO) having the formula (CH₃)₂S-O, or trifluoro acetic anhydride (TFAA) having the formula (CF₃-CO)₂O (see e.g. US7335265-1). Further, examples of useful solvents include tetrahydrofuran, dichloromethane, chloroform, toluene, acetone, isooctane, 1,1,1,-trichloroethane, ethyl acetate, N-methylpyrrolidone (NMP), dimethylsulfoxide (DMSO), dimethylformamide (DMF), and dimethylacetamide (DMAC)), acetone/cyclohexanone solvent, and mixture thereof (see e.g. US7105198 and WO2007130257).

The TRPC6 inhibitor, as used herein, can be any suitable TRPC6 inhibitor. In general, a TRPC6 inhibitor is an inhibitor of the activity of the Transient Receptor Potential C6 (TRPC6) channel. The Transient Receptor Potential C6 (TRPC6) channel belongs to the larger family of TRP ion channels (Desai et al., 2005 Eur J Physiol 451 :11-18; Clapham et al., 2001 Nat Neurosci 2:387-396; Clapham, 2003 Nature 426: 517-524; Clapham et al., 2002 IUPHAR Compendium). TRPC6 is a non-selective calcium permeable cation channel. In addition to calcium ions, TRPC6 channels are permeable to other cations, for example sodium. Thus, TRPC6 channels modulate not only intracellular calcium concentration, but also membrane potential by modulating the flux of cations including calcium and sodium ions. Although non-selective cation channels such as TRPC6 modulate, among other things, calcium ion flux, they are mechanistically distinct from voltage-gated calcium channels. Generally, voltage-gated calcium channels respond to depolarization of the potential difference across the membrane and can open to permit an influx of calcium from the extracellular medium and a rapid increase in intracellular calcium levels or concentrations.

TRPC6 function has been implicated in, among other things, the modulation of myogenic tone. TRPC6 is highly expressed in SMCs, vascular SMCs, cardiomyocytes, pulmonary arteries, the aorta, heart, liver, brain, and kidney.

A TRPC6 inhibitor as used herein, is defined as any suitable inhibitor capable of decreasing or inhibiting the activity of a TRPC6. The TRPC6 may a sequence of SEQ ID NO. 1 or a sequence having 70 %, 75 % 80 %, 85 %, 90 %, 95 %, 98 %, 99 % or 100 % sequence identity to a sequence as shown in SEQ ID NO. 1.

The inhibitor may be a compound/molecule decreasing or abolishing the activity or expression ofTRPC6 or the TRPC6 pathway. The inhibitor may achieve this effect by decreasing or inhibiting the transcription of the gene encoding the TRPC6 and/or decreasing the translation of the mRNA encoding the TRPC6. It can also be that the inhibitor leads to that TRPC6 performs its biochemical function with decreased efficiency in the presence of the inhibitor than in the absence of the inhibitor. Further, it is possible that the inhibitor results in that TRPC6 performs its cellular function with decreased efficiency in the presence of the inhibitor than in the absence of the inhibitor.

The inhibitor can also be an antagonist of the pathway to be inhibited.

In preferred embodiments, the TRPC6 inhibitor acts by binding to the TRPC6 channel.

Methods for testing if a compound/molecule is capable to decrease or block the activity of a TRPC6 are known to the skilled person. For example, an inhibitor of the TRPC6 can be tested by performing a scratch wound assay or radius migration assay as defined in the Examples and wherein the inhibitor has the same or equivalent effect on smooth muscle cell migration as observed with SAR7334.

In accordance with the present invention, the term "identical" or "percent identity" in the context of two or more polypeptide sequences such as SEQ ID NO: 1 refers to two or more sequences or subsequences that are the same, or that have a specified percentage of amino acids that are the same (e.g., at least 85 %, 90 %, 95 %, 96 %, 97 %, 98 % or 99 % identity), when compared and aligned for maximum correspondence over a window of comparison, or over a designated region as measured using a sequence comparison algorithm as known in the art, or by manual alignment and visual inspection. Sequences having, for example, 80 % to 95 % or greater sequence identity are considered to be substantially identical. Such a definition also applies to the complement of a test sequence. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Also available to those having skills in this art are the BLAST and BLAST 2.6 algorithms (Altschul Nucl. Acids Res. 25 (1977), 3389-3402). The BLASTP program for amino acid sequences uses as defaults a word size (W) of 6, an expect threshold of 10, and a comparison of both strands. Furthermore, the BLOSUM62 scoring matrix (Henikoff Proc. Natl. Acad. Sci., USA, 89, (1989), 10915; Henikoff and Henikoff (1992) 'Amino acid substitution matrices from protein blocks.' Proc Natl Acad Sci U S A. 1992 Nov 15;89(22):10915-9) can be used.

For example, BLAST2.6, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments.

In particular, in the context of the present invention a TRPC6 inhibitor is a compound which is able to inhibit the migration of SMCs.

As used herein SMCs (smooth muscle cells) refer to any smooth muscle cell. Smooth muscle cells are known to the skilled person and *inter alia* described by Jaslove and Nelson (2018) "Smooth muscle: a stiff sculptor of epithelial shapes." Philos Trans R Soc Lond B Biol Sci. 2018 Nov 5; 373(1759): 20170318. Smooth muscle is a mesenchymal tissue that surrounds the epithelia of organs including the gut, blood vessels, lungs, bladder, ureter, uterus, oviduct and epididymis. Smooth muscle may be identified as α-smooth muscle actin (α-SMA)-expressing cells. SMCs may contractile or synthetic. Contractile SMCs are elongated, spindleshaped cells, whereas synthetic SMCs are less elongated and have a cobblestone morphology which is referred to as epithelioid or rhomboid.

The skilled person also knows vascular smooth vascular cells as *inter alia* described by Rensen et al. (2007) Regulation and characteristics of vascular smooth muscle cell phenotypic diversity" Neth Heart J.; 15(3): 100-108.

The migration of SMCs can be measured by methods known in the art. Further methods are defined in the examples. For example, the effect a TRPC6 inhibitor has on SMC or VSMC migration can be determined by scratch wound assay or radius migration assay. These assays are known to the skilled person and *inter alia* described by Cory G. Scratch-wound assay Methods Mol. Biol. 2011 769:25-30; William J. Ashbya and Andries Zijlstra Established and Novel Methods of Interrogating Two-Dimensional Cell Migration Integr Biol (Camb). 2012; 4(11): 1338-1350.

The TRPC6 inhibitor may reduce the migration of SMCs to the site at risk of neointima formation or undergoing neointima formation. The TRPC6 inhibitor may additionally or alternatively reduce the migration of SMCs in a scratch wound assay (as explained in the examples) when compared to a scratch wound assay in the absence of the TRPC6 inhibitor.

In this context, the term "reduce" can mean that the amount of total SMCs that migrate, as measured by scratch wound assay or radius migration assay, is reduced by 10 %, 20 %, 30 %, 40 %, 50 %, 60 %, 70%, 80%, 90%, 95 % or by 100% in the presence of the TRPC6 inhibitor as described herein compared to the amount of SMCs that migrate in absence of the TRPC6 inhibitor (Fig. 2).

Accordingly, the TRPC6 inhibitor may act by preventing the migration of SMCs to the site at risk of neointima formation or undergoing neointima formation. Preferably, "prevent" means that, after administration of the TRPC6 inhibitor, about 70 %, 80 %, 90 %, 95 % or 100% of the total SMCs are located in the media portion of the blood vessel wall, i.e do not migrate in the sub endothelium of the blood vessel.

In preferred embodiments, the TRPC6 inhibitor is the compound SAR7334.

According to embodiments of the invention, the TRPC6 inhibitor may have the following formula (I) 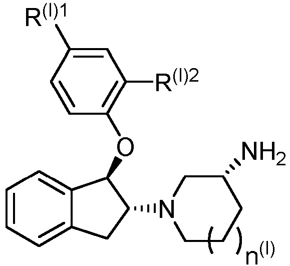 wherein: R^{(I)1} and R^{(I)2} are each independently selected from the group consisting of halogen (preferably F, Cl, or Br), -CN and -NO₂; and n^{(I)} is 0, 1 or 2; or a pharmaceutically acceptable salt, solvate or hydrate thereof.

As used herein and throughout the entire description, the term "pharmaceutically acceptable salt" refers to a salt that retains the desired biological activity of the parent compound and does not impart any undesired toxicological effects (see e.g., Berge, S. M., et al. (1977) J. Pharm. Sci. 66: 1-19). Physiologically acceptable salts of the compounds of the present invention are in particular salts with a nontoxic salt component and preferably are pharmaceutically utilizable salts. They can contain inorganic or organic salt components. Such salts can be formed, for example, from compounds of the present invention which contain an acidic group, for example a 15 carboxylic acid group (HO-CO-) or a sulfonic acid group (H0-S(0)2-) and nontoxic inorganic or organic bases. Suitable bases are, for example, alkali metal compounds or alkaline earth metal compounds, such as sodium hydroxide, potassium hydroxide, sodium carbonate or sodium hydrogencarbonate, or ammonia, organic amino compounds and quaternary ammonium hydroxides. Reactions of compounds of the present invention with bases for the preparation of the salts are in general carried out according to customary procedures in a solvent or diluent. On account of the physiological and chemical stability, advantageous salts of acidic groups are in many cases sodium, potassium, magnesium or calcium salts or ammonium salts which can also carry one or more organic groups on the nitrogen atom. Compounds of the present invention which contain a basic, i.e. protonatable, group, for example an amino group or another basic heterocycle, can be present in the form of their acid addition salts with physiologically acceptable acids, for example as salt with hydrogen chloride, hydrogen bromide, phosphoric acid, sulfuric acid, acetic acid, benzoic acid, methanesulfonic acid, p-toluenesulfonic acid, which in general can be prepared from the compounds of the present invention by reaction with an acid in a solvent or diluent according to customary procedures. As usual, in particular in the case of acid addition salts of a compound containing two or more basic groups, in an obtained salt the ratio of the salt components can deviate upward or downward from the stoichiometric ratio, such as the molar ratio 1:1 or 1:2 in the case of the acid addition salt of a compound of the present invention containing one or two basic groups with a monovalent acid, and vary depending on the applied conditions. The present invention comprises also salts containing the components in a non-stoichiometric ratio, and an indication that an acid addition salt of a compound of the present invention contains an acid in equimolar amount, for example, also allows for a lower or higher amount of acid in the obtained salt, for example about 0.8 or about 1.1 mol of acid per mol of compound of the present invention. If the compounds of the present invention simultaneously contain an acidic and a basic group in the molecule, the invention also includes internal salts (betaines, zwitterions) in addition to the salt forms mentioned. The present invention also comprises all salts of the compounds of the present invention which, because of low physiological tolerability, are not directly suitable for use as a pharmaceutical, but are suitable as intermediates for chemical reactions or for the preparation of physiologically acceptable salts, for example by means of anion exchange or cation exchange. A subject of the present invention also are solvates of the compounds of the present invention and their salts, such as hydrates and adducts with alcohols like (CrC4)alkanols, in particular physiologically acceptable solvates, as well as active metabolites of compounds of the present invention.

As used herein and throughout the entire description, the term "pharmaceutically acceptable" may in particular mean approved by a regulatory agency or other generally recognized pharmacopoeia for use in animals, and more particularly in humans.

In a particularly preferred embodiment, the TRPC6 inhibitor is SAR7334, having the following formula: 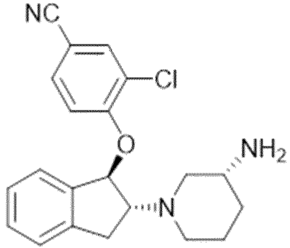 or a pharmaceutically acceptable salt, solvate or hydrate thereof.

Alternatively the compound may have the formula 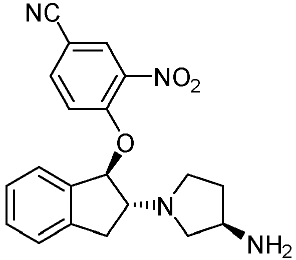 or a pharmaceutically acceptable salt, solvate or hydrate thereof.

In some embodiments, the TRPC6 inhibitor has the following formula (II): 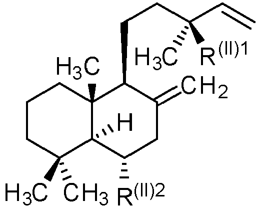 **wherein:** R^{(II)1} and R^{(II)2} are each independently selected from the group consisting of -OH, 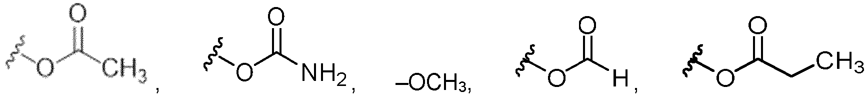 and 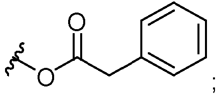 or a pharmaceutically acceptable salt, solvate or hydrate thereof.

Preferably, the TRPC6 inhibitor of formula (II) is selected from the group consisting of larixyl diacetate: 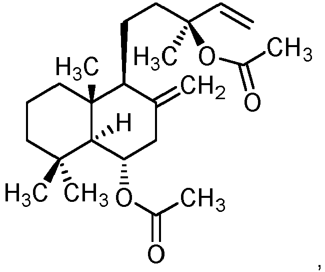
Larixyl carbamate: 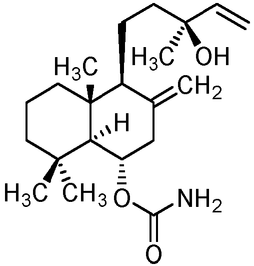
Larixyl methyether: 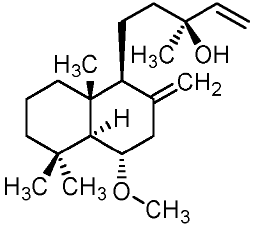
Larixyl formylester: 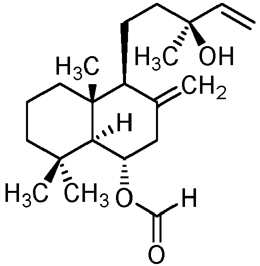
Larixyl monoproprionate: 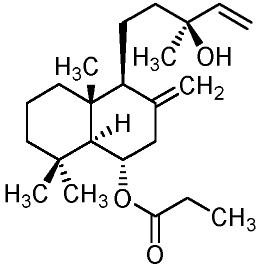
Larixyl diproprionate 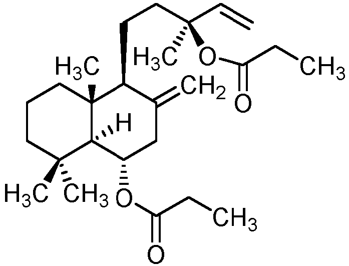
and Larixyl monophenylacetate:

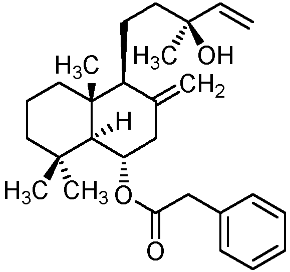

Accordingly, the TRPC6 inhibitor may be larixyl diacetate or a pharmaceutically acceptable salt, solvate or hydrate thereof. The TRPC6 inhibitor may be larixyl carmabate or a pharmaceutically acceptable salt, solvate or hydrate thereof. The TRPC6 inhibitor may be larixyl methylether or a pharmaceutically acceptable salt, solvate or hydrate thereof. The TRPC6 inhibitor may also be larixyl formyester, or a pharmaceutically acceptable salt, solvate or hydrate thereof. Also, the TRPC6 inhibitor may be larixyl monoproprionate, or a pharmaceutically acceptable salt, solvate or hydrate thereof. Furthermore, the TRPC6 inhibitor may be larixyl dipropionate, or a pharmaceutically acceptable salt, solvate or hydrate thereof. Finally, the TRPC6 inhibitor may be larixyl monophenylacetate or a pharmaceutically acceptable salt, solvate or hydrate thereof.

In preferred embodiments, the TRPC6 inhibitor may be larixyl carbamate, having the following formula:

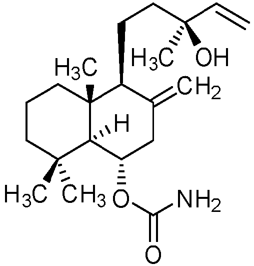

Or a pharmaceutically acceptable salt, solvate or hydrate thereof.

In some embodiments, the TRPC6 inhibitor may have the following formula (III): 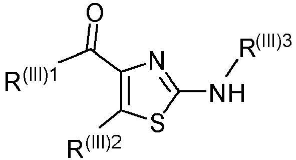 wherein: R^{(III)1} is independently selected from the group consisting of 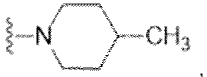 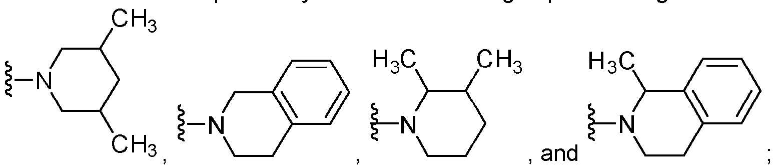 R^{(III)2} is independently selected from the group consisting of -H, -CH₃ and halogen (preferably F, Cl, Br); and R^{(III)3} is independently selected from the group consisting of 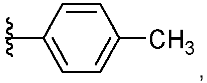 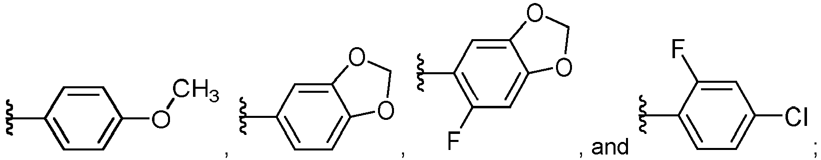 or a pharmaceutically acceptable salt, solvate or hydrate thereof.

In preferred embodiments, the TRPC6 inhibitor is selected from the group consisting of the compounds of the formulas: 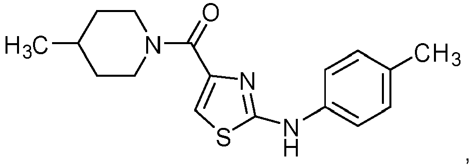 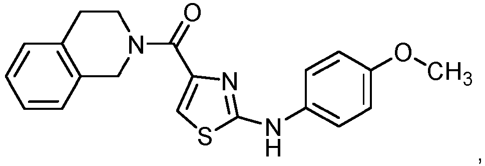 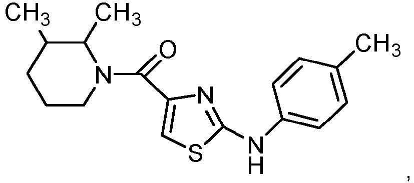 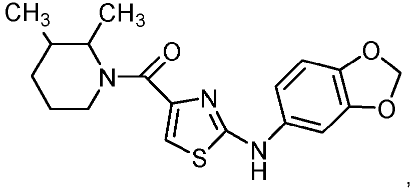 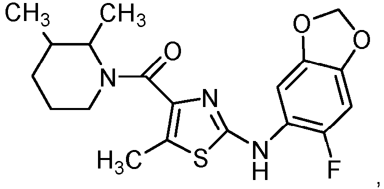 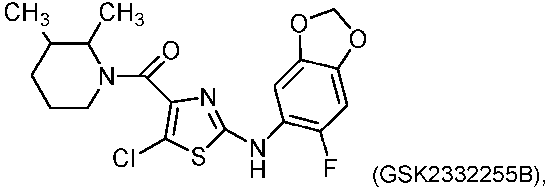 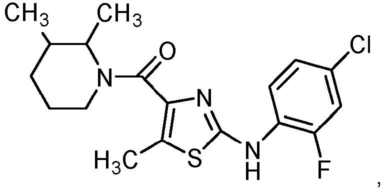 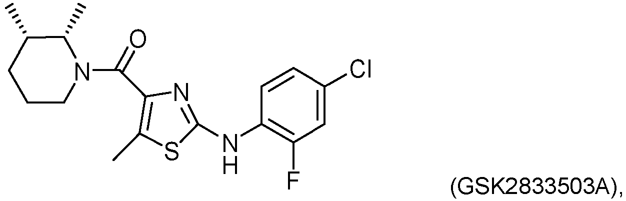 preferably 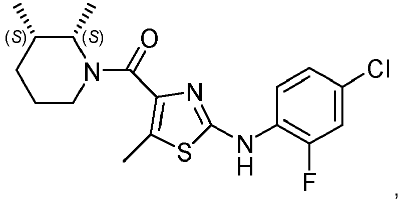 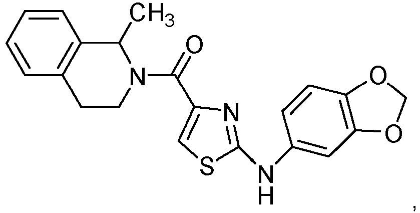 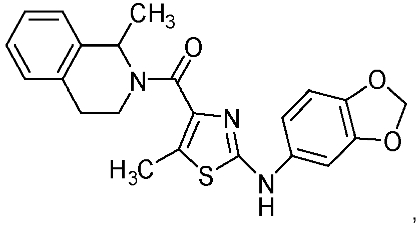 and 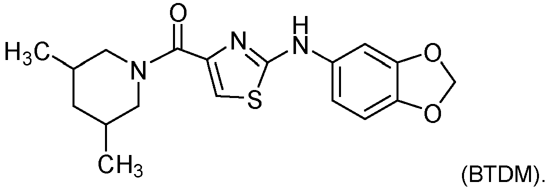 Accordingly, the TRPC6 inhibitor may be 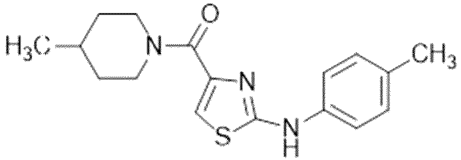 or a pharmaceutically acceptable salt, solvate or hydrate thereof. The TRPC6 inhibitor may be 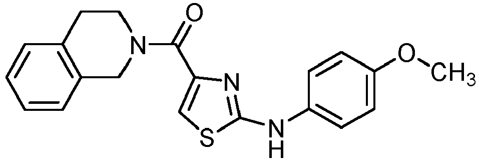 or a pharmaceutically acceptable salt, solvate or hydrate thereof. The TRPC6 inhibitor may be 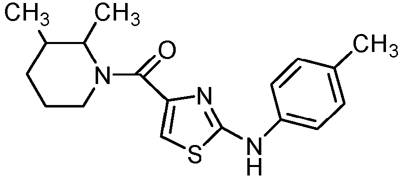 or a pharmaceutically acceptable salt, solvate or hydrate thereof. The TRPC6 inhibitor may be 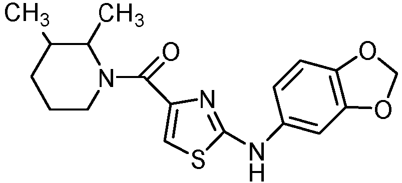 or a pharmaceutically acceptable salt, solvate or hydrate thereof. The TRPC6 inhibitor may be 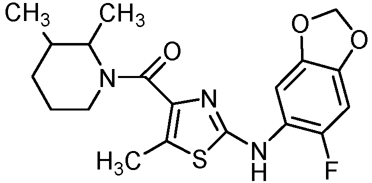 or a pharmaceutically acceptable salt, solvate or hydrate thereof. The TRPC6 inhibitor may be 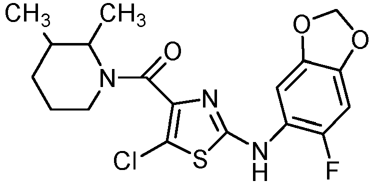 (GSK2332255B) or a pharmaceutically acceptable salt, solvate or hydrate thereof. The TRPC6 inhibitor may be 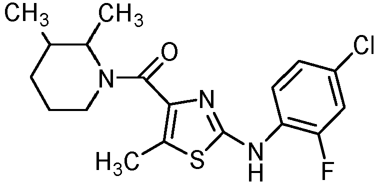 . The TRPC6 inhibitor may be 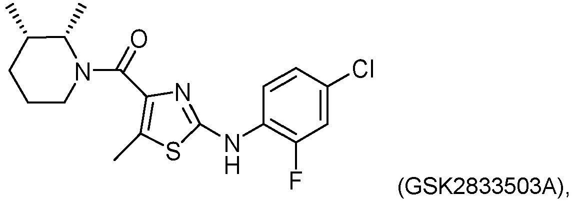 preferably 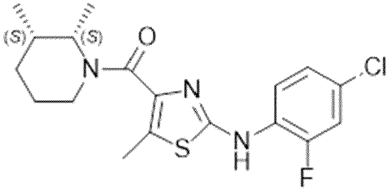 or a pharmaceutically acceptable salt, solvate or hydrate thereof. The TRPC6 inhibitor may be 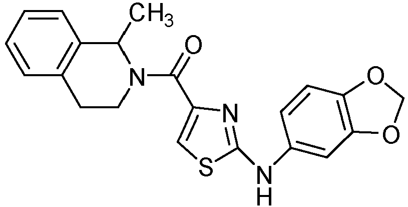 or a pharmaceutically acceptable salt, solvate or hydrate thereof. The TRPC6 inhibitor may be 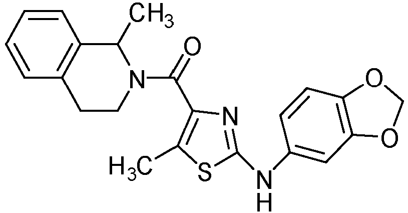 or a pharmaceutically acceptable salt, solvate or hydrate thereof. The TRPC6 inhibitor may be 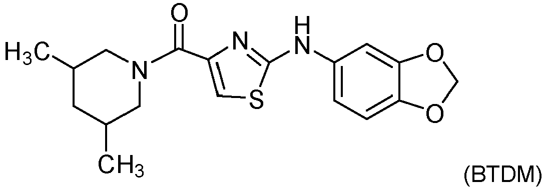 or a pharmaceutically acceptable salt, solvate or hydrate thereof.

In preferred embodiments, the TRPC6 inhibitor is GSK2332255B or a pharmaceutically acceptable salt, solvate or hydrate thereof.

In preferred embodiments, the TRPC6 inhibitor is GSK2833503A or a pharmaceutically acceptable salt, solvate or hydrate thereof.

In preferred embodiments, the TRPC6 inhibitor is BTDM or a pharmaceutically acceptable salt, solvate or hydrate thereof.

In some embodiments, the TRPC6 inhibitor is selected from the group consisting of 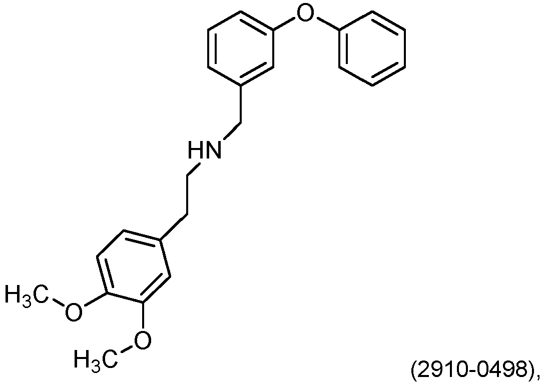 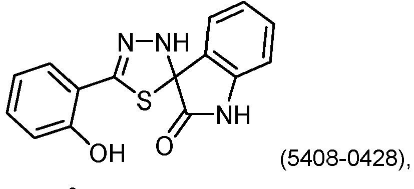 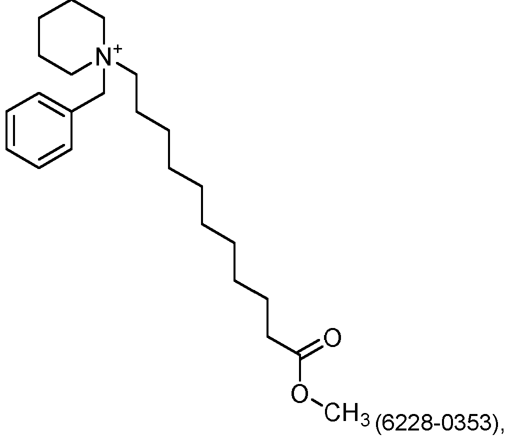 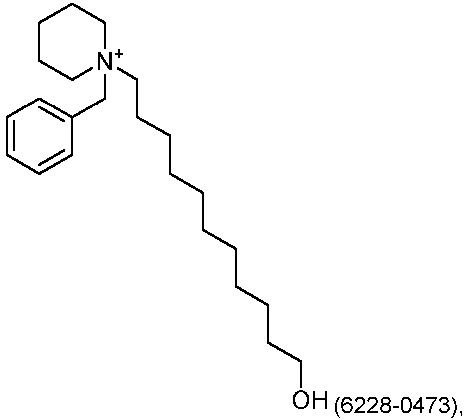 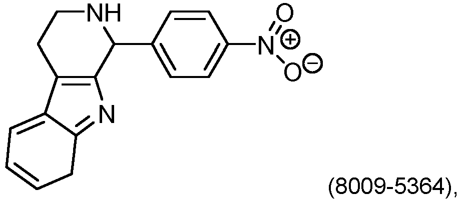 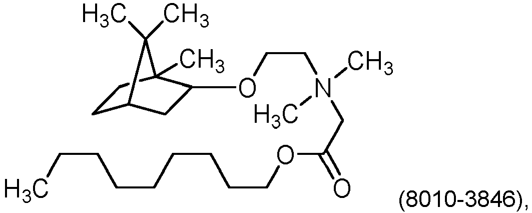 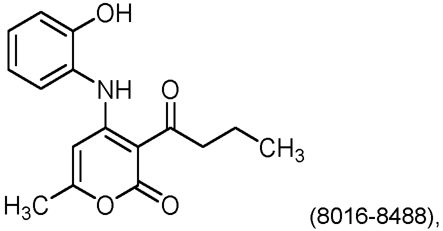 And

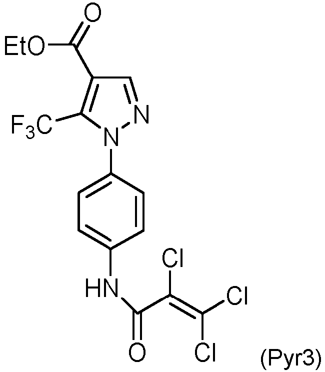

Accordingly, the TRPC6 inhibitor may be 2910-0498, or a pharmaceutically acceptable salt, solvate or hydrate thereof. The TRPC6 inhibitor may also be 5408-0428, or a pharmaceutically acceptable salt, solvate or hydrate thereof. The TRPC6 inhibitor may also be 6228-0353, or a pharmaceutically acceptable salt, solvate or hydrate thereof. The TRPC6 inhibitor may also be 6228-0473, or a pharmaceutically acceptable salt, solvate or hydrate thereof. The TRPC6 inhibitor may also be 8010-3846, or a pharmaceutically acceptable salt, solvate or hydrate thereof. The TRPC6 inhibitor may also be 8016-8488, or a pharmaceutically acceptable salt, solvate or hydrate thereof. The TRPC6 inhibitor may also be Pyr3 or a pharmaceutically acceptable salt, solvate or hydrate thereof. In preferred embodiments, the TRPC6 inhibitor is 8009-5364, or a pharmaceutically acceptable salt, solvate or hydrate thereof.

In some embodiments the TRPC6 inhibitor may have the formula (IV), as disclosed in WO 2019/081637: 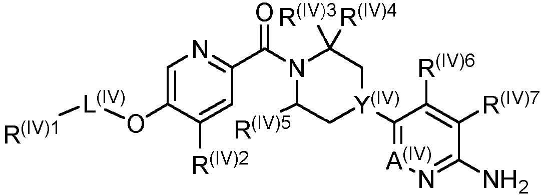 wherein:
L^{(IV)} is absent or is methylene or ethylene;
Y^{(IV)} is CH or N;
A^{(IV)} is CH or N;
R^{(IV)1} is selected from the group consisting of:
   C₁₋₆alkyl optionally substituted with 1 to 3 groups independently selected from the group consisting of halo, C₃₋₆cycloalkyl and OC₃₋₆cycloalkyl;
   phenyl optionally substituted with 1 to 3 groups independently selected from the group consisting of CF₃, halo, C₃₋₆cycloalkyl, OC₃₋₆cycloalkyl, OC₁₋₆alkyl optionally substituted with one to three halo; and
   C₃₋₆cycloalkyl optionally substituted with 1 to 3 groups independently selected from the group consisting of halo and C₁₋₆alkyl optionally substituted with 1 to 3 halo;
R^{(IV)2} is selected from the group consisting of H, C₁₋₆alkyl, OCF₃, C₃₋₆cycloalkyl, OC₁₋₆alkyl; OC₃₋₆cycloalkyl;
R^{(IV)3} is selected from the group consisting of H, C₁₋₆alkyl, C₃₋₆cycloalkyl, OC₃₋₆cycloalkyl; wherein each of the C₁₋₆alkyl, C₃₋₆cycloalkyl, OC₃₋₆cycloalkyl of the R^{(IV)3} group may be optionally substituted with one to three groups each independently selected from the group consisting of halo, OH, OC₁₋₆alkyl, SC₁₋₆alkyl, N(C₁₋₆alkyl)₂; and wherein one to three carbon atoms of the C₁₋₆alkyl of the R^{(IV)3} group may optionally be replaced by one or two moieties selected from the group consisting of NH, (NC₁₋₆alkyl), O, and S;
R^{(IV)4} and R^{(IV)5} are each independently selected from the group consisting of H or C₁₋₆alkyl;
R^{(IV)3} and R^{(IV)4} can together with the atom to which they are attached join to form a 3 to 9-membered carbocyclyl ring which optionally may contain one to three heteroatoms selected from the group consisting of N, O, and S; or
R^{(IV)3} and R^{(IV)5} can together form a 3 to 9-membered bicyclic ring which optionally may contain one to three heteroatoms selected from the group consisting of N, O, and S;
R^{(IV)6} is selected from the group consisting of H, C₁₋₆alkyl, CN, CF₃, OCF₃, C₃₋₆cycloalkyl, OC₁₋₆alkyl, and OC₃₋₆cycloalkyl;
R^{(IV)7} is selected from the group consisting of H and OC₁₋₆alkyl;
   or a pharmaceutically acceptable salt, solvate or hydrate thereof

The term "C1-n-alkyl", wherein n is an integer selected from 2, 3, 4, 5 or 6, preferably 4 or 6, either alone or in combination with another radical denotes an acyclic, saturated, branched or linear hydrocarbon radical with 1 to n C atoms. For example the term C1-5-alkyl embraces the radicals H3C-, H3C-CH2-, H3C-CH2-CH2-, H3C-CH(CH3)-, H3C-CH2-CH2-CH2-, H3C-CH2-CH(CH3)-, H3C-CH(CH3)-CH2-, H3C-C(CH3)2-, H3C-CH2-CH2-CH2-CH2-, H3C-CH2-CH2-CH(CH3)-, H3C-CH2-CH(CH3)-CH2-,H3C-CH(CH3)-CH2-CH2-, H3C-CH2-C(CH3)2-, H3C-C(CH3)2-CH2-, H3C-CH(CH3)-CH(CH3)- and H3C-CH2-CH(CH2CH3)-.

The term "C3-n-cycloalkyl", wherein n is an integer from 4 to n, either alone or in combination with another radical denotes a cyclic, saturated, unbranched hydrocarbon radical with 3 to n C atoms. For example, the term C3-6-cycloalkyl includes cyclopropyl, cyclobutyl, cyclopentyl, and cyclohexyl.

By the term "halo" added to an "alkyl", "alkylene" or "cycloalkyl" group (saturated or unsaturated) is such a alkyl or cycloalkyl group wherein one or more hydrogen atoms are replaced by a halogen atom selected from among fluorine, chlorine or bromine, preferably fluorine and chlorine, particularly preferred is fluorine. Examples include: H2FC-, HF2C-, F3C- .

The term "carbocyclyl" as used either alone or in combination with another radical, means a mono- bi- or tricyclic ring structure consisting of 3 to 9 carbon atoms and optionally a heteroatom selected from the group consisting of N, O, and S. The term "carbocyclyl" refers to fully saturated ring systems and encompasses fused, bridged and spirocyclic systems.

Many of the terms given above may be used repeatedly in the definition of a formula or group and in each case have one of the meanings given above, independently of one another. The compounds of the invention are only those which are contemplated to be 'chemically stable' as will be appreciated by those skilled in the art. For example, a compound which would have a 'dangling valency', or a 'carbanion' are not compounds contemplated by the inventive methods disclosed herein.

Accordingly, the TRPC6 inhibitor, as disclosed in WO 2019/081637, may be:

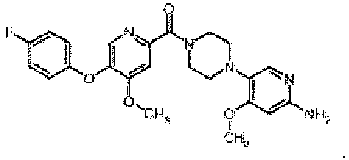

The TRPC6 inhibitor may also be:

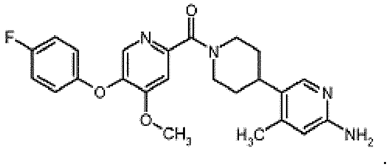

The TRPC6 inhibitor may also be:

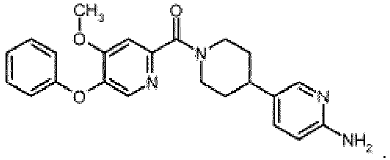

The TRPC6 inhibitor may also be:

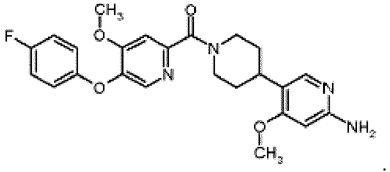

The TRPC6 inhibitor may also be:

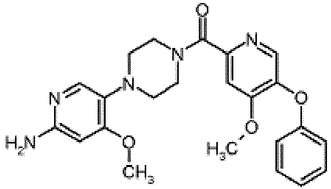

The TRPC6 inhibitor may also be:

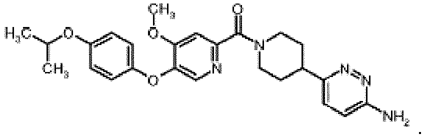

The TRPC6 inhibitor may also be:

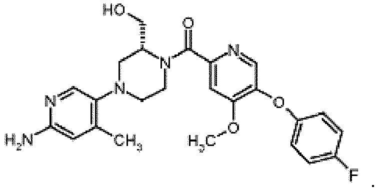

The TRPC6 inhibitor may also be:

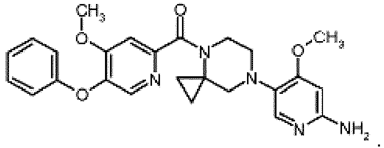

The TRPC6 inhibitor may also be:

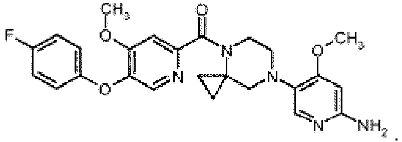

The TRPC6 inhibitor may also be:

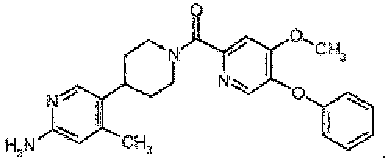

The TRPC6 inhibitor may also be:

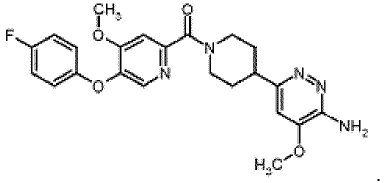

The TRPC6 inhibitor may also be:

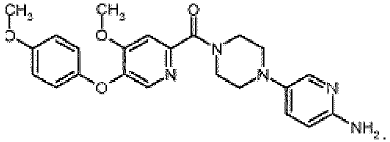

The TRPC6 inhibitor may also be:

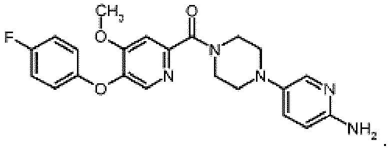

The TRPC6 inhibitor may also be:

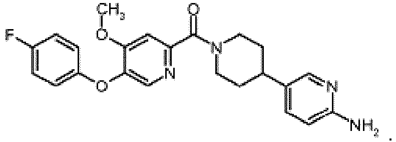

The TRPC6 inhibitor may also be:

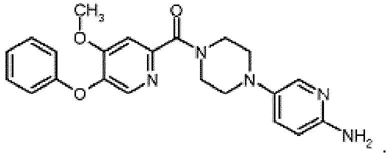

The TRPC6 inhibitor may also be:

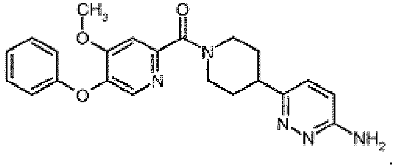

The TRPC6 inhibitor may also be:

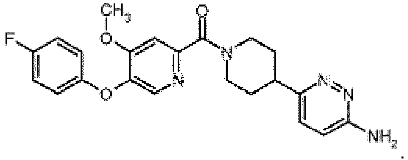

The TRPC6 inhibitor may also be:

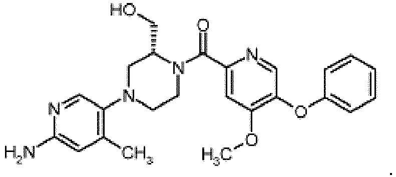

The TRPC6 inhibitor may also be:

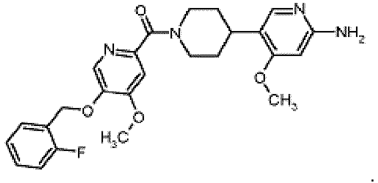

The TRPC6 inhibitor may also be:

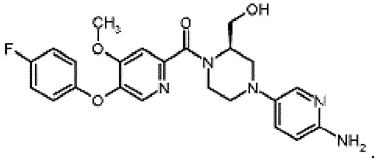

The TRPC6 inhibitor may also be:

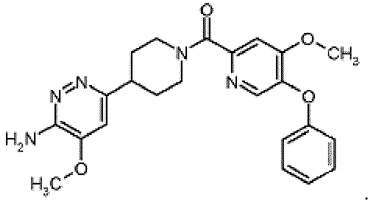

The TRPC6 inhibitor may also be:

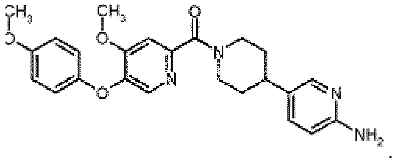

The TRPC6 inhibitor may also be:

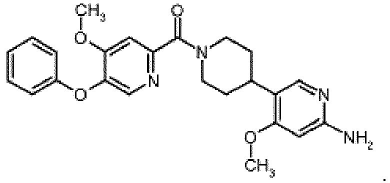

The TRPC6 inhibitor may also be:

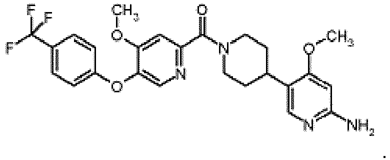

The TRPC6 inhibitor may also be:

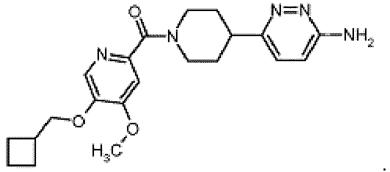

The TRPC6 inhibitor may also be:

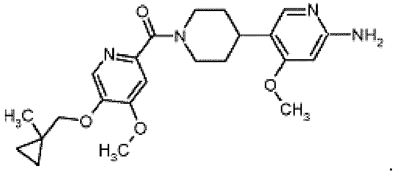

The TRPC6 inhibitor may also be:

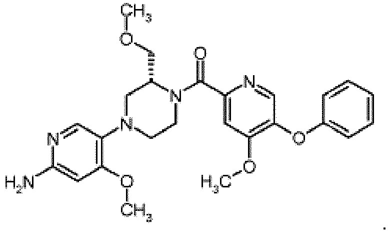

The TRPC6 inhibitor may also be:

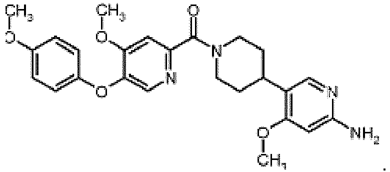

The TRPC6 inhibitor may also be:

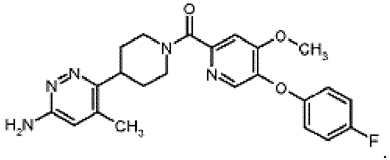

The TRPC6 inhibitor may also be:

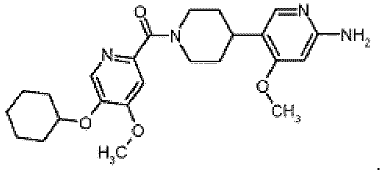

The TRPC6 inhibitor may also be:

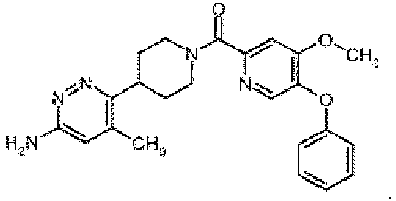

The TRPC6 inhibitor may also be:

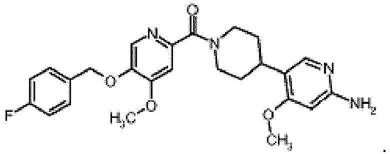

The TRPC6 inhibitor may also be:

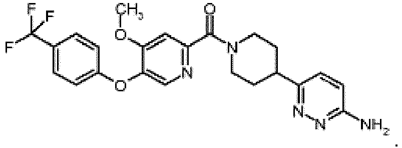

The TRPC6 inhibitor may also be:

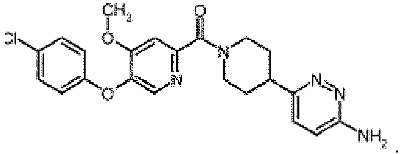

The TRPC6 inhibitor may also be:

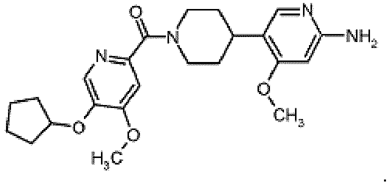

The TRPC6 inhibitor may also be:

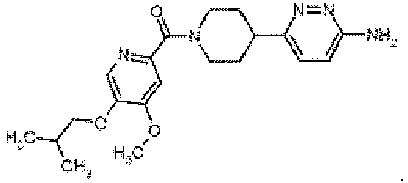

The TRPC6 inhibitor may also be:

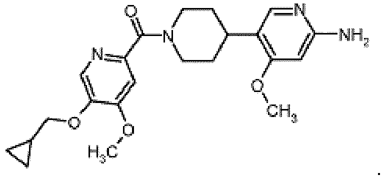

The TRPC6 inhibitor may also be:

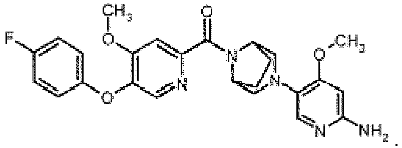

The TRPC6 inhibitor may also be:

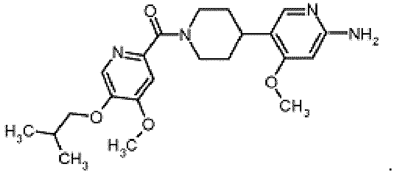

The TRPC6 inhibitor may also be:

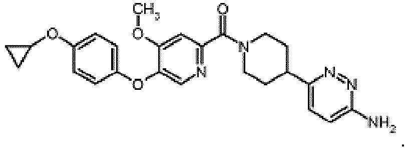

The TRPC6 inhibitor may also be:

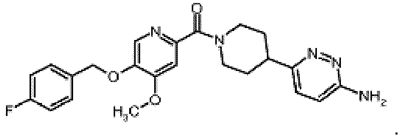

The TRPC6 inhibitor may also be:

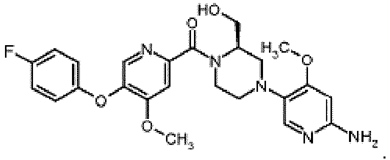

The TRPC6 inhibitor may also be:

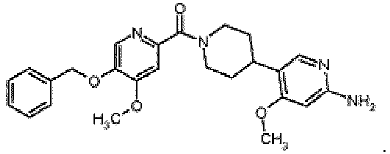

The TRPC6 inhibitor may also be:

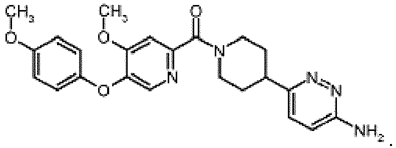

The TRPC6 inhibitor may also be:

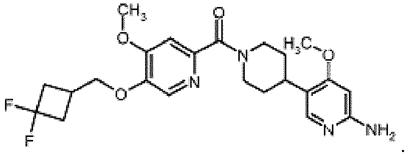

The TRPC6 inhibitor may also be:

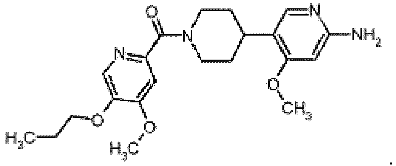

The TRPC6 inhibitor may also be:

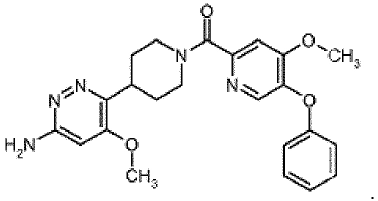

The TRPC6 inhibitor may also be:

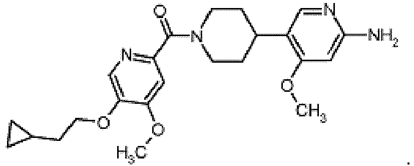

The TRPC6 inhibitor may also be:

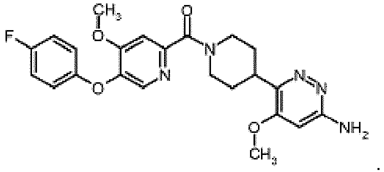

The TRPC6 inhibitor may also be:

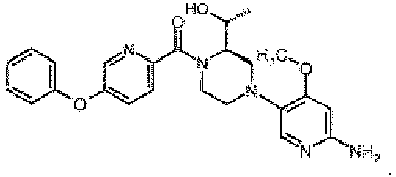

The TRPC6 inhibitor may also be:

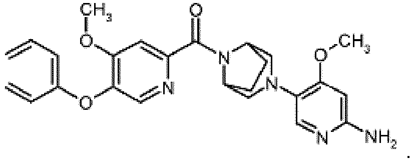

The TRPC6 inhibitor may also be:

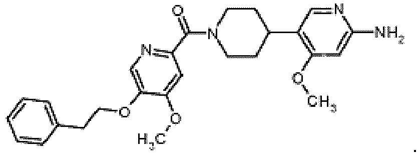

The TRPC6 inhibitor may also be:

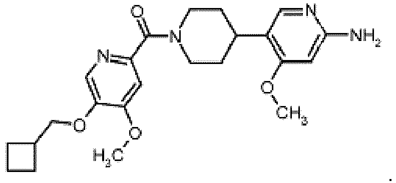

The TRPC6 inhibitor may also be:

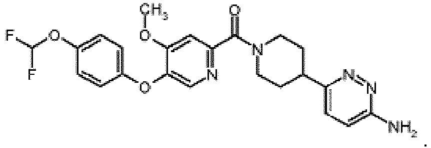

The TRPC6 inhibitor may also be:

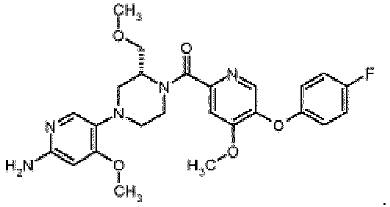

The TRPC6 inhibitor may also be:

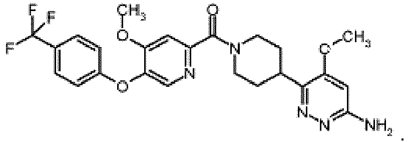

The TRPC6 inhibitor may also be:

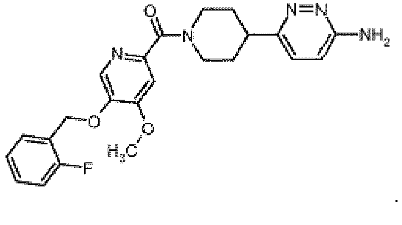

The TRPC6 inhibitor may also be:

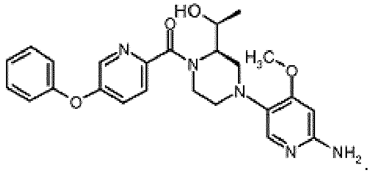

The TRPC6 inhibitor may also be:

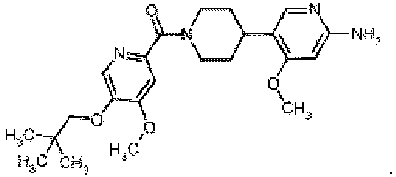

The TRPC6 inhibitor may also be:

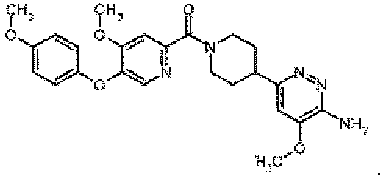

The TRPC6 inhibitor may also be:

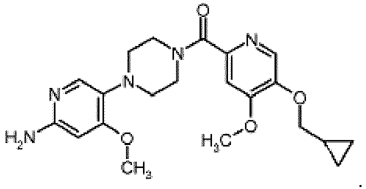

The TRPC6 inhibitor may also be:

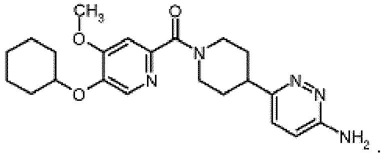

The TRPC6 inhibitor may also be:

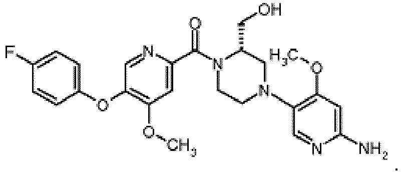

The TRPC6 inhibitor may also be:

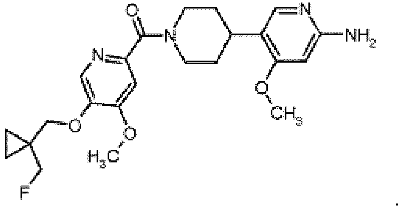

The TRPC6 inhibitor may also be:

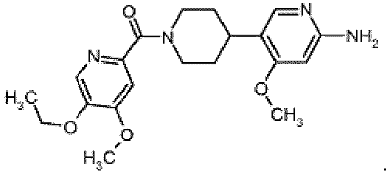

The TRPC6 inhibitor may also be:

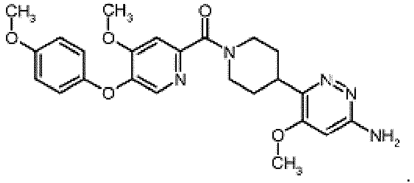

The TRPC6 inhibitor may also be:

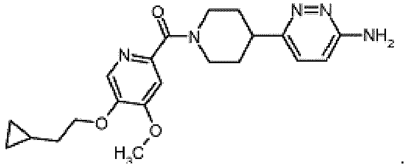

The TRPC6 inhibitor may also be:

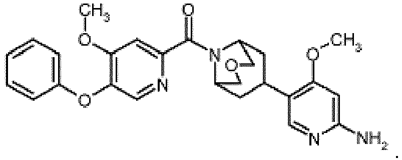

The TRPC6 inhibitor may also be:

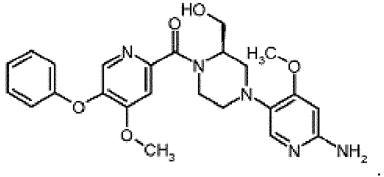

The TRPC6 inhibitor may also be:

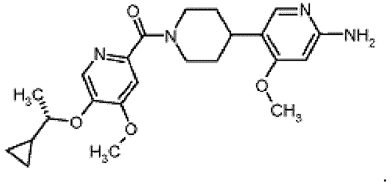

The TRPC6 inhibitor may also be:

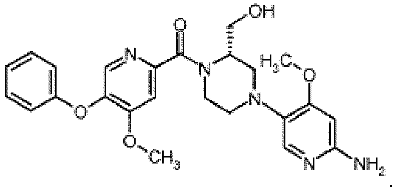

The TRPC6 inhibitor may also be:

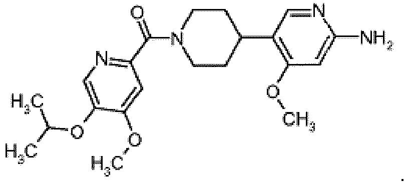

The TRPC6 inhibitor may also be:

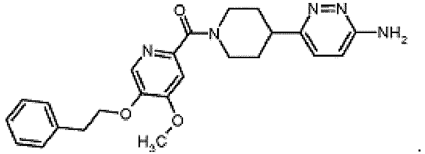

The TRPC6 inhibitor may also be:

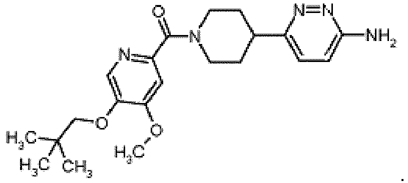

The TRPC6 inhibitor may also be:

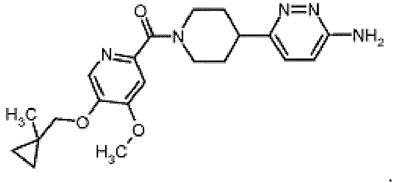

The TRPC6 inhibitor may also be:

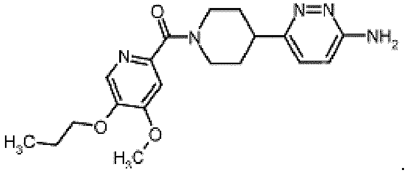

The TRPC6 inhibitor may also be:

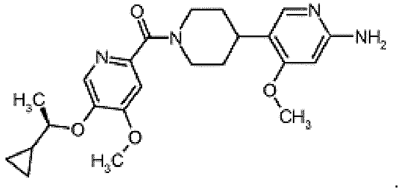

The TRPC6 inhibitor may also be:

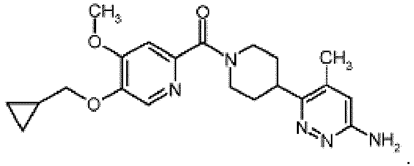

The TRPC6 inhibitor may also be:

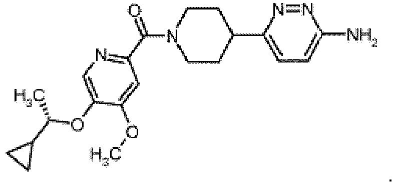

The TRPC6 inhibitor may also be:

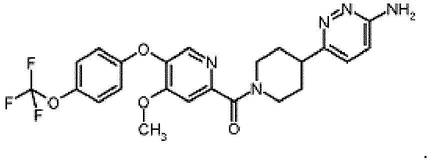

The TRPC6 inhibitor may also be:

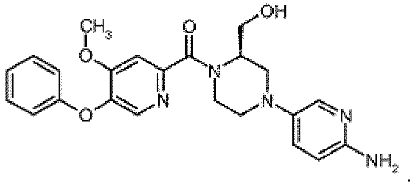

The TRPC6 inhibitor may also be:

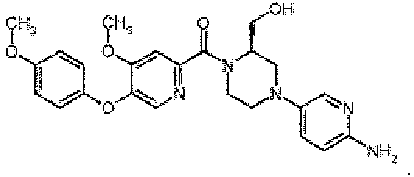

The TRPC6 inhibitor may also be:

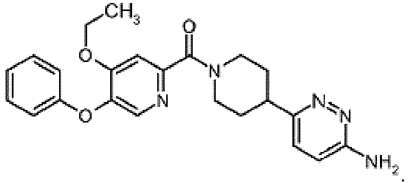

The TRPC6 inhibitor may also be:

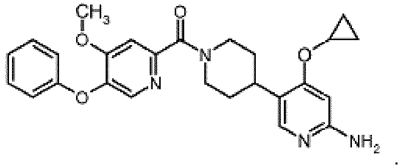

The TRPC6 inhibitor may also be:

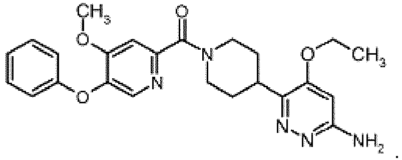

The TRPC6 inhibitor may also be:

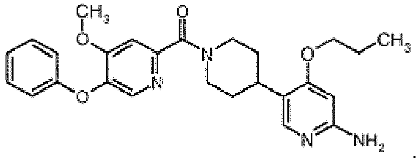

The TRPC6 inhibitor may also be:

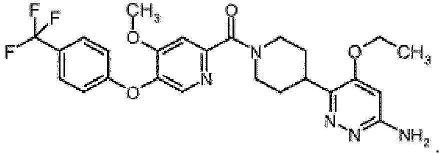

The TRPC6 inhibitor may also be:

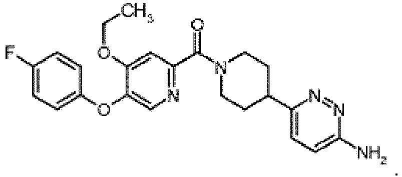

The TRPC6 inhibitor may also be:

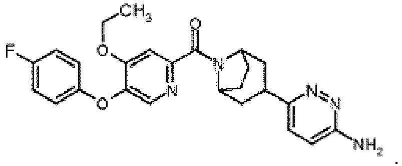

The TRPC6 inhibitor may also be:

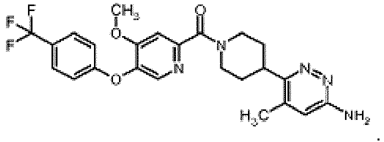

The TRPC6 inhibitor may also be:

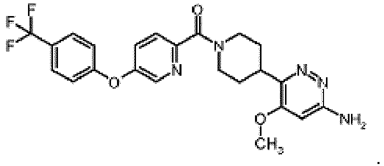

The TRPC6 inhibitor may also be:

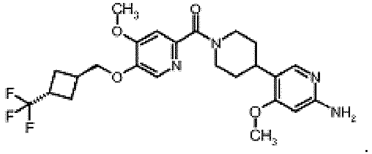

The TRPC6 inhibitor may also be:

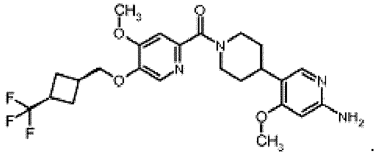

The TRPC6 inhibitor may also be:

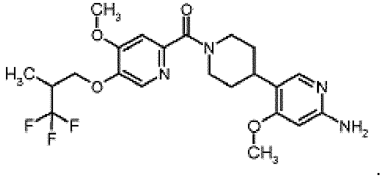

The TRPC6 inhibitor may also be:

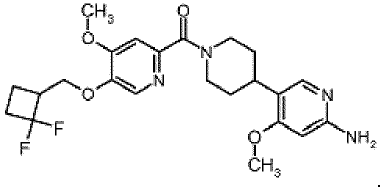

In some embodiments, the TRPC6 inhibitor may have the following formula (V), as disclosed in WO2019/161010: 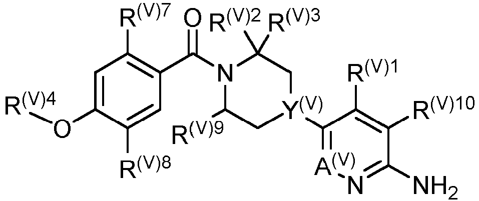 wherein:
Y^{(V)} is CH or N;
A^{(V)} is CH or N;
R^{(V)1} is H, C1-3alkyl, or OC1-3alkyl;
R^{(V)2} is H, C1-3alkyl or C3-6cycloalkyl wherein each of the C1-3alkyl or C3-6cycloalkyl of the R2 group may be optionally substituted with OH, halo or OC1-3alkyl;
R^{(V)3} is H or C1-3alkyl;
R^{(V)2} and R^{(V)3} together with the carbon to which they are attached may optionally join to form a 3- to 6-membered carbocyclic ring;
R^{(V)4} represents
   C1-6alkyl which may optionally be substituted with one to three groups independently selected from the group consisting of halo,
   C3-6cycloalkylmethyl and C3-6cycloalkylethyl, where the C3-6cycloalkyl of the C3-6cycloalkylmethyl and C3-6cycloalkylethyl may optionally be substituted with one to three groups independently selected from the group consisting of halo and methyl,
   1,2,3-thiadiazoylmethyl, thiazoylmethyl, isoxazolylmethyl or
   a group of formula 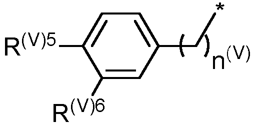
   wherein n(V) is 0 or 1;
R^{(V)5} is selected from the group consisting of **H,** halo, CF3, OCF3, CN, C1-3alkyl, OC1-3alkyl, C3-6cycloalkyl; wherein each of the C1-3alkyl and OC1-3alkyl of the R(V)5 groups may optionally be substituted with one to three groups each independently selected from the group consisting of halo, oxo, NH2, NH(C1-3alkyl), and N(C1-3alkyl)2;
R^{(V)6} is selected from the group consisting of H, halo, C1-3alkyl, and OC1-3alkyl;
wherein
R^{(V)5} and R^{(V)6} may join to form a 5- or 6-membered carbocyclic ring wherein one or two carbon atoms of the 5- or 6-membered carbocyclic ring may optionally be replaced by one or two oxygen atoms;
R^{(V)7} is selected from the group consisting of H, halo, C1-3alkyl and OC1-3alkyl, wherein the C1-3alkyl of the R^{(V)7} group may optionally be substituted with one to three substituents selected from the group consisting of halo;
R^{(V)8} is selected from the group consisting of H and halo;
R^{(V)9} is H or C1-3alkyl; wherein
R^{(V)2} and R^{(V)9} may join to form a bicyclic ring;
R^{(V)10} is H or C1-3alkyl;
or a pharmaceutically acceptable salt, solvate or hydrate thereof.

Accordingly the TRPC6 inhibitor, as disclosed in WO 2019/161010, may be - 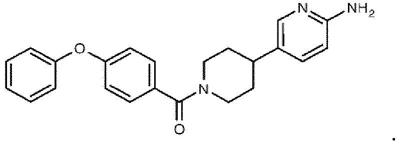 The TRPC6 inhibitor may also be:

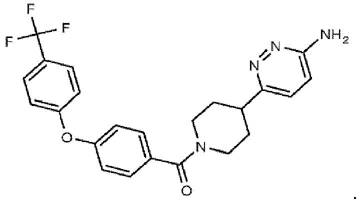

The TRPC6 inhibitor may also be:

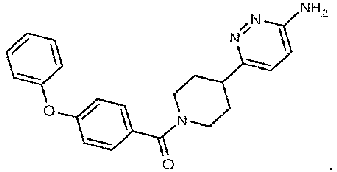

The TRPC6 inhibitor may also be:

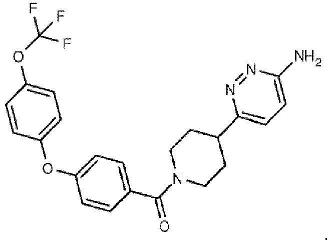

The TRPC6 inhibitor may also be:

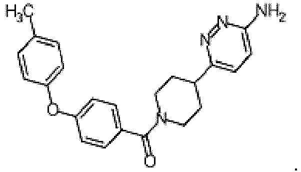

The TRPC6 inhibitor may also be:

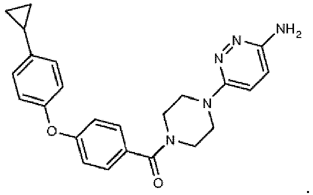

The TRPC6 inhibitor may also be:

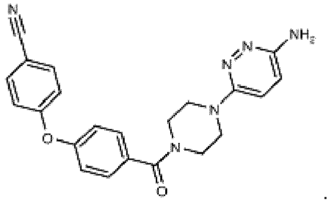

The TRPC6 inhibitor may also be:

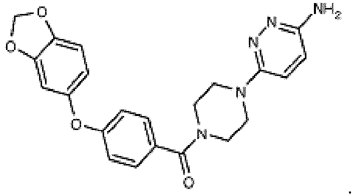

The TRPC6 inhibitor may also be:

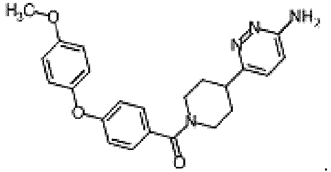

The TRPC6 inhibitor may also be:

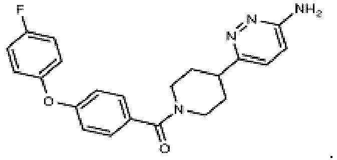

The TRPC6 inhibitor may also be:

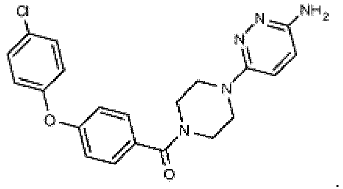

The TRPC6 inhibitor may also be:

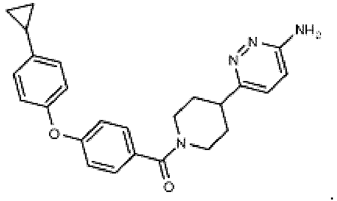

The TRPC6 inhibitor may also be:

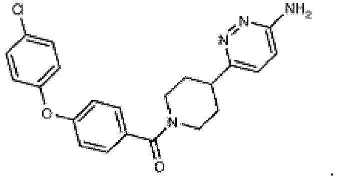

The TRPC6 inhibitor may also be:

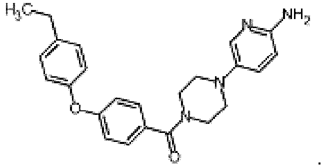

The TRPC6 inhibitor may also be:

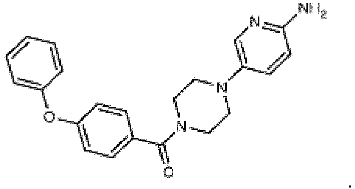

The TRPC6 inhibitor may also be:

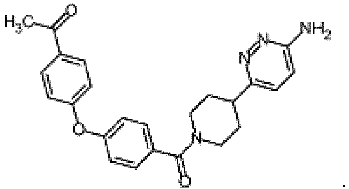

The TRPC6 inhibitor may also be:

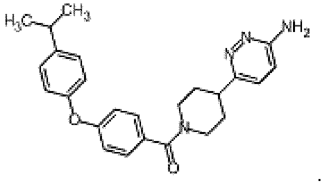

The TRPC6 inhibitor may also be:

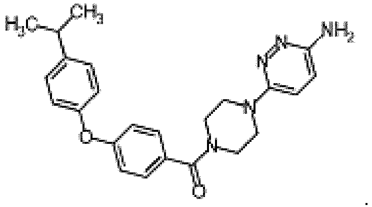

The TRPC6 inhibitor may also be:

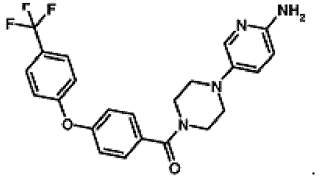

The TRPC6 inhibitor may also be:

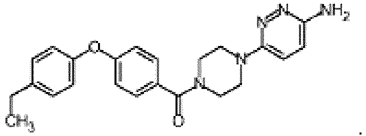

The TRPC6 inhibitor may also be:

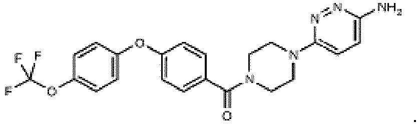

The TRPC6 inhibitor may also be:

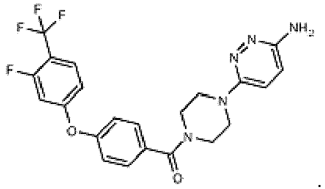

The TRPC6 inhibitor may also be:

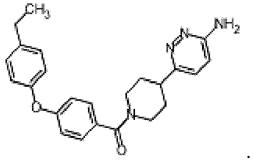

The TRPC6 inhibitor may also be:

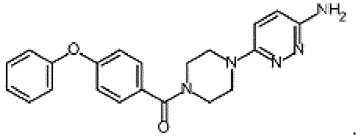

The TRPC6 inhibitor may also be:

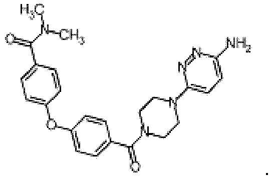

The TRPC6 inhibitor may also be:

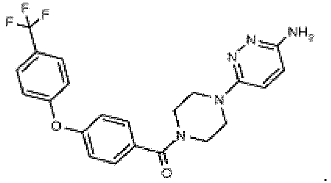

The TRPC6 inhibitor may also be:

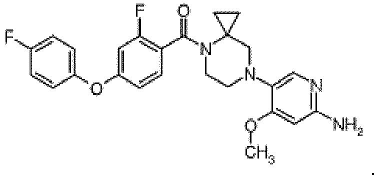

The TRPC6 inhibitor may also be:

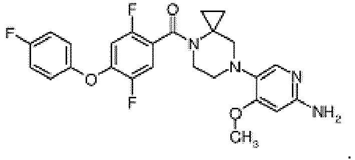

The TRPC6 inhibitor may also be:

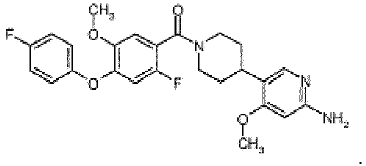

The TRPC6 inhibitor may also be:

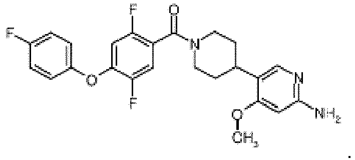

The TRPC6 inhibitor may also be:

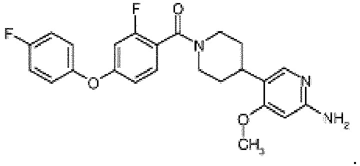

The TRPC6 inhibitor may also be:

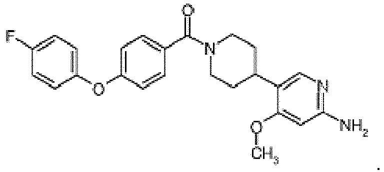

The TRPC6 inhibitor may also be:

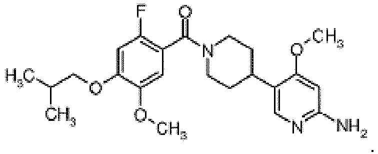

The TRPC6 inhibitor may also be:

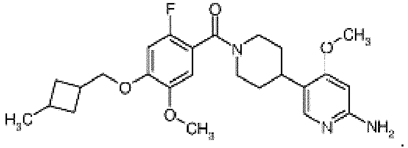

The TRPC6 inhibitor may also be:

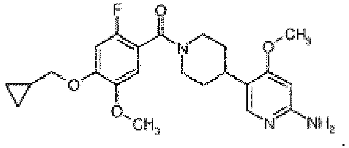

The TRPC6 inhibitor may also be:

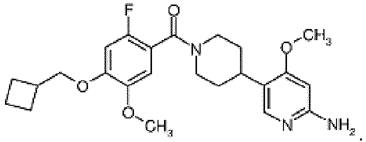

The TRPC6 inhibitor may also be:

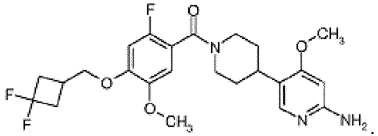

The TRPC6 inhibitor may also be:

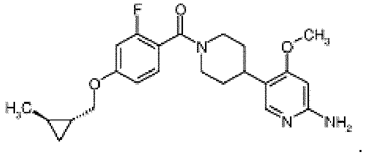

The TRPC6 inhibitor may also be:

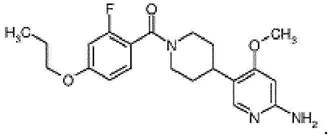

The TRPC6 inhibitor may also be:

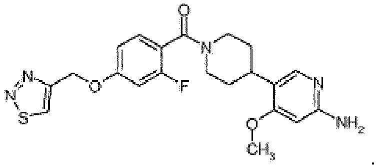

The TRPC6 inhibitor may also be:

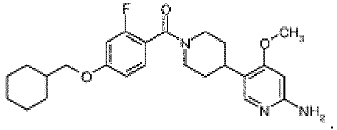

The TRPC6 inhibitor may also be:

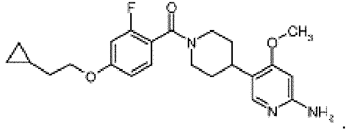

The TRPC6 inhibitor may also be:

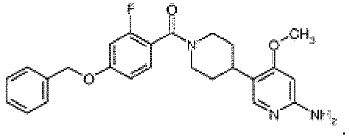

The TRPC6 inhibitor may also be:

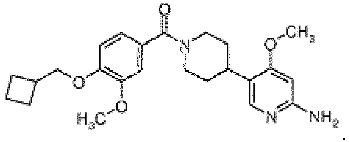

The TRPC6 inhibitor may also be:

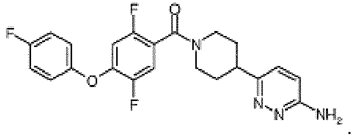

The TRPC6 inhibitor may also be:

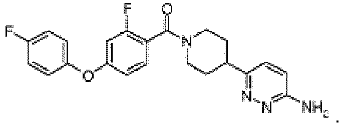

The TRPC6 inhibitor may also be:

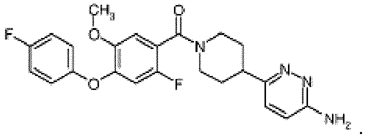

The TRPC6 inhibitor may also be:

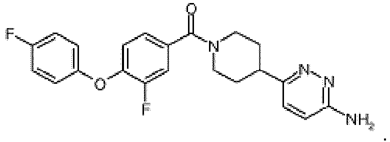

The TRPC6 inhibitor may also be:

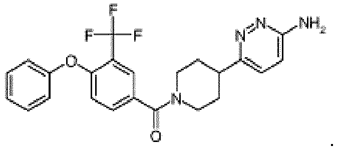

The TRPC6 inhibitor may also be:

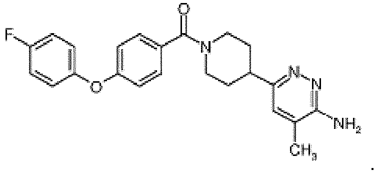

The TRPC6 inhibitor may also be:

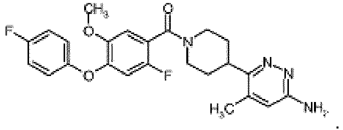

The TRPC6 inhibitor may also be:

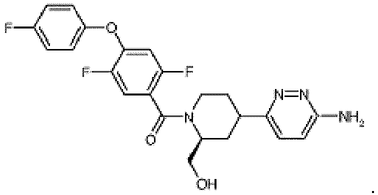

The TRPC6 inhibitor may also be:

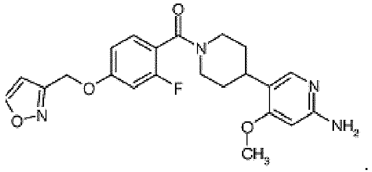

The TRPC6 inhibitor may also be:

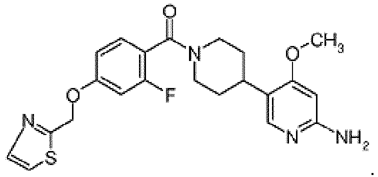

The TRPC6 inhibitor may also be BI 764198, available from Boehringer Ingelheim, Ingelheim am Rhein, Germany (https://www.boehringer-ingelheim.de/pressemitteilung/entwicklung-potentieller-therapie-fuer-schwere-covid19-komplikationen).

The present invention also relates to a method of manufacturing a medical device suitable for being inserted into the lumen of an anatomic structure of a subject, wherein the method comprises contacting the surface of the medical device with a coating composition comprising a TRPC6 inhibitor. The medical device may be any medical device as disclosed herein, e.g. a stent or a balloon. Accordingly, the medical device may be a catheter. The medical device may be a stent. Also, the medical device may be a balloon. The medical device may be a bioabsorbable scaffold. The medical device may be a microcatheter.

According to the invention, said method may comprise contacting a surface of the medical device with a coating composition comprising the TRPC6 inhibitor. In particular, in the context of the invention said contacting comprises the step of preparing a coating composition including the TRPC6 inhibitor as defined herein. The skilled person knows how to prepare a suitable coating composition, and any coating composition which comprises a TRPC6 inhibitor and which is suitable for depositing a coating onto a medical device may be used. For example, the coating composition may be a liquid, e.g. a solution comprising the TRPC6 inhibitor.

In this context, the means for the administration of a TRPC6 inhibitor may refer to a coating which is formed by a depositing a coating composition onto the medical device.

Further according to the invention, the coating composition applied on the surface of the medical device may comprise a TRPC6 inhibitor and a polymer as disclosed herein.

Said coating composition may further comprise a polymer, and/or a solvent as disclosed herein. In particular, the medical device according to the invention can be coated with a coating composition comprising a biocompatible polymer. Additionally, said polymer may be dissolved in a solvent.

Representative examples of polymers that can be used in the coating composition include ethylene vinyl alcohol copolymer (commonly known by the generic name EVOH or by the trade name EVAL); poly(hydroxyvalerate); poly(L-lactic acid); polycaprolactone; poly(lactide-co-glycolide); poly(hydroxybutyrate); poly(hydroxybutyrate-co-valerate); polydioxanone; polyorthoester; polyanhydride; poly(glycolic acid); poly(D,L-lactic acid); poly(glycolic acid-co-trimethylene carbonate); polyphosphoester; polyphosphoester urethane; poly(amino acids); cyanoacrylates; poly(trimethylene carbonate); poly(iminocarbonate); copoly(ether-esters) (e.g., PEO/PLA); polyalkylene oxalates; polyphosphazenes; biomolecules, such as fibrin, fibrinogen, cellulose, starch, collagen and hyaluronic acid; polyurethanes; silicones; polyesters; polyolefins; polyisobutylene and ethylene-alphaolefin copolymers; acrylic polymers and copolymers; vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile; polyvinyl ketones; polyvinyl aromatics, such as polystyrene; polyvinyl esters, such as polyvinyl acetate; copolymers of vinyl monomers with each other and olefins, such as ethylene-methyl methacrylate copolymers, acrylonitrile-styrene copolymers, ABS resins, and ethylene-vinyl acetate copolymers; polyamides, such as Nylon 66 and polycaprolactam; alkyd resins; polycarbonates; polyoxymethylenes; polyimides; polyethers; epoxy resins; polyurethanes; rayon; rayon-triacetate; cellulose; cellulose acetate; cellulose butyrate; cellulose acetate butyrate; cellophane; cellulose nitrate; cellulose propionate; cellulose ethers; and carboxymethyl cellulose. Furthermore, various combinations of polymers can be employed. The appropriate mixture of polymers can be coordinated with biologically active materials of interest to produce desired effects when coated on a medical device in accordance with the invention.

Representative examples of solvents can include N,N-dimethylacetamide (DMAC) having the formula CH₃-CO-N(CH₃)₂, N,N-dimethylformamide (DMFA) having the formula H-CO-N(CH₃)₂, tetrahydrofuran (THF) having the formula C₄H₈O, dimethylsulfoxide (DMSO) having the formula (CH₃)₂S-O, or trifluoro acetic anhydride (TFAA) having the formula (CF₃-CO)₂O (see e.g. US7335265-1). Further, examples of useful solvents include tetrahydrofuran, dichloromethane, chloroform, toluene, acetone, isooctane, 1,1,1,-trichloroethane, ethyl acetate, N-methylpyrrolidone (NMP), dimethylsulfoxide (DMSO), dimethylformamide (DMF), and dimethylacetamide (DMAC)), acetone/cyclohexanone solvent, and mixture thereof (see e.g. US7105198 and WO2007130257).

Depending on the volatility of the particular solvent employed, the solvent can evaporate essentially upon contact with the medical device, e.g. a stent, so that a coating comprising the TRPC6 inhibitor is formed. Evaporation of the solvent(s) can be induced by application of a warm gas between each repetition which can prevent coating defects and minimize interaction between the active agent and the solvent. The medical device may be positioned below a nozzle blowing the warm gas. After evaporation of the solvent, the TRPC6 inhibitor remains on the surface of the medical device. In addition or alternatively, the TRPC6 inhibitor can remain in pores of the medical device.

Although most of the medical devices (or at least a part of them) that are used today to decrease the rate of restenosis are coated with polymeric material, polymers are known to induce inflammatory responses which can translate into delayed healing and thus increased risk for an adverse outcome. Accordingly, the medical device of the invention may comprise a polymer-free coating. Accordingly, said coating composition may comprise a TRPC6 inhibitor and a solvent and does not comprise a polymer.

The coating composition comprising the TRPC6 inhibitor and preferably a solvent and a polymer, may then applied to the medical device by methods known in the art. For example, said methods include dip-coating and/or spray-coating or any other acceptable method known in the art.

The medical device may be coated by any procedure suitable for coating a solid material with a solution. The medical device may e.g. be dipped into a solution containing the TRPC6 inhibitor. Alternatively, it can be dipped consecutively in two solutions, wherein one solution contains the TRPC6 inhibitor and the other the second drug defined elsewhere herein.

The concentration of the drug solution is preferably from about 0.1 to about 10 %, more preferably from about 0.2 to about 5 %, still more preferably from about 0.3 to about 3 % and most preferably from about 0.5 to about 1 %.

Thereafter, the medical device may be dried e.g. by mild heating, air-drying or any other suitable method known to the artisan. The medical device may be coated using e.g. dip coating. Dip-coating typically involves immersing the medical device into a liquid coating Solution.

Also, a method of coating a medical device, e.g. a stent, via dip coating may e.g. comprise immersing a portion of the medical device into a coating liquid, and withdrawing the immersed portion of the medical device from the coating liquid. The medical device is simultaneously rotated with respect to the coating composition while the medical device is being immersed and withdrawn. The various methods of dip-coating are known to the skilled in the art. A method of dip-coating can be the one described in US7105198B2.

In embodiments of the invention, the medical device, e.g. a stent, is spray-coated. Typically, the conventional spray-coating methods are usually implemented with a device such as an airbrush. A coating on a medical device may be fabricated by spraying a coating composition including polymer and drug on the medical device. Spray coating a medical device typically involves mounting or disposing a medical device on a support, followed by spraying a coating material from a nozzle onto the mounted medical device. A spray apparatus, such as EFD 780S spray device with VALVEMATE 7040 control system (manufactured by EFD Inc., East Providence, R.I., can be used to apply a composition to a stent. The various methods of spray coating are known to the person skilled in the art. A method of spray coating can be the one described in WO2007/130257.

In embodiments, the coating machine described by Wessely and coworkers may be used, which permits individual, on-site coating of medical devices such as stents e.g. with a unique microporous surface (Wessely R. et al., 2005, Arterioscler Thromb Vasc Biol 25: 748 - 753) allowing for individualizable, dose-adjustable, and multiple coatings with identical or various compounds, designated ISAR (individualizable drug-eluting stent system to abrogate restenosis). Preferably, medical devices, such as e.g. stents, can be coated using the drug solutions as defined above.

Therefore, according to the method of the invention, the surface of the medical device defined elsewhere herein may be contacted with the coating composition of the invention by dip coating or spray coating.

The present invention also relates to a medical device obtainable or being obtained by a method as disclosed herein.

The present invention also relates to a TRPC6 inhibitor for use in the treatment or prevention of a disease associated with neointimal hyperplasia, wherein the neointimal hyperplasia is associated with the migration of smooth muscular cells.

The stenosis and/or restenosis may be a stenosis and/or restenosis of a blood vessel. The stenosis and/or restenosis may be a stenosis and/or restenosis of the esophagus. The stenosis and/or restenosis may be a stenosis and/or restenosis of the trachea. The stenosis and/or restenosis may be a stenosis and/or restenosis of the urethra. preferably a blood vessel, more preferably an artery. Preferably, the stenosis and/or restenosis is a stenosis and/or restenosis of a blood vessel, more preferably of an artery. Thus, the disease may be a vascular stenotic/restenotic lesion or a stenosis and/or restenosis in heart valves.

Non-limiting examples of a stenosis and/or restenosis of blood vessels include peripheral artery stenosis and/or restenosis, coronary artery stenosis and/or restenosis, carotid artery stenosis and/or restenosis which may predispose to (strokes and transient ischemic episodes) or renal artery stenosis and/or restenosis. Thus, the disease may be a peripheral artery stenosis and/or restenosis, coronary artery stenosis and/or restenosis or renal artery stenosis and/or restenosis.

The stenosis and/or restenosis may also be a stenosis and/or restenosis in heart valves. The stenosis in heart valves can be any stenosis and/or restenosis in heart valves. Examples of a stenosis and/or restenosis in heart valves includes the pulmonary valve stenosis and/or restenosis, mitral valve stenosis and/or restenosis, tricuspid valve stenosis and/or restenosis, aortic valve stenosis and/or restenosis.

Thus, the disease may be a pulmonary valve stenosis and/or restenosis, mitral valve stenosis and/or restenosis, tricuspid valve stenosis and/or restenosis, aortic valve stenosis and/or restenosis, preferably an aortic valve stenosis and/or restenosis.

Stenosis may also be a stenosis and/or restenosis of the esophagus. Smooth muscle cells also seem to play a role in esophageal stenosis Non-limiting examples include pyloric stenosis and/or restenosis or esophageal stricture.

Thus, the subject may also have been or may be afflicted with or be at risk of pyloric stenosis and/or restenosis or esophageal stricture.

Stenosis and/or restenosis may also be a stenosis and/or restenosis of the trachea. Such stenosis and/or restenosis are known to the skilled person. Non-limiting examples include subglottic stenosis or larygotracheal stenosis and/or restenosis. Thus, the disease may be subglottic stenosis or larygotracheal stenosis and/or restenosis.

Stenosis and/or restenosis may also be a stenosis and/or restenosis of the urethra. Non-limiting examples include urethral meatal stenosis and/or restenosis.

As used herein urethral meatal stenosis and/or restenosis may be any urethral meatal stenosis and/or restenosis. Thus, the disease is a urethral meatal stenosis and/or restenosis.

The disease may be a post-angioplasty restenosis (PARS). The restenosis may be an in-stent restenosis or ISR.

The use may comprise inhibiting or decreasing the migration of smooth muscle cells (SMCs), preferably by inhibiting or decreasing the migration of SMCs within the lumen of the anatomical structure compared to the migration present in the absence of the inhibitor. In particular, the migration of the SMCs may be inhibited or decreased/prevented by the TRPC6 inhibitor.

Especially, treatment or prevention may be achieved by applying a compound which inhibits the migration of smooth vascular muscle cells, thereby reducing the risk of neointimal hyperplasia/restenosis.

As such the term "treating" or "treatment" includes administration of a TRPC6 inhibitor, preferably in the form of a medicament, to a subject, defined elsewhere herein, suffering from a disease associated with neointima hyperplasia for the purpose of ameliorating or improving symptoms.

As used herein, the terms "prevent", "prevention" or "prophylaxis", and "preventing" refers to the reduction in the risk of acquiring or developing a disease associated with neointima formation. Also meant by "prophylaxis" is the reduction or inhibition of the recurrence of a disease associated with neointima formation.

In particular, the TRPC6 inhibitor can prevent neointima formation by inhibiting or decreasing the migration of smooth muscle cells (SMC). For example, the migration of SMCs to the site at risk of neointima formation or affected by neointima formation may be decreased or inhibited.

Preferably, "prevent" may mean that, after administration of the TRPC6 inhibitor, about 70 %, 80 %, 90 %, 95 % or 100% of the total SMCs are located in the media portion of the blood vessel wall, i.e. do not migrate in the sub endothelium of the blood vessel.

The migration of the SMCs can *inter alia* be determined by cell migration assays as defined elsewhere herein and described in the Examples.

A disease associated with neointima hyperplasia as used herein may refer to any disease that includes the risk of occurrence of neointimal hyperplasia.

Preferably, the disease may be restenosis or stenosis. It is envisioned that the TRPC6 inhibitor is systemically administered for treatment or prevention.

Thus, the TRPC6 inhibitor is administered via a medical device as disclosed herein. The medical device may be catheter. The medical device may be a stent. The medical device may be a balloon. The medical device may be a microcatheter. The medical device may be a bioabsorbable scaffold. Preferably, the medical device is a stent or a balloon. This administration may include inserting the medical device into a lumen of an anatomical structure of a subject, preferably to a site at risk of neointima hyperplasia or affected by neointima hyperplasia. For example, administration may include implanting a stent comprising a coating, wherein the coating comprises the TRPC6 inhibitor.

The TRPC6 inhibitor may be administered in a therapeutically effective amount.

It is further envisioned that the TRPC6 inhibitor is administered between 2 and 7 days after the vascular injury. It is also envisioned that the medical device is inserted between 2 and 7 days after the vascular injury.

The present invention also relates to a method of treating or preventing a disease associated with neointimal hyperplasia, said method comprising administering a TRPC6 inhibitor to a subject in need thereof. Methods of administration, treatment and/or diagnosis are not according to the invention.

The TRPC6 inhibitor may be administered in a therapeutically effective amount. The TRPC6 inhibitor may be administered to a subject in need thereof. It is also contemplated that the subject in need thereof is a subject at risk or affected by neointimal hyperplasia. The neointimal hyperplasia may be associated with the migration of smooth muscular cells.

The present invention also relates to an in-vitro method of inhibiting migration of smooth muscle cells, said method comprising contacting the smooth muscle cells with a TRPC6 inhibitor and determining the migration of the smooth muscle cells compared to the migration of smooth muscle cells in the absence or before the contacting with the TRPC6 inhibitor.

It is envisioned that the migration of the smooth muscle cells is determined by scratch wound assay and/or by radius migration assay. The method is an in vitro method.

The present invention also relates to a kit comprising the medical device as disclosed herein.

The present invention also relates to an *in vitro* test kit comprising:
(a) smooth muscle cells (SMCs); and
(b) a surface suitable for SMC cultivation coated with a TRPC6 inhibitor and
c) optionally a negative control.

The present invention also relates to a pharmaceutical composition comprising a TRPC6 inhibitor and a pharmaceutically acceptable excipient.

Therefore, the TRPC6 inhibitor may be in the form of orally administrable suspensions or tablets. Said orally administrable suspensions and tablets are prepared according to techniques available in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents, and lubricants known in the art. The compound may also be administered as part of an injectable preparation (intravenously or intraarterially). The injectable solutions or suspensions may be formulated according to known art, using suitable non-toxic, parenterally acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

It is further envisioned that the pharmaceutical composition additionally comprises at least one further drug. The further drug may be a mTOR-Inhibitor of the limus family (e.g. sirolimus, everolimus, biolimus, zotarolimus etc., preferably sirolimus). Alternatively or in addition, the further drug may be paclitaxel. The further drug may also be a anti-infectives such as antibiotics and antiviral agents, analgesics and analgesic combinations, anorexics and appetite suppressants, anthelmintics, anesthetics, antiarthritics, antiasthma agents, anticonvulsants, antidepressants, antidiabetic agents, antidiarrheals, antihistamines, antiinflammatory agents, antimigraine preparations, antimotion sickness agents, antinauseants, antineoplastics, antiparkinsonism agents, antipruritics, antipsychotics, antipyretics, antispasmodics, anticholinergics, sympathomimetics, xanthine derivatives, cardiovascular preparations including calcium channel blockers, beta blockers, antiarrhythmics, antihypertensives, diuretics, vasodilators (general, coronary, peripheral and cerebral), central nervous system stimulants, cough and cold preparations, decongestants, diagnostics, hormones, hypnotics, immunosuppressives, muscle relaxants, parasympatholytics, parasympathomimetics, psychostimulants, sedatives, tranquilizers, antioxidants, vitamins, minerals, and herbal extracts or preparations.

It is further contemplated that the pharmaceutical composition is coated on a medical device. The medical device may be a catheter. The medical device may be a stent. The medical device may be a balloon. The medical device may be a microcatheter. The medical device may be a bioabsorbable scaffold. Preferably, the medical device is a stent or a balloon.

The present invention also relates to a pharmaceutical composition as disclosed herein for use in the treatment or prevention of a disease associated with neointimal hyperplasia, wherein the neointimal hyperplasia is associated with the migration of smooth muscular cells as also described elsewhere herein.

The TRPC6 inhibitor is selected from the group consisting of compounds 1 to 95 in the Table below:

| Cpd No. | Structure | Compound Name |
|---|---|---|
| **1** | 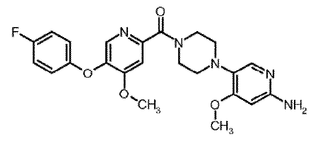 | [4-(6-Amino-4-methoxy-pyridin-3-yl)-piperazin-1-yl]-[5-(4-fluoro-phenoxy)-4-methoxy-pyridin-2-yl]-methanone |
| **2** | 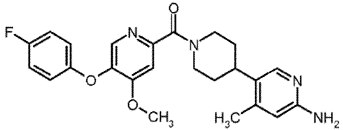 | (6-Amino-4-methyl-3',4',5',6'-tetrahydro-2'H-[3,4'-bipyridinyl-1'-yl)-[5-(4-fluoro-phenoxy)-4-methoxy-pyridin-2-yl]-methanone |
| **3** | 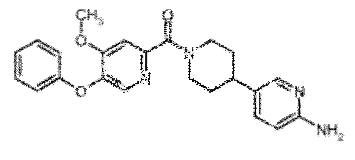 | (6-Amino-3',4',6,6'-tetrahydro-2'H-[3,4']bipyridinyl-1"-yl)-(4-methoxy-5-phenoxy-pyridin-2-yl)-methanone |
| **4** | 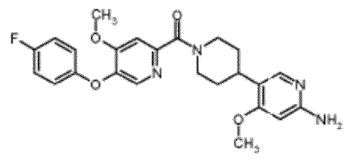 | (6-Amino-4-methoxy-3',4',5',6'-tetrahydro-2'H-[3,4"]bipyndinyl-1'-yl)-[5-(4-fluoro-phenoxy)-4-methoxy-pyridin-2-yl]-methanone |
| **5** | 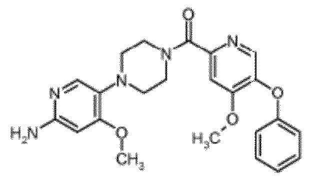 | (4-(6-Amino-4-methoxy-pyridin-3-yl)-piperazin-1-yl]-(4-methoxy-5-phenoxy-pyridin-2-yl)-methanone |
| **6** | 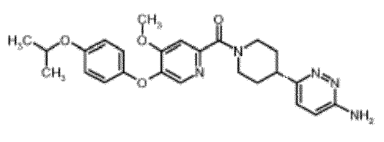 | [4-(6-Amino-pyridazin-3-yl)-piperidin-1-yl]-[5-(4-isopropoxy-phenoxy)-4-mathoxy-pyridin-2-yl]-methanone |
| **7** | 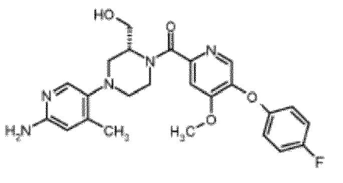 | ((R)-4-(6-Amino-4-methyl-pyridin-3-yl)-2-hydroxymethyl-piperazin-1-yl]-[5-(4-fluoro-phenoxy)-4-methoxy-pyridin-2-yl]-methanone |
| **8** | 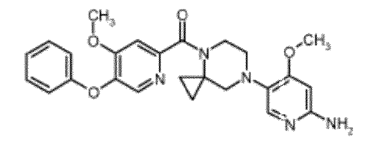 | [7-(6-Amino-4-methoxy-pyridin-3-yl)-4,7-diaza-spiro[2.5]oct-4-yl]-(4-methoxy-5-phenoxy-pyridin-2-yl)-methanone |
| **9** | 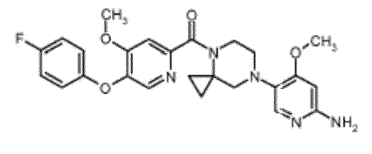 | [7-(6-Amino-4-methoxy-pyridin-3-yl)-4,7-diaza-spiro[2.5]oct-4-yl]-[5-(4-fluoro-phenoxy)-4-methoxy-pyridin-2-yl]-methanone |
| **10** | 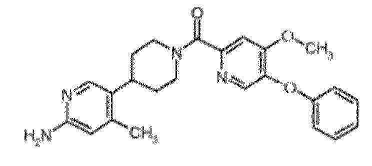 | (6-Amino-4-methyl-3',4',5',6'-tetrahydro-2'H-[3.4"]bipyridinyl-1'-yl)-(4-methoxy-5-phenoxy-pyridin-2-yl)-methanone |
| **11** | 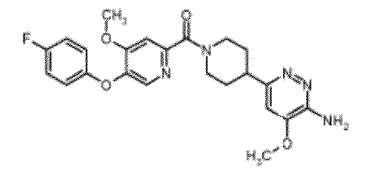 | [4-(6-Amino-5-methoxy-pyridazin-3-yl)-piperidin-1-yl]-[5-(4-fluoro-phenoxy)-4-methoxy-pyridin-2-yl]-methanone |
| **12** | 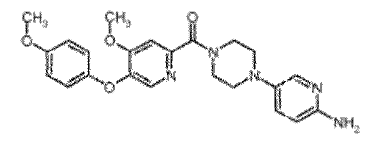 | (4-(6-Amino-pyridin-3-yl)-piperazin-1-yl]-[4-methoxy-5-(4-methoxy-phenoxy)-pyridin-2-yl)-methanone |
| **13** | 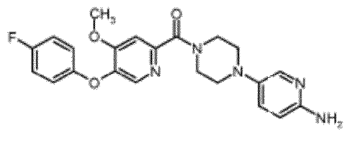 | [4-(6-Amino-pyridin-3-yl}-piperazin-1-yl]-[5-(4-fluoro-phenoxy)-4-methoxy-pyridin-2-yl]-methanone |
| **14** | 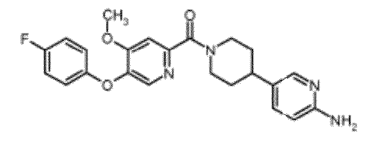 | (6-Amino-3',4',5',6'-tetrahydro-2'H-[3,4']bipyridinyl-1'-yl)-[5-(4-fluoro-phenoxy)-4-methoxy-pyridin-2-yl)-methanone |
| **15** | 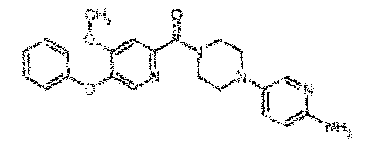 | [4-(6-Amino-pyridin-3-yl)-piperazin-1-yl]-(4-methoxy-5-phenoxy-pyridin-2-yl)-methanone |
| **16** | 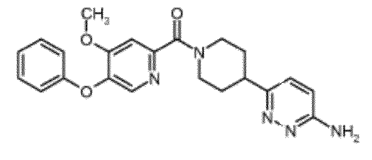 | [4-(6-Amino-pyridazin-3-yl)-piperidin-1-yl]-(4-methoxy-5-phenoxy-pyridin-2-yl)-methanone |
| **17** | 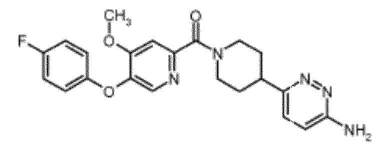 | [4-(6-Amino-pyridazin-3-yl)-piperidin-1-yl]-(5-(4-fluoro-phenoxy)-4-methoxy-pyridin-2-yl]-methanone |
| **18** | 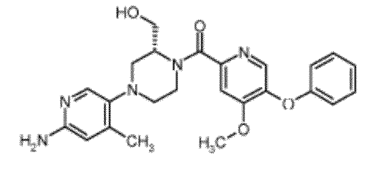 | [(R)-4-(6-Amino-4-methyl-pyridin-3-yl)-2-hydroxymethyl-piperazin-1-yl]-(4-methoxy-5-phenoxy-pyridin-2-yl)-methanone |
| **19** | 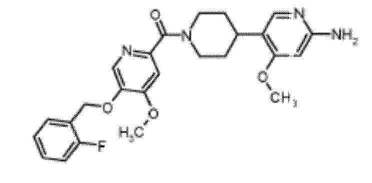 | (6-Amino-4-methoxy-3',4',5' 6'-tetrahydro-2'H-[3 ,4']bipyridinyl-1'-yl)-(5-(2-fluoro-benzyloxy)-4-methoxy-pyridin-2-yl]-methanone |
| **20** | 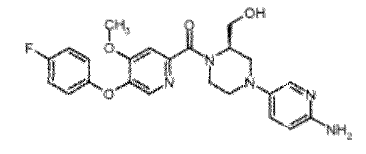 | [(R)-4-(6-Amino-pyridin-3-yl)-2-hydroxymethyl-piperazin-1-yl]-[5-(4-fluoro-phenoxy)-4-methoxy-pyridin-2-yl]-methanone |
| **21** | 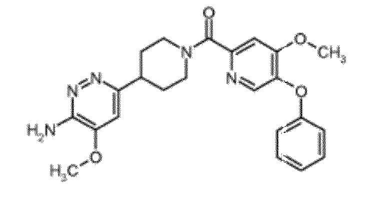 | [4-(6-Amino-5-methoxy-pyridazin-3-yl)-piperidin-1-yl]-(4-methoxy-5-phenoxy-pyridin-2-yl)-methanone |
| **22** | 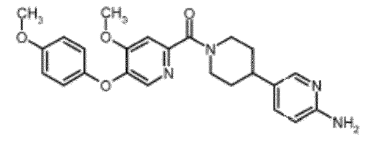 | (6-Amino-3', 4'.5',6' -tetrahydro-2'H-[3,4']bipyridinyl-1'-yl)-(4-methoxy-5-(4-methoxy-phenoxy)-pyridin-2-yl]-methanone |
| **23** | 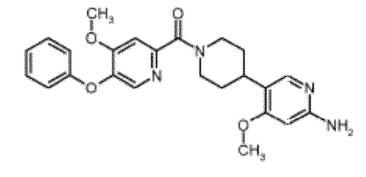 | (6-Amino-4-methoxy-3,4',5',6'-tetrahydro-2'H-[3,4']bipyridinyl-1'-yl)-(4-methoxy-5-phenoxy-pyridin-2-yl)-methanone |
| **24** | 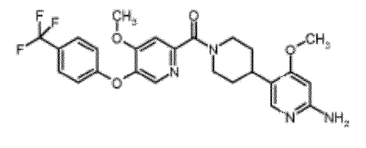 | (6-Amino-4-methoxy-3',4',5',6'-tetrahydro-2'H-[3,4']bipyridinyl-1'-yl)-[4-methoxy-5-(4-trifluoromethyl-phenoxy)-pyridin-2-yl]-methanone |
| **25** | 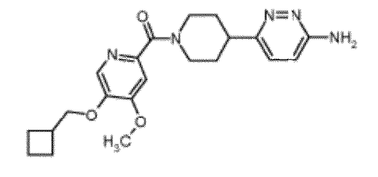 | [4-(6-Amino-pyridazin-3-yl)-piperidin-1-yl]-(5-cyclobutylmethoxy-4-methoxy-pyridin-2-yl}-methanone |
| **26** | 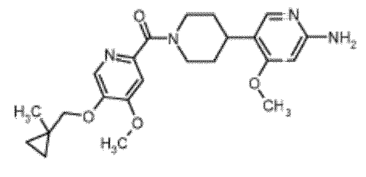 | (6-Amino-4-methoxy-3',4',5',6'-tetrahydro-2'H-[3,4']bipyridinyl-1'-yl)-[4-methoxy-5-(1-methyl-cyclopropylmethoxy)-pyridin-2-yl]-methanone |
| **27** | 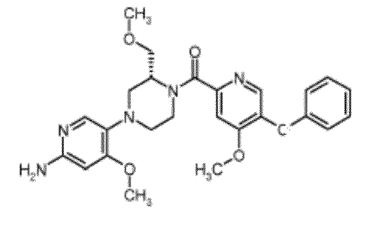 | [(R)-4-(6-Amino-4-methoxy-pyridin-3-yl)-2-methoxymethyl-piperazin-1-yl]-(4-metboxy-5-phenoxy-pyridin-2-yl)-methanone |
| **28** | 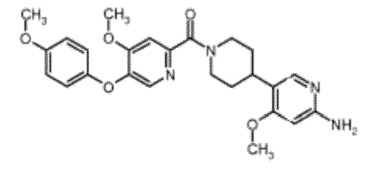 | (6-Amino-4-methoxy-3',4',5',6'-tetrahydro-2'H-[3,4']bipyridinyl-1'-yl)-[4-methoxy-5-(4-methoxy-phenoxy)-pyridin-2-yl]-methanone |
| **29** | 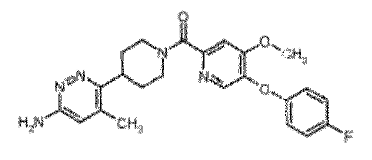 | [4-(6-Amino-4-methyl-pyridazin-3-yl)-piperidin-1-yl]-[5-(4-fluoro-phenoxy)-4-methoxy-pyridin-2-yl]-methanone |
| **30** | 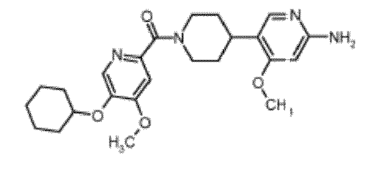 | (6-Amino-4-methoxy-3',4',5',6'-tetrahydro-2'H-[3,4']bipyridinyl-1'-yl)-(5-cyclohexyloxy-4-methoxy-pyridin-2-yl)-methanone |
| **31** | 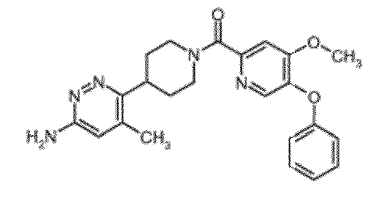 | [4-(6-Amino-4-methyl-pyridazin-3-yl)-piperidin-1-yl]-(4-methoxy-5-phenoxy-pyridin-2-yl)-methanone |
| **32** | 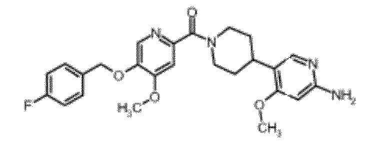 | (6-Amino-4-methoxy-3,4',5',6'-tetrahydro-2'H-[3,4']bipyridlnyl-1'-yl)-[5-(4-fluoro-benzyloxy]-4-methoxy-pyridin-2-yl)-methanone |
| **33** | 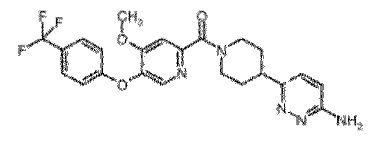 | [4-(6-Amino-pyridazin-3-yl)-piperidin-1-yl]-[4-methoxy-5-(4-trifluoromethyl-phenoxy)-pyridin-2-yl]-methanone |
| **34** | 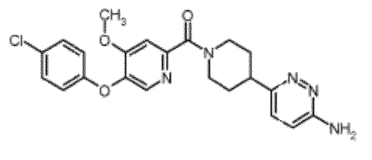 | [4-(6-Amino-pyridazin-3-yl)-piperidin-1-yl]-[5-(4-chloro-pbenoxy)-4-methoxy-pyridin-2-yl]-methanone |
| **35** | 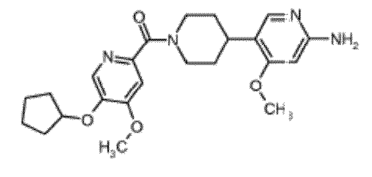 | (6-Amino-4-methoxy-3',4',5',6'-tetrahydro-2'H-[3,4']bipyridinyl-1'-yl)-(5-cyclopentyloxy-4-methoxy-pyridin-2-yl)-methanone |
| **36** | 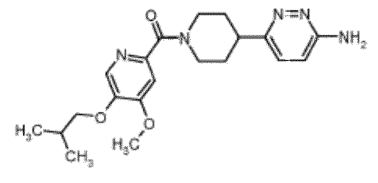 | [4-(6-Amino-pyridazin-3-yl)-piperidin-1-yl]-(5-isobutoxy-4-methoxy-pyridin-2-yl)-methanone |
| **37** | 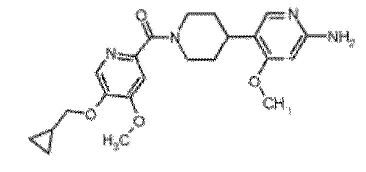 | (6-Amino-4-methoxy-3',4',5',6'-tetrahydro-2'H-[3,4']bipyridinyl-1'-yl)-(5-cyclopropylmethoxy-4-methoxy-pyridin-2-yl)-methanone |
| **38** | 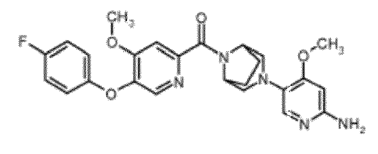 | [3-(6-Amino-4-methoxy-pyridin-3-yl)-3,8-diaza-bicyclo[3.2.1]oct-8-yl]-[5-(4-fluoro-phenoxy)-4-methoxy-pyridin-2-yl]-methanone |
| **39** | 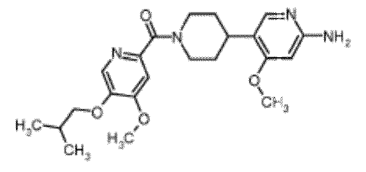 | (6-Amino-4-methoxy-3',4',5'.6'-tetrahydro-2'H-[3,4']bipyridinyl-1'-yl)-(5-isobutoxy-4-methoxy-pyridin-2-yl)-methanone |
| **40** | 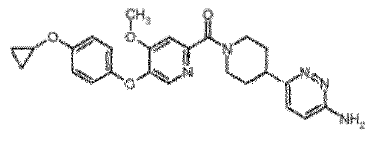 | [4-(6-Amino-pyridazin-3-yl)-piperidin-1-yl]-[5-(4-cyclopropoxy-phenoxy)-4-methoxy-pyridin-2-yl)-methanone |
| **41** | 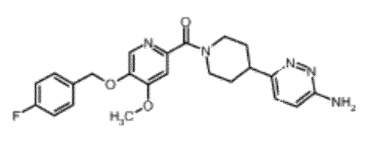 | [4-(6-Amino-pyridazin-3-yl)-piperidin-1-yl]-[5-(4-fluoro-benzyloxy)-4-methoxy-pyridin-2-yl]-methanone |
| **42** | 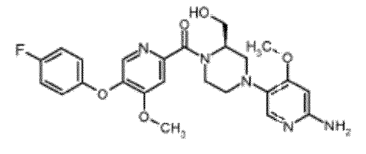 | [(R)-4-(6-Amino-4-methoxy-pyridin-3-yl)-2-hydroxymethyl-piperazin-1-yl)-[5-(4-fluoro-phenoxy)-4-methoxy-pyridin-2-yl]-methanone |
| **43** | 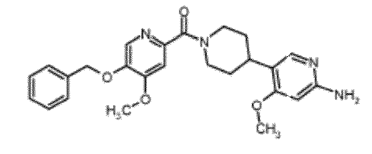 | (6-Amino-4-methoxy-3',4',5',6'-tetrahydro-2'H-[3,4']bipyridinyl-1'-yl)-(5-benzytoxy-4-methoxy-pyridin-2-yl)-methanone |
| **44** | 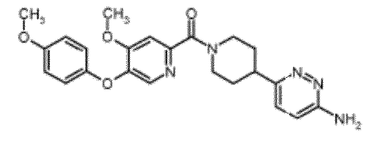 | [4-(6-Amino-pyridazin-3-yl)-piperidin-1-yl]-[4-methoxy-5-[4-methoxy-phenoxy)-pyridin-2-yl]-methanone |
| **45** | 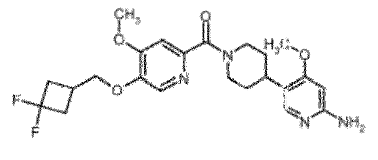 | (6-Amino-4-methoxy-3',4',5',6'-tetrahydro-2'H-[3,4']bipyridinyl-1'-yl)-[5-(3,3-difluoro-cyclobutylmethoxy)-4-methoxy-pyridin-2-yl]-methanone |
| **46** | 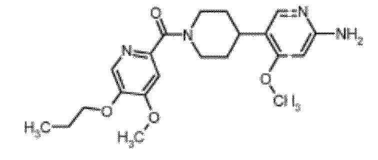 | (6-Amino-4-methoxy-3',4',5',6'-tetrahydro-2'H-[3,4']bipyridinyl-1'-yl)-(4-methoxy-5-propoxy-pyridin-2-yl)-methanone |
| **47** | 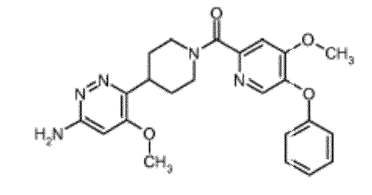 | [4-(6-Amino-4-methoxy-pyridazin-3-yl)-piperidin-yl]-(4-methoxy-5-phenoxy-pyridin-2-yl)-methanone |
| **48** | 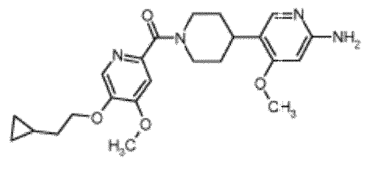 | (6-Amino-4-methoxy-3',4',5',6'-tetrahydro-2'H-[3,4']bipyridinyt-1'-yl)-(5-(2-cyclopropyl-ethoxy)-4-methoxy-pyridin-2-yl]-methanone |
| **49** | 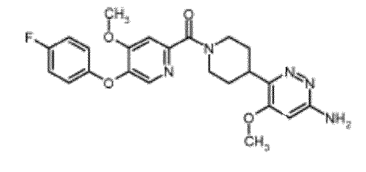 | [4-(6-Amino-4-methoxy-pyridazin-3-yl)piperidin-1-yl]-[5-(4-fluoro-phenoxy)-4-methoxy-pyridin-2-yl]-methanone |
| **50** | 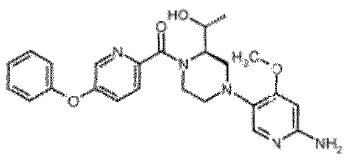 | (1 R)-1-[(2R)-4-(6-amino-4-methoxypyridin-3-yl)-1-(5-phenoxypyridine-2-carbonyl)piperazin-2-yl]ethan-1-ol |
| **51** | 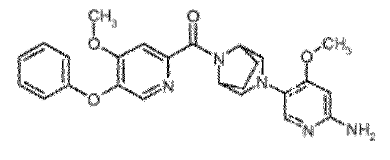 | [3-(6-Amino-4-methoxy-pyridin-3-yl)-3,8-diaza-bicyclo[3.2.1]oct-8-yl]-(4-methoxy-5-phenoxy-pyridin-2-yl)-methanone |
| **52** | 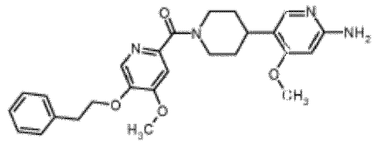 | (6-Amino-4-methoxy-3',4',5',6'-tetrahydro-2'H-[3,4']bipyridinyl-1'-yl)-(4-methoxy-5-phenethyloxy-pyridin-2-yl)-methanone |
| **53** | 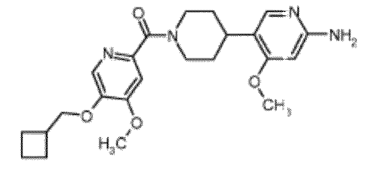 | (6-Amino-4-methoxy-3',4',5',6'-tetrahydro-2'H-[3,4']bipyridinyl-1'-yl)-(5-cyclobutylmethoxy-4-methoxy-pyridin-2-yl)-methanone |
| **54** | 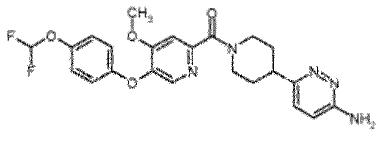 | [4-(6-Amino-pyridazin-3-yl)-piperidin-1-yl]-[5-(4-difluoromethoxy-phenoxy)-4-methoxy-pyridin-2-yl]-methanone |
| **55** | 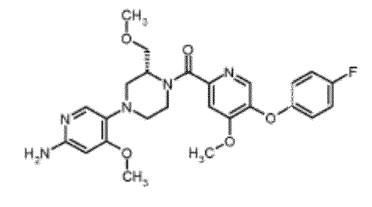 | [(R)-4-(6-Amino-4-methoxy-pyridin-3-yl)-2-methoxymethyl-piperazin-1-yl]-[5-(4-fluoro-phenoxy)-4-methoxy-pyridin-2-yl]-methanone |
| **56** | 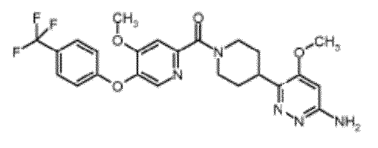 | (4-(6-Amino-4-methoxy-pyridazin-3-yl)-piperidin-1-yl]-[4-methoxy-5-(4-trifluoromethyl-phenoxy)-pyridin-2-yl]-methanone |
| **57** | 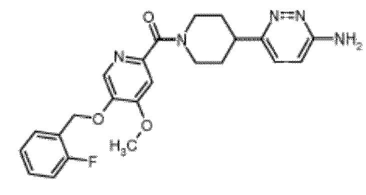 | [4-(8-Amino-pyridazin-3-yl-piperidin-1-yl]-[5-(2-fluoro-benzyloxy)-4-methoxy-pyridin-2-yl]-methanone |
| **58** | 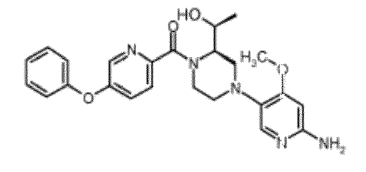 | (1S)-1-1(2R)-4-(6-amino-4-methoxypyridin-3-yl)-(5-phenoxypyridine-2-carbonyl)piperazin-2-yl]ethan-1-ol |
| **59** | 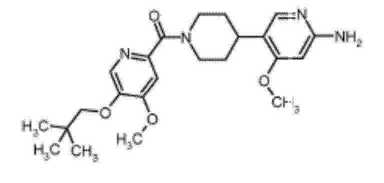 | (6-Amino-4-methoxy-3',4',5',6'-tetrahydro-2'H-[3,4']bipyridinyl-1'-yl)-[5-(2,2-dimethyl-propoxy)-4-methoxy-pyridin-2-yl]-methanone |
| **60** | 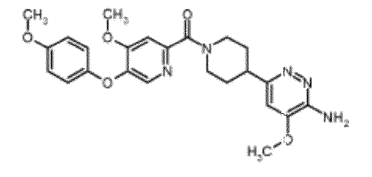 | 14-(6-Amino-5-methoxy-pyridazin-3-yl)-piperidin-1-yl]-[4-methoxy-5-(4-methoxy-phenoxy)-pyridin-2-yl]-methanone |
| **61** | 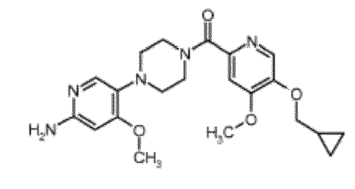 | [4-(6-Amino-4-methoxy-pyridin-3-yl)-piperazin-1-yl]-(5-cyclopropylmethoxy-4-methoxy-pyridin-2-yl)-methanone |
| **62** | 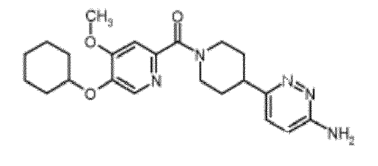 | [4-(6-Amino-pyridazin-3-yl]-piperidin-1-yl]-(5-cyclohexyloxy-4-methoxy-pyridin-2-yl]-methanone |
| **63** | 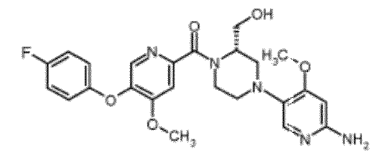 | [(S)-4-(6-Amino-4-methoxy-pyridin-3-yl)-2-hydroxymethyl-piperazin-1-yl]-[5-(4-fluoro-phenoxy)-4-methoxy-pyridin-2-yl}-methanone |
| **64** | 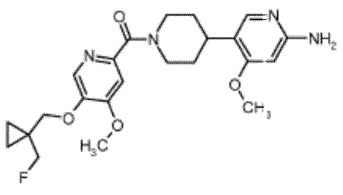 | (6-Amino-4-methoxy-3',4',5',6'-tetrahydro-2'H-[3,4']bipyridinyl-1'-yl)-[5-(1-fluoromethyl-cyclopropylmethoxy)-4-methoxy-pyridin-2-yl]-methanonse |
| **65** | 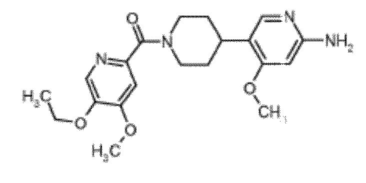 | (6-Amino-4-methoxy-3',4',5',6'-tetrahydro-2'H-[3,4']bipyridinyl-1'-yl)-(5-ethoxy-4-methoxy-pyridin-2-yl)-methanone |
| **66** | 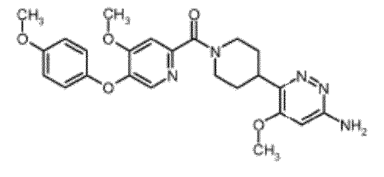 | [4-(6-Amino-4-methoxy-pyridazin-3-yl)-piperidin-1-yl]-[4-methoxy-5-(4-methoxy-phenoxy)-pyridin-2-yl]-methanone |
| **67** | 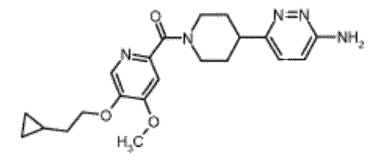 | [4-(6-Amino-pyridazin-3-yl)-piperidin-1-yl]-5-(2-cyclopropyl-ethoxy)-4-methoxy-pyridin-2-yl]-methanone |
| **68** | 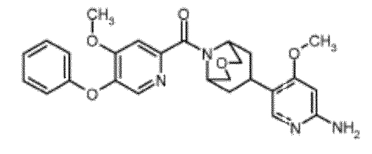 | [7-(6-Amino-4-methoxy-pyridin-3-yl)-3-oxa-9-aza-bicyclo[3.3.1]non-9-yl]-(4-methoxy-5-phenoxy-pyridin-2-yl)-methanone |
| **69** | 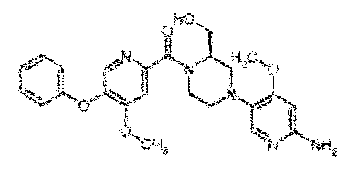 | [(R)-4-(6-Amino-4-methoxy-pyridin-3-yl)-2-hydroxymethyl-piperazin-1-yl]-(4-methoxy-5-phenoxy-pyridin-2-yl)-methanone |
| **70** | 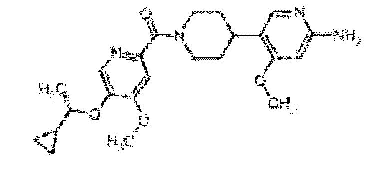 | (6-Amino-4-methoxy-3',4',5',6'-tetrahydro-2'H-[3,4']bipyridinyl-1'-yl)-[5-((S)-1-cyclopropyl-ethoxy)-4-methoxy-pyridin-2-yl]-methanone |
| **71** | 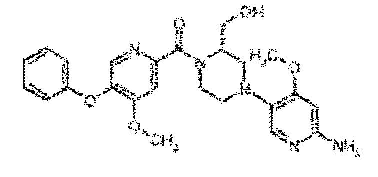 | [(S)-4-(6-Amino-4-methoxy-pyridin-3-yl)-2-hydroxymethyl-piperazin-1-yl]-(4-methoxy-5-phenoxy-pyridin-2-yl)-methanone |
| **72** | 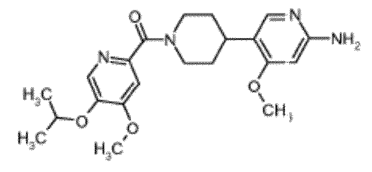 | (6-Amino-4-mothoxy-3',4',5',6'-tetrahydro-2'H-[3,4']bipyridinyl-1'-yl)-(5-isopropoxy-4-mathoxy-pyridin-2-yl)-methanone |
| **73** | 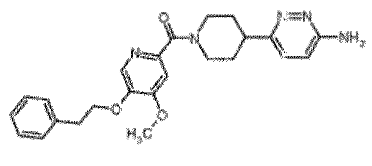 | [4-(6-Amino-pyridazin-3-yl)-piperidin-1-yl]-(4-methoxy-5-phenethyloxy-pyridin-2-yl)-methanone |
| **74** | 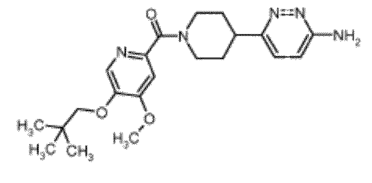 | [4-(6-Amino-pyridazin-3-yl)-piperidin-1-yl]-[5-(2,2-dimethyl-propoxy)-4-methoxy-pyridin-2-yl]-methanone |
| **75** | 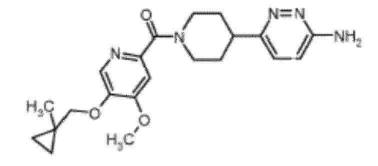 | [4-(6-Amino-pyridazin-3-yl)-piperidin-1-yl]-[4-methoxy-5-(1-methyl-cyclopropylmethoxy)-pyridin-2-yl]-methanone |
| **76** | 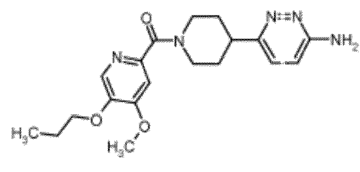 | [4-(6-Amino-pyridazin-3-yl)-piperidin-1-yl]-(4-methoxy-5-propoxy-pyridin-2-yl)-methanone |
| **77** | 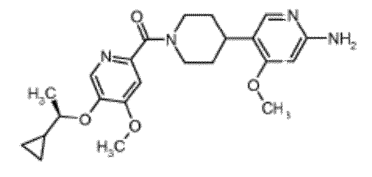 | (6-Amino-4-methoxy-3',4',5',6'-tetrahydro-2'H-[3,4']bipyridioyl-1'-yl)-[5-((R)-1-cyclopropyl-ethoxy)-4-methoxy-pyridin-2-yl]-methanone |
| **78** | 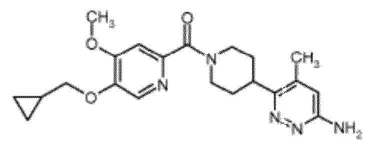 | [4-(6-Amino-4-methyl-pyridazin-3-yl)-piperidin-1-yl]-(5-cyclopropylmethoxy-4-methoxy-pyridin-2-yl)-methanone |
| **79** | 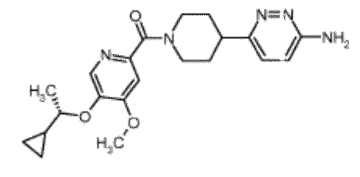 | [4-(6-Amino-pyridazin-3-yl)-piperidin-1-yl]-[5-((S)-1-cyclopropyl-ethoxy)-4-methoxy-pyridin-2-yl]-methanone |
| **80** | 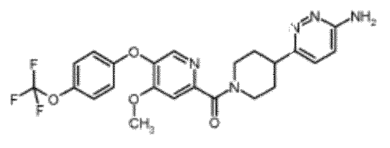 | [4-(6-Amino-pyridazin-3-yl)-piperidin-1-yl]-[4-methoxy-5-(4-trifluoromethoxy-phenoxy)-pyridin-2-yl]-methanone |
| **81** | 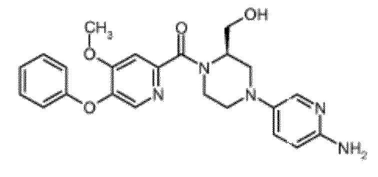 | [(R)-4-(6-Amino-pyridin-3-yl)-2-hydroxymethyl-piperazin-1-yl]-(4-methoxy-5-phenoxy-pyridin-2-yl)-methanone |
| **82** | 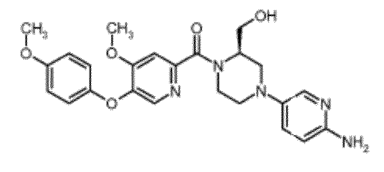 | [(R)-4-(6-Amino-pyridin-3-yl)-2-hydroxymethyl-piperazin-1-yl]-[4-methoxy-5-(4-methoxy-phenoxy)-pyridin-2-yl]-methanone |
| **83** | 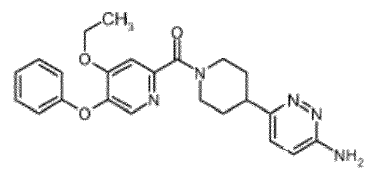 | [4-(6-Amino-pyridazin-3-yl)-piperidin-1-yl]-[5-(phenoxy)-4-ethoxy-pyridin-2-yl]-methanone |
| **84** | 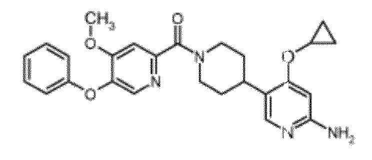 | (6-Amino-4-cyclopropoxy-3',4',5',6'-tetrahydro-2'H-[3,4']bipyridinyl-1'-yl)-[5-(phenoxy)-4-methoxy-pyridin-2-yl]-methanone |
| **85** | 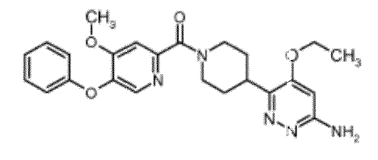 | [4-(6-Amino-4-ethoxy-pyridazin-3-yl)-piperidin-1-yl]-[4-methoxy-5-(phenoxy)-pyridin-2-yl]-methanone |
| **86** | 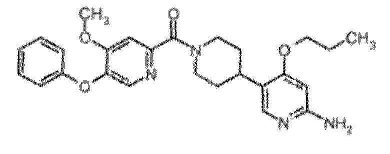 | (6-Amino-4-propoxy-3',4',5',6'-tetrahydro-2'H-[3,4']bipyridinyl-1'-yl)-[5-(phenoxy)-4-methoxy-pyridin-2-yl]-methanone |
| **87** | 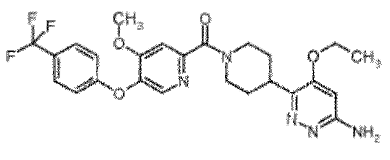 | (6-Amino-4-ethoxy-3',4',5',6'-tetrahydro-2'H-[3,4']bipyridinyl-1'-yl)-[5-(4-trifluoromethyl-phenoxy)-4-methoxy-pyridin-2-yl]-methanone |
| **88** | 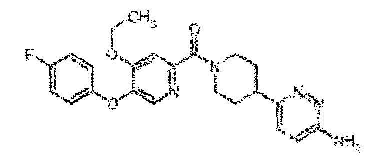 | [4-(6-Amino-pyridazin-3-yl)-piperidin-1-yl]-[5-(4-fluoro-phenoxy)-4-ethoxy-pyridin-2-yl}-methanone |
| **89** | 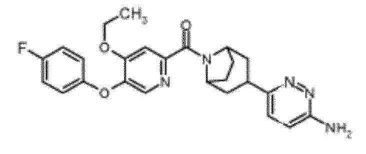 | [3-(6-Amino--pyridazin-3-yl)-8-aza-bicyclo[3.2.1]oct-8-yl]-[4-ethoxy-5-(4-fluoro-phenoxy)-pyridin-2-yl]-methanone |
| **90** | 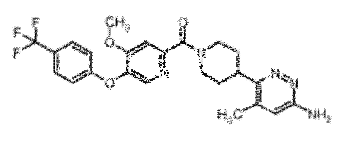 | 6-(1-{4-Methoxy-5-[4-(trifluoromethyl)phenoxy]pyridine-2-carbonyl}piperidin-4-yl)-5-methylpyridazin-3-amine |
| **91** | 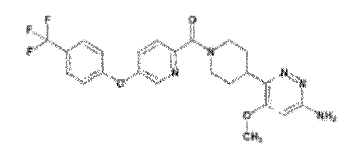 | 5-Methoxy-6-(1-{5-[4-(trifluoromethyl)-phenoxy]-pyridine-2-carbonyl}pipedin-4-yl)-pyridazin-3-amine |
| **92** | 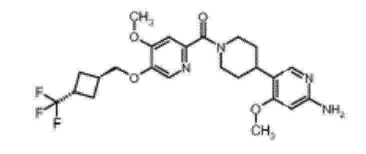 | 4-Methoxy-5-[1-(4-methoxy-5-{[trans-3-(trifluoromethyl)cyclobutyt]-methoxy}pyridine-2-carbonyl)-piperidin-4-yl]pyridin-2-amine |
| **93** | 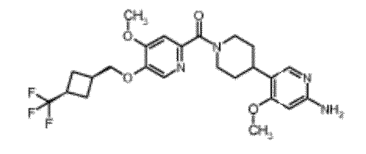 | 4-Methoxy-5-[1-(4-methoxy-5-{[(cis-3-(trifluoromethyl)-cyclobutyl]methoxy}-pyridine-2-carbonyl)piperidin-4-yl]pyridin-2-amine |
| **94** | 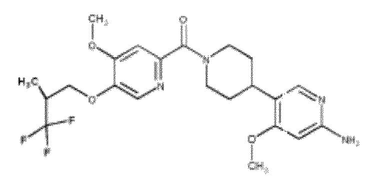 | 4-Methoxy-5-(1-{4-methoxy-5-[(2)-3,3,3-trifluoro-2-methylpropoxy}-pyridine-2-carbonyl}piperidin-4-yl)pyridin-2-amine |
| **95** | 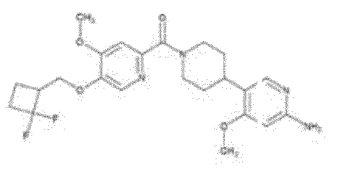 | 5-(1-{5-[(2,2-Difluorocyclobutyl)-methoxy]-4-methoxy-pyridine-2-carbonyl}-piperidin-4-yl)-4-methoxypyridin-2-amine |

or a pharmaceutically acceptable salt, solvate or hydrate thereof.

The TRPC6 inhibitor is selected from the group consisting of compounds 1 to 54 in the Table below:

| **Cpd No.** | **Structure** | **Structure Name** |
|---|---|---|
| **1** | 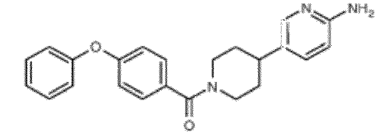 | 5-[1-(4-phenoxybenzoyl)piperidin-4-yl]pyridin-2-amine |
| **2** | 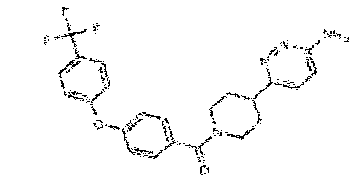 | 6-(1-{4-[4-(trifluoromethyl)phenoxy]benzoyl} piperidin-4-yl)pyridazin-3-amine |
| **3** | 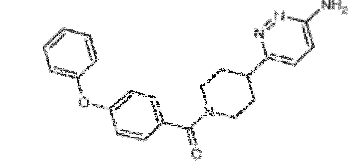 | 6-[1-(4-phenoxybenzoyl)piperidin-4-yl]pyridazin-3-amine |
| **4** | 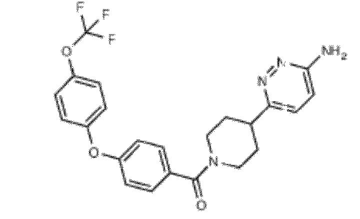 | 6-(1-{4-[4-(trifluoromethoxy)phenoxy]benzoy l}piperidin-4-yl)pyridazin-3-amine |
| **5** | 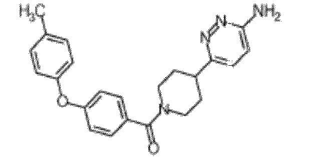 | 6-{1-[4-(4-methylphenoxy)benzoyl]piperidin-4-yl}pyridazin-3-amine |
| **6** | 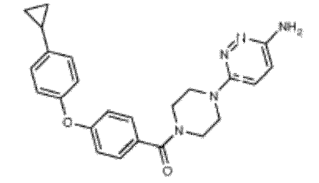 | 6-{4-[4-(4-cyclopropylphenoxy)benzoyl]piper azin-1-yl}pyridazin-3-amine |
| **7** | 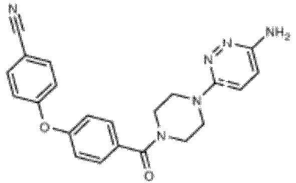 | 4-{4-[4-(6-aminopyridazin-3-yl)piperazine-1-carbonyl]phenoxy}benzonitrile |
| **8** | 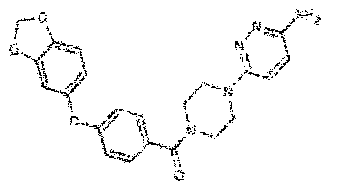 | 6-{4-[4-(2H-1,3-benzodioxol-5-yloxy)benzoyl]piperazin-1-yl}pyridazin-3-amine |
| **9** | 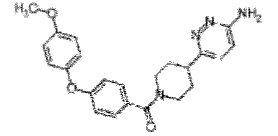 | 6-{1-[4-(4-methoxyphenoxy)benzoyl]piperidi n-4-yl)pyridazin-3-amine |
| **10** | 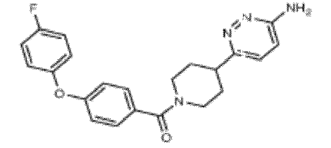 | 6-{1-[4-(4-fluorophenoxy)benzoyl]piperidin-4-yl}pyridazin-3-amine |
| **11** | 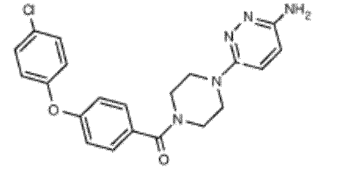 | 6-(4-[4-(4-chlorophenoxy)benzoyl]piperazin-1-yl}pyridazin-3-amine |
| **12** | 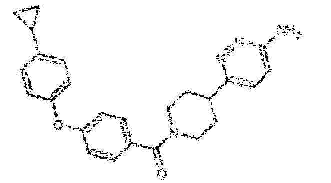 | 6-{1-[4-(4-cyclopropylphenoxy)benzoyl]piper idin-4-yl}pyridazin-3-amine |
| **13** | 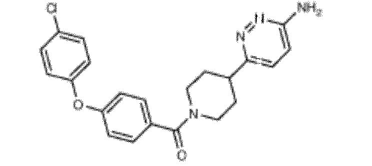 | 6-{1-14-(4-chlorophenoxy)benzoyl]pipridin-4-yl)pyridazin-3-amine |
| **14** | 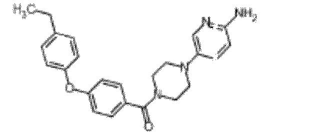 | 5-{4-[4-(4-ethylphenoxy)benzoyl]piperazin-1-yl}pyridin-2-amine |
| **15** | 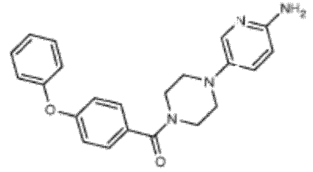 | 5-[4-(4-phenoxybenzoyl)piperazin-1-yl]pyridin-2-amine |
| **16** | 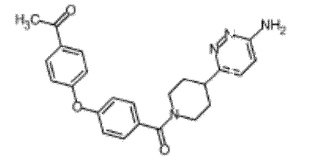 | 1-(4-{4-{4-(6-aminopyridazin-3-yl)piperidine-1-carbonyl]phenoxy}phenyl)ethan-1-one |
| **17** | 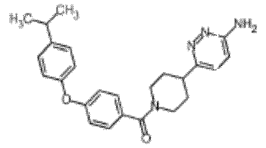 | 6-(1-{4-[4-(propan-2-yl)phenoxy]benzoyl}piperidin-4-yl)pyridazin-3-amine |
| **18** | 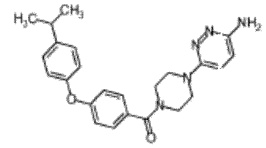 | 6-(4-{4-[4-(propan-2-yl)phenoxy]benzoyl}piperazin- 1-yl)pyridazin-3-amine |
| **19** | 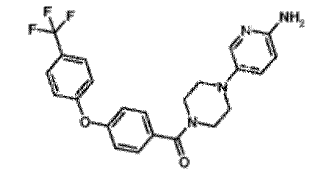 | 5-(4-{4-[4-(trifluoromethyl)phenoxy]benzoyl} piperazin-1-yl)pyridin-2-amine |
| **20** | 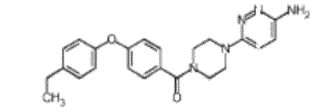 | 6-{4-[4-(4-ethylphenoxy)benzoyl]piperazin-1-yl}pyridazin-3-amine |
| **21** | 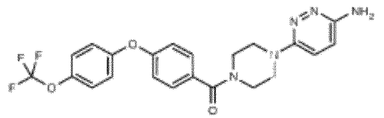 | 6-(4-{4-[4-(trifluoromethoxy)phenoxy]benzoy l}piperazin-1-yl)pyridazin-3-amine |
| **22** | 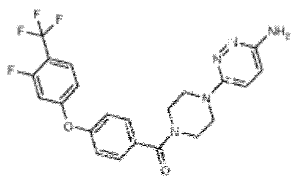 | 6-(4-{4-[3-fluoro-4-(trifluoromethyl)phenoxy]benzoyl} piperazin-1-yl)pyridazin-3-amine |
| **23** | 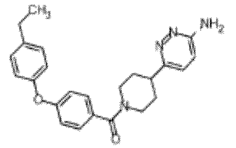 | 6-{1-[4-(4-ethylphenoxy)benzoyl]piperidin-4-yl}pyridazin-3-amine |
| **24** | 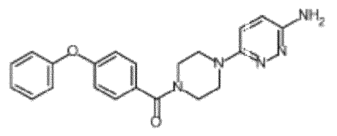 | 6-[4-(4-phenoxybenzoyl)piperazin-1-yl]pyridazin-3-amine |
| **25** | 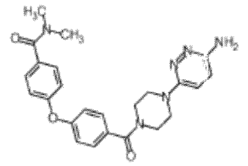 | 4-{4-[4-(6-aminopyridazin-3-yl)piperazine-1-carbonyllphenoxy}-N,N-dimethylbenzamide |
| **26** | 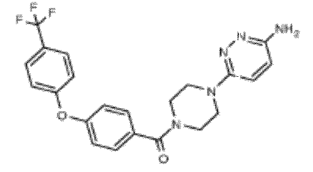 | 6-(4-{4-[4-(trifluorotnethyl)phenoxy]benzoyl} piperazin-1-yl)pyridazin-3-amine |
| **27** | 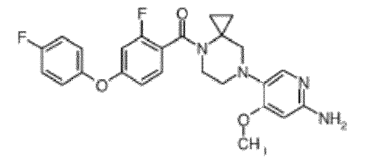 | 5-{4-[2-Fluoro-4-(4-fluorophenoxy)benzoy]-4,7-diazaspiro[2.5]octan-7-yl}-4-methoxypyridin-2-amine |
| **28** | 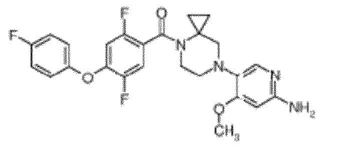 | 5-{4-[2,5-Difluoro-4-(4-fluorophenoxy)benzoyl]-4,7-diazaspiro[2.5]octan-7-yl}-4-methoxypyridin-2-amine |
| **29** | 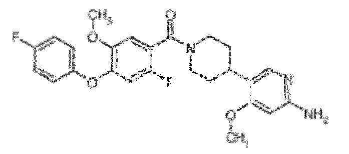 | 5-{1-[2-Fluoro-4-(4-fluorophenoxy)-5-methoxybenzoyl]piperidin-4-yl}-4-methoxypyridin-2-amine |
| **30** | 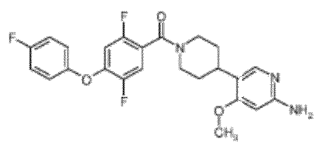 | 5-{1-[2,5-difluoro-4-(4-fluorophenoxy)benzoyl]piperidin -4-yl}-4-methoxypyridin-2-amine |
| **31** | 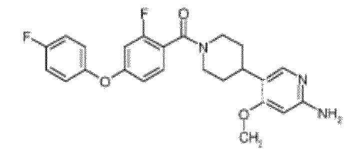 | 5-{1-[2-Fluoro-4-(4-fluorophenoxy)benzoyl]piperidine-4-yl}-4-methoxypyridin-2-amine |
| **32** | 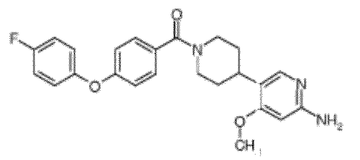 | 5-{1-[4-(4-Fluorophenoxy)benzoyl]piperidin-4-yl}-4-methoxypyridin-2-amine |
| **33** | 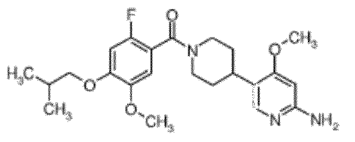 | 5-{1-[2-Fluoro-5-methoxy-4-(2-methylpropoxy)benzoyl]-piperidin-4-yl]-4-methoxypyridin-2-amine |
| **34** | 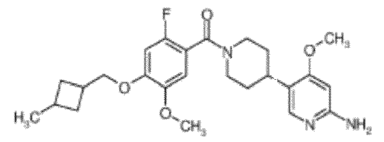 | 5-(1-{2-Fluoro-5-methoxy-4-[(3-methylcyclobutyl)methoxyjbenzoy 1}piperidin-4-yl)-4-methoxypyridin-2-amine |
| **35** | 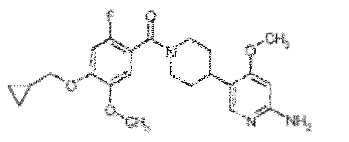 | 5-{1-[4-(Cyclopropylmethoxy)-2-fluoro-5-methoxybenzoyl]piperidin-4-yl}-4-methoxypyridin-2-amine |
| **36** | 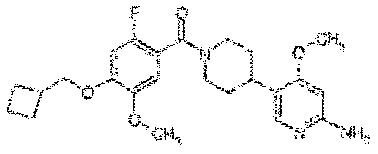 | 5-{1-[4-(Cyclobutylmethoxy)-2-fluoro-5-methoxybenzoyl]piperidin-4-yl}-4-methoxypyridin-2-amine |
| **37** | 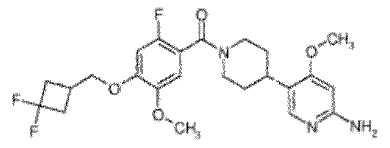 | 5-(1-{4-[(3,3-Difluorocyclobutyl)methoxy]-2-fluoro-5-methoxybenzoyl}piperidin-4-yl)-4-methoxypyridin-2-amine |
| **38** | 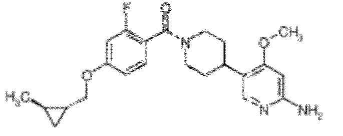 | 5-[1-(2-Fluoro-4-{[*trans-2-*methylcyclopropyl]methoxy}-benzoyl)piperidin-4-yl]-4-methoxypyridin-2-amine |
| **39** | 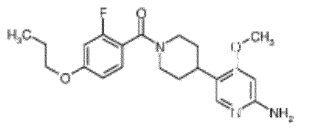 | 5-[1-(2-Fluoro-4-propoxybenzoyl)piperidin-4-yl]-4-methoxypyridin-2-amine |
| **40** | 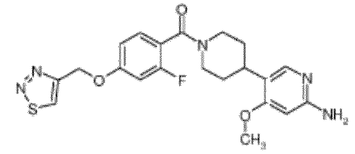 | 5-(1-{2-Fluoro-4-[(1,2,3-thiadiazol-4-yl)methoxy]benzoyl}piperidin-4-yl)-4-methox ypyridin-2-amine |
| **41** | 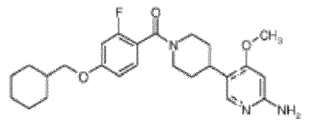 | 5-{1-[4-(Cyclohexylmethoxy)-2-fluorobenzoyl]piperidin-4-yl}-4-methoxypyridin-2-amine |
| **42** | 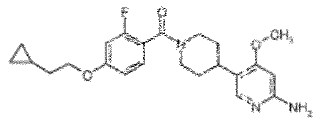 | 5-{1-[4-(2-Cyclopropylethoxy)-2-fluorobenzoyl]piperidin-4-yl}-4-methoxypyridin-2-amine |
| **43** | 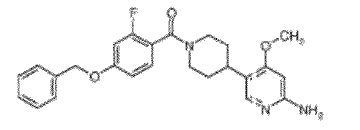 | 5-{1-{4-(Benzyloxy)-2-fluorobenzoyl]piperidin-4-yl}-4-methoxypyridin-2-amine |
| **44** | 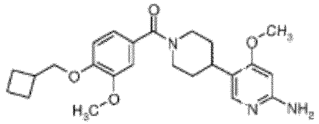 | 5-{1-[4-(Cyclobutylmethoxy)-3-methoxybenzoyl]piperidin-4-yl}-4-methoxypyridin-2-amine |
| **45** | 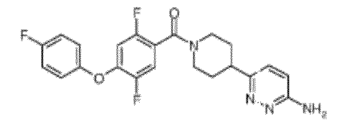 | 6-{1-[2,5-Difluoro-4-(4-fluorophenoxy)benzoyl]piperidin-4-yl}pyridazin-3-amine |
| **46** | 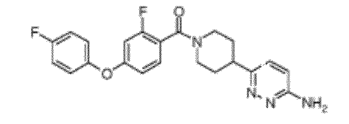 | 6-{1-[2-Fluoro-4-(4-fluorophenoxy)benzoyl]piperidin -4-yl}pyridazin-3-amine |
| **47** | 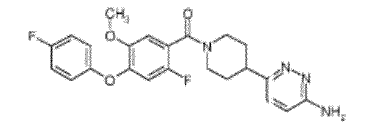 | 6-{1-[2-Fluoro-4-(4-fluorophenoxy)-5-methoxybenzoyl]piperidin-4-yl]pyridazin-3-amine |
| **48** | 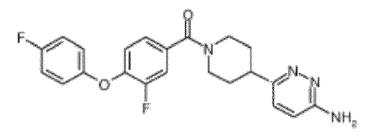 | 6-{1-[3-Fluoro-4-(4-fluorophenoxy)benzoyl]piperidin-4-yl}pyridazin-3-amine |
| **49** | 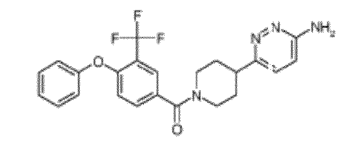 | 6-{1-(4-Phenoxy-3-(trifluoromethyl)benzoyl]piperidin-4-yl}pyridazin-3-amine |
| **50** | 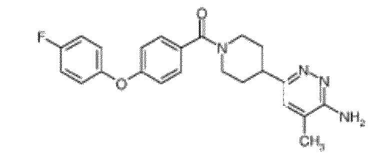 | 6-{1-[4-(4-Fluorophenoxy)benzoyl]piperidin-4-yl}-4-methylpyridazin-3-amine |
| **51** | 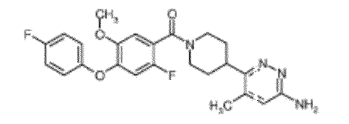 | 6-{1-[2-Fluoro-4-(4-fluorophenoxy)-5-methoxybenzoyl]piperidin-4-yl}-5-methylpyridazin-3-amine |
| **52** | 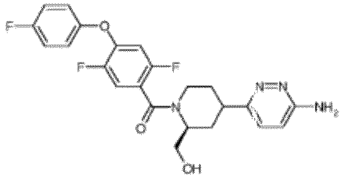 | [(2*S*)-4-(6-Aminopyridazin-3-yl)-1-[2.5-difluoro-4-(4-fluorophenoxy)benzoyl]piperidin-2-yl]methanol |
| **53** | 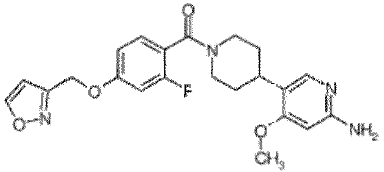 | 5-(1-(2-Fluoro-4-[(1,2-oxazol-3-yl)methoxy]benzoyl}piperidin-4-yl)4-methoxypyridin-2-amine, |
| **54** | 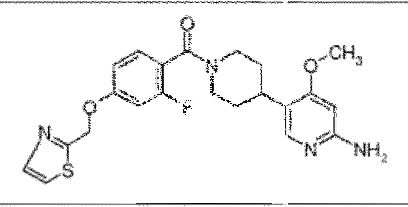 | 5-(1-{2-Fluoro-4-[(1,3-thiazol-2-yl)methoxy]benzoyl}piperidin-4-yl)-4-methoxypyridin-2-amine |

or a pharmaceutically acceptable salt, solvate or hydrate thereof.

Unless otherwise stated, the following terms used in this document, including the description and claims, have the definitions given below.

Those skilled in the art will recognize, or be able to ascertain, using not more than routine experimentation, many equivalents to the specific embodiments of the invention described herein. Such equivalents are intended to be encompassed by the present invention.

It is to be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

The following sequence has been used in the present invention:

| S E Q I D | What | Sequence |
|---|---|---|
| 1 | Uniprot no. Q9Y210\| HUMAN Short transient receptor potential channel 6 | MSQSPAFGPRRGSSPRGAAGAAARRNESQDYLLMDSELGEDGCPQAP LPCYGYYPCFRGS DNRLAHRRQTVLREKGRRLANRGPAYMFSDRSTSLSIEEERFLDAAEYG NIPVVRKMLEE CHSLNVNCVDYMGQNALQLAVANEHLEITELLLKKENLSRVGDALLLAIS KGYVRIVEAI LSHPAFAEGKRLATSPSQSELQQDDFYAYDEDGTRFSHDVTPIILAAHCQ EYEIVHTLLR KGARIERPHDYFCKCNDCNQKQKHDSFSHSRSRINAYKGLASPAYLSLS SEDPVMTALEL SNELAVLANIEKEFKNDYKKLSMQCKDFVVGLLDLCRNTEEVEAILNGDV ETLQSGDHGR PNLSRLKLAIKYEVKKFVAHPNCQQQLLSIWYENLSGLRQQTMAVKFLVV LAVAIGLPFL ALIYWFAPCSKMGKIMRGPFMKFVAHAASFTIFLGLLVMNAADRFEGTKL LPNETSTDNA KQLFRMKTSCFSWMEMLIISWVIGMIWAECKEIWTQGPKEYLFELWNML DFGMLAIFAAS FIARFMAFWHASKAQSIIDANDTLKDLTKVTLGDNVKYYNLARIKWDPSD PQIISEGLYA IAVVLSFSRIAYILPANESFGPLQISLGRTVKDIFKFMVIFIMVFVAFMIGMF NLYSYYI GAKQNEAFTTVEESFKTLFWAIFGLSEVKSVVINYNHKFIENIGYVLYGVY NVTMVIVLL NMLIAMINSSFQEIEDDADVEWKFARAKLWFSYFEEGRTLPVPFNLVPSP KSLFYLLLKL KKWISELFQGHKKGFQEDAEMNKINEEKKLGILGSHEDLSKLSLDKKQVG HNKQPSIRSS |
| | | EDFHLNSFNNPPRQYQKIMKRLIKRYVLQAQIDKESDEVNEGELKEIKQDI SSLRYELLE EKSQNTEDLAELIRELGEKLSMEPNQEETNR |

### EXAMPLES

### Example 1

### Wire-induced femoral artery injury

All animal studies were performed with permission of the government of Upper Bavaria (55.2-1-54-2532-17-14) and according to international guidelines. Wire injury was performed in in 8 to 12 weeks old C57BL/6J (Jackson Laboratories, Bar Habor, ME, USA) and *Trpc6*^{*-*/*-*} mice which have been described previously (59). Wire-injury was performed in 8 to 12 weeks old C57BL/6J mice as described previously (60). In brief, after skin incision, the left femoral artery was exposed by blunt dissection and a 0.38 mm guidewire (No. C-SF-15-15, COOK, Bloomington, IN, USA) was inserted towards the iliac artery. To denude and dilate the femoral artery, the wire was left in place for 1 minute. Surgery was carried out using a dissection microscope (Stemi200C, Zeiss, Jena, Germany). Mice were sacrificed after 3, 7, or 14 days by intraperitoneal administration of an overdose of pentobarbital. To avoid blood inside the femoral arteries mice were perfused with PBS via the left ventricle and the descending aorta. Both femoral arteries (injured and non-injured) were carefully removed, snap-frozen in liquid nitrogen and stored at -80 °C until further analysis. For histological analyses, femoral arteries were fixed in 4 % paraformaldehyde.

### Histology and morphometry

Femoral arteries were embedded in paraffin. 2 µm serial cross-sections were cut starting from the ligation as reference point using a microtome (HM 340E, Thermo Fisher Scientific, Waltham, MA, USA). Sections were mounted on microscope slides, deparaffinized and rehydrated in graded alcohol. Ten to 15 sections per femoral artery at 25 µm intervals were stained with haematoxylin and eosin and embedded in pertex (Medite Cancer Diagnostics, Chicago, IL, USA). Stained tissue sections were digitalized (Leica DMRB and Leica DFC450C, Leica Camera AG, Wetzlar, Germany) and morphometrically analyzed by an investigator blinded to *Trpc6* genotype using ImageJ v. 1.47. Lumen area as well as circumferences of the internal (IEL) and external elastic lamina (EEL) were measured. Intima and media area as well as neointima/media ratio were calculated.

### Immunoblotting

Trpc6 protein expression was analyzed in non-injured and injured (3, 7 or 14 days) femoral arteries of C57BL/6J mice. Snap-frozen femoral arteries were manually disrupted in 40µl radioimmunoprecipitation assay (RIPA) buffer (Cell Signaling Technology, Denvers, MA, USA). After 3x30 s of sonification cell lysate was separated from cell debris by centrifugation at 4 °C. Protein concentration was determined using a BCA assay (Pierce, Thermo Fisher, Schwerte, Germany). Laemmli buffer (4x, Sigma-Aldrich, St. Louis, MO, USA) was added and the mixture was incubated at 95°C for 5min. 10µg protein of each sample was loaded on a 4-20% gradient gel. Gel electrophoresis was performed using a Mini-PROTEAN Tetra Cell system (Bio-Rad Laboratories, Hercules, CA, USA) and immunoblotting was performed using a transfer system (Trans-Blot^{®} Turbo, Bio-Rad Laboratories, Hercules, CA, USA) according to the manufacturer's instructions. After blocking with 5% low fat milk in phosphate buffered saline with Tween (PBST), membranes were incubated overnight at 4°C with the primary anti-Trpc6 antibody (rabbit AK 861 affinity purified, 1:200 (66)). Membranes were washed with PBST three times for 5min and anti-rabbit IgG-HRP linked secondary antibody (Cell Signaling Technology, Danvers, MA, USA, 7074S, 1:100,000) was applied for 1h at room temperature. Membranes were rinsed with PBST three times for 10min and incubated with enhanced chemiluminescence substrate (Thermo Fisher Scientific SuperSignal West Dura Extended Duration Substrate; Life Technologies, Carlsbad, CA, USA). An ImageQuant LAS 4000 system (GE Healthcare Life Sciences, Pittsburgh, PA, USA) was used for luminescence detection. Signal intensities were quantified using ImageQuant TL (GE Healthcare Life Sciences, Pittsburgh, PA, USA) and normalized to signal intensities for Gapdh in the same sample.

### Cell culture

Human aortic smooth muscle cells (hAoSMC) were purchased from PromoCell (Heidelberg, Germany) and cultured at 37 °C in a humidified 5 % CO₂ atmosphere in the recommended cell culture medium (PromoCell, Heidelberg, Germany).

### Cell migration assay

For scratch wound assays, cells were seeded in 24 well plates at a density of 100,000 cells per well. After they had grown to confluence for 24h the cell monolayer was scratched in the middle with a 200µl pipette tip. Cells were washed with PBS and incubated in growth medium supplemented with OAG (Sigma-Aldrich, St. Louis, MO, USA) at final concentration of 100µM or SAR 7334 (Bio-Techne, Wiesbaden, Germany) at a final concentration of 100nM. 1% DMSO was used as control. Wound area was measured directly after the scratch (t₀) and after 6h (t₁), 8h (t₂) and 12h (t₃) with a phase-contrast microscope (Axiovert 100, Zeiss, Oberkochen, Germany) and assessed with ImageJ version 1.47v. Wound reclosure was measured as Δt₀-t₁ (6h), Δt₀-t₂ (8h), and Δt₀-t₃ (12h) in pixels. Scratch assays were performed in triplicates. For radius assays, 24-well plates were coated with gelatin solution (Pelobiotech, Planegg, Germany). Culture cylinders (total diameter 4mm, inner diameter 2mm; Bioptechs, Butler, PA, USA) were then placed in the middle of the well and cells were seeded at a density of 100,000 cells per well around the cylinders. 20µL of SAR 7334 with a final concentration of 100nM in gelatin were added to the inner of the cylinders. 1% DMSO was used as control. After 24h of incubation cylinders were removed and pictures were acquired immediately (t₀) and after 48h (t₁) using a phase-contrast microscopy system (Axiovert 100, Zeiss, Oberkochen, Germany). Wound reclosure was measured as Δt₀-t₁ (48h) in pixels. Measurements were performed in duplicates.

### Cell proliferation assay

Cell proliferation was assessed using of a colorimetric bromodeoxyuridine (BrdU) enzyme-linked immunosorbent assay (ELISA) according to manufacturer's protocol (Roche Applied Science, Penzberg, Germany). Briefly, cells were seeded in 96 well plates at a density of 10,000 cells per well in starving medium, i.e., lacking FBS and growth factors. After 24h, the starving medium was replaced by growth medium which was either supplemented with 1-Oleoyl-2-acetyl-sn-glycerol (OAG; Sigma-Aldrich, St. Louis, MO, USA) at a final concentration of 100 µM, SAR 7334 (Tocris Bioscience, Bristol, UK) at a final concentration of 100nM or with 1% of DMSO which was used as vehicle. After incubation for another 24h, 10µl of BrdU solution were added. Absorbance was measured at 370nm with a reference wavelength of 492nm in the Infinite M200 PRO microplate reader using the iControl v1.10 software (both Tecan Group, Männedorf, Switzerland) after further 24h. Measurements were performed in triplicates.

### Analysis of binary restenosis and late lumen loss in humans

Available genotyping data from previous genome-wide association studies and subsequent analyses (67, 68) were used to correlate rs2513192 genotype and risk of binary restenosis. Rs2513192 genotype was available in 4,247 individuals and 6,028 lesions. Characteristics are depcited in table S2. Information on binary restenosis, defined as a diameter reduction of 50 % or more, was available in 3,068 (72.2%) individuals (one or more lesions with binary restenosis) and 4,279 (71 %) lesions. A logistic regression model with computation of odds ratios (OR) and 95% Confidence Intervals (CI) was used to assess the association of rs2513192 genotype with the primary endpoint binary restenosis. To avoid overfitting, selection of covariates in the logistic regression model was performed using the least absolute shrinkage and selection operator regression method after entering all baseline and procedural characteristics as candidates (R package "glmnet", version 2.0-13). The resulting variables for the logistic regression model were age, gender, body mass index, diabetes mellitus, hypertension, smoking status, hypercholesterolemia, previous myocardial infarction, previous coronary artery bypass graft surgery, clinical presentation with acute coronary syndrome, multivessel disease, lesion complexity, chronic occlusion, restenotic lesion, lesion length, reference diameter before stent implantation, stenosis before implantation, implantation of drug eluting stents as well as total stented length.

### Statistical analyses

Distribution of data was assessed using Kolmogorov-Smirnov test. Unless otherwise stated, normally distributed data was analyzed using Student's unpaired/paired t-test. Not normally distributed data was analyzed with Mann-Whitney-test. Categorical data was analyzed using chi-square test. P-values < 0.05 were regarded significant. GraphPad Prism version 8.0.1 for Mac OS X (GraphPad Software, La Jolla, CA, USA) was used.

### Example 2

Expression of Trpc6 in the femoral artery of the mouse after vascular damage. NI, non-injured (undamaged vessel), 3d/7d/14d, 3/7/14 days after vessel damage; a.u., arbitrary units; Gapdh, loading control.

### Example 3

### Cell migration assay

For scratch wound assays, cells were seeded in 24 well plates at a density of 100,000 cells per well. After they had grown to confluence for 24h the cell monolayer was scratched in the middle with a 200µl pipette tip. Cells were washed with PBS and incubated in growth medium supplemented with OAG (Sigma-Aldrich, St. Louis, MO, USA) at final concentration of 100µM or SAR 7334 (Bio-Techne, Wiesbaden, Germany) at a final concentration of 100nM. 1% DMSO was used as control. Wound area was measured directly after the scratch (t0) and after 6h (t1), 8h (t2) and 12h (t3) with a phase-contrast microscope (Axiovert 100, Zeiss, Oberkochen, Germany) and assessed with ImageJ version 1.47v. Wound reclosure was measured as Δt0-t1 (6h), Δt0-t2 (8h), and Δt0-t3 (12h) in pixels. Scratch assays were performed in triplicates. For radius assays, 24-well plates were coated with gelatin solution (Pelobiotech, Planegg, Germany). Culture cylinders (total diameter 4mm, inner diameter 2mm; Bioptechs, Butler, PA, USA) were then placed in the middle of the well and cells were seeded at a density of 100,000 cells per well around the cylinders. 20µL of SAR 7334 with a final concentration of 100nM in gelatin were added to the inner of the cylinders. 1% DMSO was used as control. After 24h of incubation cylinders were removed and pictures were acquired immediately (t0) and after 48h (t1) using a phase-contrast microscopy system (Axiovert 100, Zeiss, Oberkochen, Germany). Wound reclosure was measured as Δt0-t1 (48h) in pixels. Measurements were performed in duplicates.

### Example 4

### Analysis of binary restenosis and late lumen loss in humans

Available genotyping data from previous genome-wide association studies and subsequent analyses (67, 68) were used to correlate rs2513192 genotype and risk of binary restenosis. Rs2513192 genotype was available in 4,247 individuals and 6,028 lesions. Characteristics are depcited in table S2. Information on binary restenosis, defined as a diameter reduction of 50 % or more, was available in 3,068 (72.2%) individuals (one or more lesions with binary restenosis) and 4,279 (71 %) lesions. A logistic regression model with computation of odds ratios (OR) and 95% Confidence Intervals (CI) was used to assess the association of rs2513192 genotype with the primary endpoint binary restenosis. To avoid overfitting, selection of covariates in the logistic regression model was performed using the least absolute shrinkage and selection operator regression method after entering all baseline and procedural characteristics as candidates (R package "glmnet", version 2.0-13). The resulting variables for the logistic regression model were age, gender, body mass index, diabetes mellitus, hypertension, smoking status, hypercholesterolemia, previous myocardial infarction, previous coronary artery bypass graft surgery, clinical presentation with acute coronary syndrome, multivessel disease, lesion complexity, chronic occlusion, restenotic lesion, lesion length, reference diameter before stent implantation, stenosis before implantation, implantation of drug eluting stents as well as total stented length.

### Example 5

### Manufacture of a stent comprising a coating with a TRPC6 inhibitor

TRPC6 inhibitor SAR7334 was dissolved in a solvent (e.g. dichloromethane or tetrahydrofuran) to give a coating composition comprising SAR7334 in a concentration of 1% by weight based on 100% by weight of the coating composition. A stainless steel microporous coronary stent was dipped into the coating composition in order to prepare a coating via dip coating. After removing from the coating composition, the stent was dried in a stream of warm air to give a coating on the stent, wherein the TRPC6 inhibitor SAR 7334 is localized on the surface and/or in pores of the stent. Alternatively, the stent may be coated by spray coating, e.g. using the stent-coating machine described by Wessely and coworkers (Wessely R. et al., 2005, Arterioscler Thromb Vasc Biol 25: 748 - 753, also see above).

## Claims

1. A medical device suitable for being inserted into a lumen of an anatomic structure of a subject, said medical device comprising means for administration of a TRPC6 inhibitor, wherein said means comprises the TRPC6 inhibitor;
wherein:
the medical device is selected from the group consisting of a stent, a balloon, a microcatheter or a bioabsorbable scaffold; and/or
said means is a coating of the medical device.

2. The medical device of any one of the preceding claims, wherein the lumen of the anatomic structure is a lumen of a blood vessel, the esophagus, trachea or urethra, preferably a blood vessel, more preferably an artery.

3. The medical device of any one of the preceding claims, wherein the subject is a mammal, preferably a human.

4. The medical device of any one of the preceding claims, wherein said means is a coating of the medical device, and the medical device is a catheter.

5. The medical device of any one of the preceding claims, wherein the coating further comprises a polymer; and/or wherein the coating further comprises a solvent.

6. The medical device of any one of the preceding claims,
(a) wherein the TRPC6 inhibitor has the following formula (I): 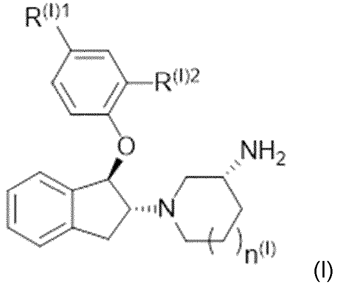 wherein:
R(^{I)1} and R^{(I)2} are each independently selected from the group consisting of halogen (preferably F, Cl, or Br), -CN and -NO₂; and
n^{(I)} is 0, 1 or 2;
or a pharmaceutically acceptable salt, solvate or hydrate thereof; preferably wherein the TRPC6 inhibitor is 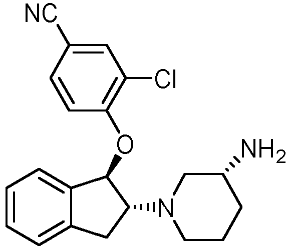 (SAR7334) or a pharmaceutically acceptable salt, solvate or hydrate thereof;
(b) wherein the TRPC6 inhibitor has the following formula (II): 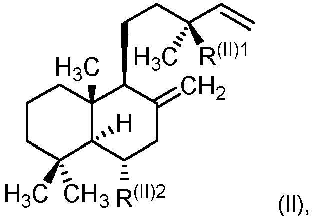 **wherein:**
R^{(II)1} and R^{(II)2} are each independently selected from the group consisting of -OH, 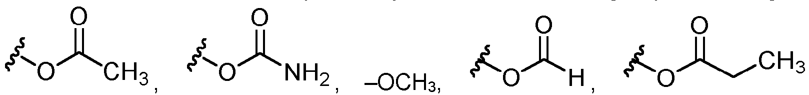 and 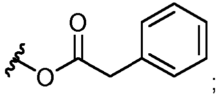
or a pharmaceutically acceptable salt, solvate or hydrate thereof;
(c) wherein the TRPC6 inhibitor has the following formula (III): 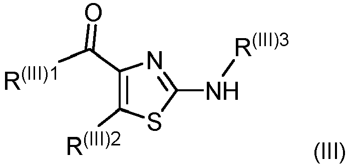 wherein:
R^{(III)1} is independently selected from the group consisting of 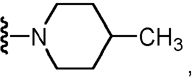 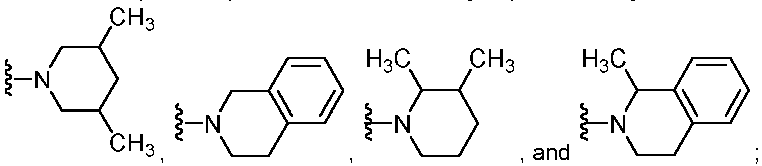
R^{(III)2} is independently selected from the group consisting of -H, -CH₃ and halogen (preferably F, Cl, Br); and
R^{(III)3} is independently selected from the group consisting of 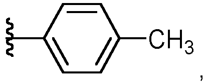 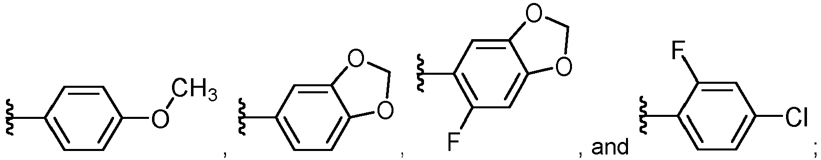
or a pharmaceutically acceptable salt, solvate or hydrate thereof;
(d) wherein the TRPC6 inhibitor has the following formula (IV): 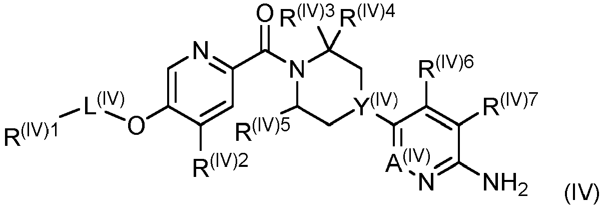 wherein:
L^{(IV)} is absent or is methylene or ethylene;
Y^{(IV)} is CH or N;
A^{(IV)} is CH or N;
R^{(IV)1} is selected from the group consisting of:
C₁₋₆alkyl optionally substituted with 1 to 3 groups independently selected from the group consisting of halo, C₃₋₆cycloalkyl and OC₃₋₆cycloalkyl;
phenyl optionally substituted with 1 to 3 groups independently selected from the group consisting of CF₃, halo, C₃₋₆cycloalkyl, OC₃₋₆cycloalkyl, OC₁₋₆alkyl optionally substituted with one to three halo; and
C₃₋₆cycloalkyl optionally substituted with 1 to 3 groups independently selected from the group consisting of halo and C₁₋₆alkyl optionally substituted with 1 to 3 halo;
R^{(IV)2} is selected from the group consisting of H, C₁₋₆alkyl, OCF₃, C₃₋₆cycloalkyl, OC₁₋₆alkyl; OC₃₋₆cycloalkyl;
R^{(IV)3} is selected from the group consisting of H, C₁₋₆alkyl, C₃₋₆cycloalkyl, OC₃₋₆cycloalkyl; wherein each of the C₁₋₆alkyl, C₃₋₆cycloalkyl, OC₃₋₆cycloalkyl of the R^{(IV)3} group may be optionally substituted with one to three groups each independently selected from the group consisting of halo, OH, OC₁₋₆alkyl, SC₁₋₆alkyl, N(C₁₋₆alkyl)₂; and
wherein one to three carbon atoms of the C₁₋₆alkyl of the R^{(IV)3} group may optionally be replaced by one or two moieties selected from the group consisting of NH, (NC₁₋₆alkyl), O, and S;
R^{(IV)4} and R^{(IV)5} are each independently selected from the group consisting of H or C₁₋₆alkyl;
R^{(IV)3} and R^{(IV)4} can together with the atom to which they are attached join to form a 3 to 9-membered carbocyclyl ring which optionally may contain one to three heteroatoms selected from the group consisting of N, O, and S; or
R^{(IV)3} and R^{(IV)5} can together form a 3 to 9-membered bicyclic ring which optionally may contain one to three heteroatoms selected from the group consisting of N, O, and S;
R^{(IV)6} is selected from the group consisting of H, C₁₋₆alkyl, CN, CF₃, OCF₃, C₃₋₆cycloalkyl, OC₁₋₆alkyl, and OC₃₋₆cycloalkyl;
R^{(IV)7} is selected from the group consisting of H and OC₁₋₆alkyl;
or a pharmaceutically acceptable salt, solvate or hydrate thereof; and/or
(e) wherein the TRPC6 inhibitor has the following formula (V): 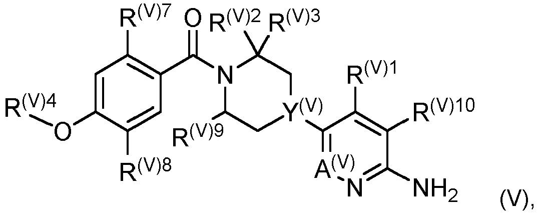 wherein:
Y^{(V)} is CH or N;
A^{(V)} is CH or N;
R^{(V)1} is H, C₁₋₃alkyl, or OC₁₋₃alkyl;
R^{(V)2} is H, C₁₋₃alkyl or C₃₋₆cycloalkyl wherein each of the C₁₋₃alkyl or C₃₋₆cycloalkyl of the R² group may be optionally substituted with OH, halo or OC₁₋₃alkyl;
R^{(V)3} is H or C₁₋₃alkyl;
R^{(V)2} and R^{(V)3} together with the carbon to which they are attached may optionally join to form a 3- to 6-membered carbocyclic ring;
R^{(V)4} represents
C₁₋₆alkyl which may optionally be substituted with one to three groups independently selected from the group consisting of halo,
C₃₋₆cycloalkylmethyl and C₃₋₆cycloalkylethyl, where the C₃₋₆cycloalkyl of the C₃₋₆cycloalkylmethyl and C₃₋₆cycloalkylethyl may optionally be substituted with one to three groups independently selected from the group consisting of halo and methyl,
1,2,3-thiadiazoylmethyl, thiazoylmethyl, isoxazolylmethyl or
a group of formula 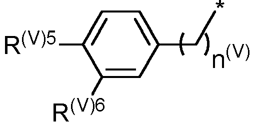
wherein n^{(V)} is 0 or 1;
R^{(V)3} is selected from the group consisting of H, halo, CF₃, OCF₃, CN, C₁₋₃alkyl, OC₁₋₃alkyl, C₃₋₆cycloalkyl; wherein each of the C₁₋₃alkyl and OC₁₋₃alkyl of the R^{(V)5} groups may optionally be substituted with one to three groups each independently selected from the group consisting of halo, oxo, NH₂, NH(C₁₋₃alkyl), and N(C₁₋₃alkyl)₂;
R^{(V)6} is selected from the group consisting of H, halo, C₁₋₃alkyl, and OC₁₋₃alkyl;
wherein
R^{(V)5} and R^{(V)6} may join to form a 5- or 6-membered carbocyclic ring wherein one or two carbon atoms of the 5- or 6-membered carbocyclic ring may optionally be replaced by one or two oxygen atoms;
R^{(V)7} is selected from the group consisting of H, halo, C₁₋₃alkyl and OC₁₋₃alkyl, wherein the C₁₋₃alkyl of the R^{(V)7} group may optionally be substituted with one to three substituents selected from the group consisting of halo;
R^{(V)8} is selected from the group consisting of H and halo;
R^{(V)9} is H or C₁₋₃alkyl; wherein
R^{(V)2} and R^{(V)9} may join to form a bicyclic ring;
R^{(V)10} is H or C₁₋₃alkyl;
or a pharmaceutically acceptable salt, solvate or hydrate thereof.

7. A method of manufacturing a medical device suitable for being inserted into a lumen of an anatomic structure of a subject according to any one of claims 1 to 6, wherein the method comprises contacting a surface of the medical device with a coating composition comprising a TRPC6 inhibitor.

8. A TRPC6 inhibitor for use in the treatment or prevention of a disease associated with neointimal hyperplasia, wherein the neointimal hyperplasia is associated with the migration of smooth muscular cells;
wherein said TRPC6 inhibitor is administered via a medical device, said medical device comprising means for administration of the TRPC6 inhibitor, and wherein the medical device is a medical device as defined in any one of claims 1 to 6; and/or wherein the TRPC6 inhibitor is administered systemically.

9. The TRPC6 inhibitor for use of claim 8, said use comprising inhibiting the migration of smooth muscle cells (SMCs).

10. The TRPC6 inhibitor for use of any one of claims 8 or 9, wherein the disease is stenosis and/or restenosis, preferably stenosis or restenosis of a blood vessel.

11. An in vitro method of inhibiting migration of smooth muscle cells, said method comprising contacting the smooth muscle cells with a TRPC6 inhibitor, and determining the migration of the smooth muscle cells compared to the migration of smooth muscle cells in the absence or before the contacting with the TRPC6 inhibitor.

12. An *in vitro* test kit comprising:
(a) smooth muscle cells (SMCs);
(b) a surface suitable for SMC cultivation coated with a TRPC6 inhibitor.

## Patentansprüche

1. Medizinische Vorrichtung, geeignet zum Einführen in ein Lumen einer anatomischen Struktur eines Subjekts, wobei die medizinische Vorrichtung Mittel zur Verabreichung eines TRPC6-Inhibitors umfasst, wobei das Mittel den TRPC6-Inhibitor umfasst;
wobei:
die medizinische Vorrichtung ausgewählt wird aus der Gruppe bestehend aus einem Stent, einem Ballon, einem Mikrokatheter oder einem bioresorbierbaren Gerüst besteht; und/oder
das Mittel ist eine Beschichtung der medizinischen Vorrichtung.

2. Medizinische Vorrichtung nach einem der vorhergehenden Ansprüche, wobei das Lumen der anatomischen Struktur ein Lumen eines Blutgefäßes, der Speiseröhre, der Luftröhre oder der Harnröhre ist, vorzugsweise ein Blutgefäß, besonders bevorzugt eine Arterie.

3. Medizinische Vorrichtung nach einem der vorhergehenden Ansprüche, wobei das Subjekt ein Säugetier, vorzugsweise ein Mensch, ist.

4. Medizinische Vorrichtung nach einem der vorhergehenden Ansprüche, wobei das Mittel eine Beschichtung der medizinischen Vorrichtung ist und die medizinische Vorrichtung ein Katheter ist.

5. Medizinische Vorrichtung nach einem der vorhergehenden Ansprüche, wobei die Beschichtung ferner ein Polymer umfasst; und/oder wobei die Beschichtung weiterhin ein Lösungsmittel umfasst.

6. Medizinische Vorrichtung nach einem der vorhergehenden Ansprüche,
(a) wobei der TRPC6-Inhibitor die folgende Formel (I) aufweist: 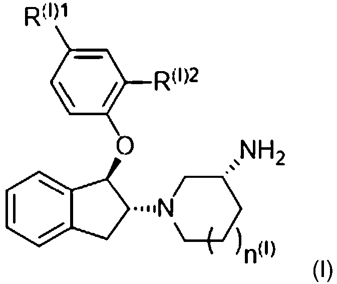 wobei:
R(^{I)1} und R^{(I)2} sind jeweils unabhängig voneinander ausgewählt aus der Gruppe bestehend aus Halogen (vorzugsweise F, Cl oder Br), -CN und -NO₂; und
n^{(I)} ist 0, 1 oder 2;
oder ein pharmazeutisch akzeptables Salz, Solvat oder Hydrat davon; vorzugsweise wobei der TRPC6-Inhibitor 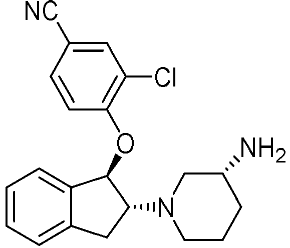 (SAR7334) ist, oder ein pharmazeutisch akzeptables Salz, Solvat oder Hydrat davon;
(b) wobei der TRPC6-Inhibitor die folgende Formel (II) aufweist: 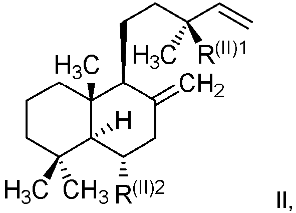 **wobei:**
R^{(II)1} und R^{(II)2} werden jeweils unabhängig voneinander ausgewählt aus der Gruppe bestehend aus -OH, 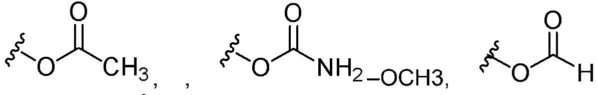 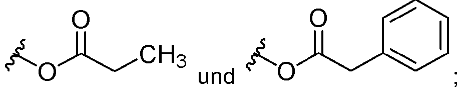
oder ein pharmazeutisch verträgliches Salz, Solvat oder Hydrat davon;
c) wobei der TRPC6-Inhibitor die folgende Formel (III) aufweist: 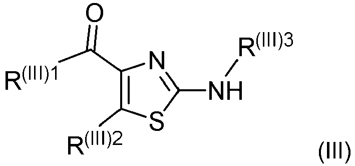 wobei:
R^{(III)1} wird unabhängig ausgewählt aus der Gruppe bestehend aus 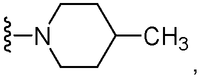 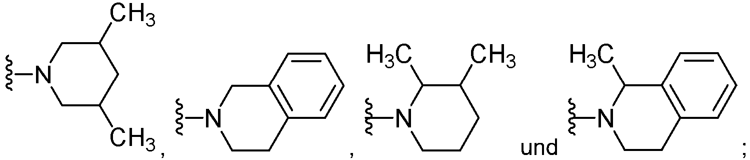
R^{(III)2} wird unabhängig ausgewählt aus der Gruppe bestehend aus -H, -CH₃ und Halogen (vorzugsweise F, Cl, Br) ; und
R^{(III)3} **wird** unabhängig ausgewählt aus der Gruppe bestehend aus 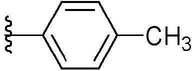 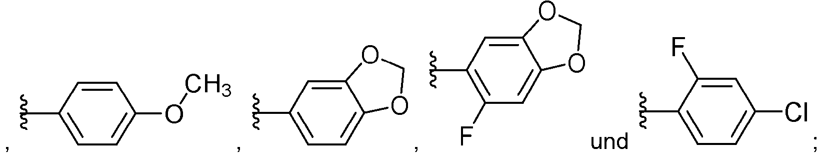
oder ein pharmazeutisch verträgliches Salz, Solvat oder Hydrat davon;
d) wobei der TRPC6-Inhibitor die folgende Formel (IV) aufweist: 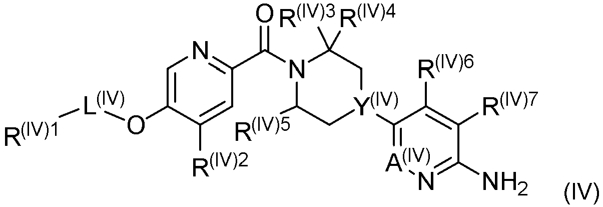 wobei:
L^{(IV)} fehlt oder ist Methylen oder Ethylen;
Y^{(IV)} ist CH oder N;
A^{(IV)} ist CH oder N;
R^{(IV)1} wird ausgewählt aus der Gruppe bestehend aus:
C₁₋₆alkyl, gegebenenfalls substituiert mit 1 bis 3 Gruppen, unabhängig voneinander ausgewählt aus der Gruppe bestehend aus Halo, C₃₋₆cycloalkyl und OC₃₋₆cycloalkyl;
Phenyl, gegebenenfalls substituiert mit 1 bis 3 Gruppen, unabhängig voneinander ausgewählt aus der Gruppe bestehend aus CF₃, Halo, C₃₋₆cycloalkyl, OC₃₋₆cycloalkyl, OC₁₋₆alkyl, gegebenenfalls substituiert mit einem bis drei Halo; und
C₃₋₆cycloalkyl, gegebenenfalls substituiert mit 1 bis 3 Gruppen, unabhängig voneinander ausgewählt aus der Gruppe bestehend aus Halo und C₁₋₆alkyl, gegebenenfalls substituiert mit 1 bis 3 Halo;
R^{(IV)2} wird ausgewählt aus der Gruppe bestehend aus H, C₁₋₆alkyl, OCF₃, C₃₋₆cycloalkyl, OC₁₋₆alkyl; OC₃₋₆cycloalkyl;
R^{(IV)3} wird ausgewählt aus der Gruppe bestehend aus H, C₁₋₆alkyl, C₃₋₆cycloalkyl, OC₃₋₆cycloalkyl; wobei jedes der C₁₋₆alkyl, C₃₋₆cycloalkyl, OC₃₋₆cycloalkyl der R^{(IV)3} Gruppe gegebenenfalls durch eine bis drei Gruppen substituiert werden kann, die jeweils unabhängig voneinander ausgewählt wird aus der Gruppe bestehend aus Halo, OH, OC₁₋₆alkyl, SC₁₋₆alkyl, N(C₁₋₆alkyl)₂; und wobei ein bis drei Kohlenstoffatome des C₁₋₆alkyls der R^{(IV)3} Gruppe gegebenenfalls durch eine oder zwei Gruppen ersetzt werden kann, die ausgewählt sind aus der Gruppe bestehend aus NH, (NC₁₋₆alkyl), O und S;
R^{(IV)4} und R^{(IV)5} sind jeweils unabhängig voneinander ausgewählt aus der Gruppe bestehend aus H- oder C₁₋₆alkyl;
R^{(IV)3} und R^{(IV)4} können sich zusammen mit dem Atom, an das sie gebunden sind, zu einem 3- bis 9-gliedrigen Carbocyclylring verbinden, der gegebenenfalls ein bis drei Heteroatome enthalten kann, ausgewählt aus der Gruppe bestehend aus N, O und S; oder
R^{(IV)3} und R^{(IV)5} können zusammen einen 3- bis 9-gliedrigen bicyclischen Ring bilden, der gegebenenfalls ein bis drei Heteroatome enthalten kann, ausgewählt aus der Gruppe bestehend aus N, O und S;
R^{(IV)6} ist ausgewählt aus der Gruppe bestehend aus H, C₁₋₆alkyl, CN, CF₃, OCF₃, C₃₋₆cycloalkyl, OC₁₋₆alkyl und OC₃₋₆cycloalkyl;
R^{(IV)7} wird ausgewählt aus der Gruppe bestehend aus H und OC₁₋₆alkyl;
oder ein pharmazeutisch verträgliches Salz, Solvat oder Hydrat davon; und/oder
(e) wobei der TRPC6-Inhibitor die folgende Formel (V) aufweist: 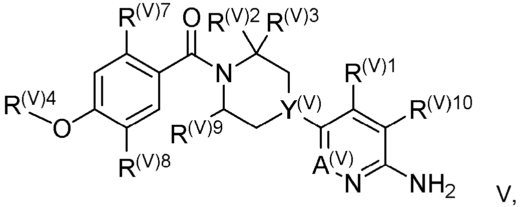 worin:
Y^{(V)} ist CH oder N;
A^{(V)} ist CH oder N;
R^{(V)1} ist H, C₁₋₃alkyl oder OC₁₋₃alkyl;
R^{(V)2} ist H, C₁₋₃alkyl oder C₃₋₆cycloalkyl, wobei jedes der C₁₋₃alkyl oder C₃₋₆cycloalkyl der R²-Gruppe gegebenenfalls mit OH, Halo oder OC₁₋₃alkyl substituiert werden kann;
R^{(V)3} ist H oder C₁₋₃alkyl;
R^{(V)2} und R^{(V)3} können sich zusammen mit dem Kohlenstoff, an den sie gebunden sind, gegebenenfalls zu einem 3- bis 6-gliedrigen carbocyclischen Ring verbinden;
R^{(V)4} steht für
C₁₋₆-Alkyl, das gegebenenfalls mit einer bis drei Gruppen substituiert werden kann, die unabhängig voneinander ausgewählt sind aus der Gruppe bestehend aus Halo ,
C₃₋₆cycloalkylmethyl und C₃₋₆cycloalkylethyl, wobei das C₃₋₆cycloalkyl des C₃₋₆cycloalkyllmethyl und C₃₋₆cycloalkylethyl gegebenenfalls mit einer bis drei Gruppen substituiert werden kann, die unabhängig voneinander ausgewählt sind aus der Gruppe bestehend aus Halo und Methyl,
1,2,3-Thiadiazoylmethyl, Thiazoylmethyl, Isoxazolylmethyl oder
Eine Gruppe der Formel 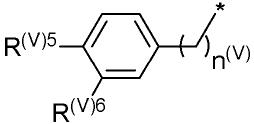
wobei n^{(V)} 0 oder 1 ist;
R^{(V)5} wird ausgewählt aus der Gruppe bestehend aus H, Halo, CF₃, OCF₃, CN, C₁₋₃alkyl, OC₁₋₃alkyl, C₃₋₆cycloalkyl; wobei jede der C₁₋₃alkyl- und OC₁₋₃alkyl-Gruppen der R^{(V)5}-Gruppen gegebenenfalls mit einer bis drei Gruppen substituiert werden kann, die jeweils unabhängig voneinander ausgewählt sind aus der Gruppe bestehend aus Halo, Oxo, NH₂, NH(C₁₋₃alkyl) und N(C₁₋₃alkyl)₂;
R^{(V)6} wird ausgewählt aus der Gruppe bestehend aus H, Halo, C₁₋₃alkyl und OC₁₋₃alkyl;
wobei
R^{(V)5} und R^{(V)6} können sich zu einem 5- oder 6-gliedrigen carbocyclischen Ring verbinden, wobei ein oder zwei Kohlenstoffatome des 5- oder 6-gliedrigen carbocyclischen Rings gegebenenfalls durch ein oder zwei Sauerstoffatome ersetzt werden können;
R^{(V)7} wird ausgewählt aus der Gruppe bestehend aus H, Halo, C₁₋₃alkyl und OC₁₋₃alkyl, wobei das C₁₋₃alkyl der R^{(V)7}-Gruppe gegebenenfalls mit einem bis drei Substituenten substituiert sein kann, ausgewählt aus der Gruppe bestehend aus Halo;
R^{(V)8} wird ausgewählt aus der Gruppe bestehend aus H und Halo;
R^{(V)9} ist H oder C₁₋₃alkyl, wobei
R^{(V)2} und R^{(V)9} können sich zu einem bicyclischen Ring verbinden;
R^{(V)10} ist H oder C₁₋₃alkyl;
oder ein pharmazeutisch verträgliches Salz, Solvat oder Hydrat davon.

7. Verfahren zur Herstellung einer medizinischen Vorrichtung, die geeignet ist, in ein Lumen einer anatomischen Struktur eines Subjekts eingeführt zu werden, nach einem der Ansprüche 1 bis 6, wobei das Verfahren das Kontaktieren einer Oberfläche der medizinischen Vorrichtung mit einer Beschichtungszusammensetzung umfasst, die einen TRPC6-Inhibitor umfasst.

8. TRPC6-Inhibitor zur Verwendung bei der Behandlung oder Vorbeugung einer Krankheit, die mit einer neointimalen Hyperplasie assoziiert ist, wobei die neointimale Hyperplasie mit der Migration glatter Muskelzellen verbunden ist;
wobei der TRPC6-Inhibitor über eine medizinische Vorrichtung verabreicht wird, wobei die medizinische Vorrichtung Mittel zur Verabreichung des TRPC6-Inhibitors umfasst, und wobei die medizinische Vorrichtung eine medizinische Vorrichtung, wie in einem der Ansprüche 1 bis 6 definiert, ist; und/oder
wobei der TRPC6-Inhibitor systemisch verabreicht wird.

9. TRPC6-Inhibitor zur Verwendung nach Anspruch 8, wobei die Verwendung die Hemmung der Migration von glatten Muskelzellen (SMCs) umfasst.

10. TRPC6-Inhibitor zur Verwendung nach einem der Ansprüche 8 oder 9, wobei die Krankheit eine Stenose und/oder Restenose ist, vorzugsweise eine Stenose oder Restenose eines Blutgefäßes.

11. In-vitro-Verfahren zur Hemmung der Migration von glatten Muskelzellen, wobei das Verfahren die Kontaktierung der glatten Muskelzellen mit einem TRPC6-Inhibitor und das Bestimmen der Migration der glatten Muskelzellen im Vergleich zur Migration von glatten Muskelzellen in Abwesenheit oder vor der Kontaktierung mit dem TRPC6-Inhibitor umfasst.

12. Ein *in vitro* Testkit umfassend:
a) glatte Muskelzellen (SMCs);
b) eine für die SMC-Kultivierung geeignete Oberfläche, die mit einem TRPC6-Inhibitor beschichtet ist.

## Revendications

1. Dispositif médical apte à être inséré dans une lumière d'une structure anatomique d'un sujet, ledit dispositif médical comprenant des moyens pour l'administration d'un inhibiteur de TRPC6, dans lequel ledit moyen comprend l'inhibiteur de TRPC6 ;
où:
le dispositif médical est choisi dans le groupe composé d'un stent, d'un ballonnet, d'un microcathéter ou d'un échafaudage bioabsorbable ; et/ou
ce moyen est un revêtement du dispositif médical.

2. Dispositif médical selon l'une quelconque des revendications précédentes, dans lequel la lumière de la structure anatomique est une lumière d'un vaisseau sanguin, de l'œsophage, de la trachée ou de l'urètre, de préférence un vaisseau sanguin, de préférence une artère.

3. Dispositif médical visé par l'une quelconque des revendications précédentes, dans lequel le sujet est un mammifère, de préférence un humain.

4. Dispositif médical selon l'une quelconque des revendications précédentes, dans lequel ledit moyen est un revêtement du dispositif médical, et le dispositif médical est un cathéter.

5. Dispositif médical selon l'une quelconque des revendications précédentes, dans lequel le revêtement comprend en outre un polymère ; et/ou dans lequel le revêtement comprend en outre un solvant.

6. Dispositif médical selon l'une quelconque des revendications précédentes,
a) dans lequel l'inhibiteur de TRPC6 a la formule suivante (I) : 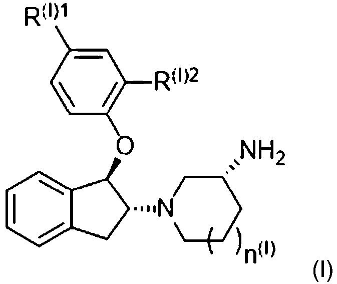 où:
R(^{I)1} et R² sont chacun choisis indépendamment dans le groupe constitué par les halogènes (de préférence F, Cl ou Br), -CN et -NO₂ ; et
n^{(I)} est égal à 0, 1 ou 2 ;
ou un sel, un solvate ou un hydrate pharmaceutiquement acceptable de celui-ci ; de préférence dans lequel l'inhibiteur de TRPC6 est 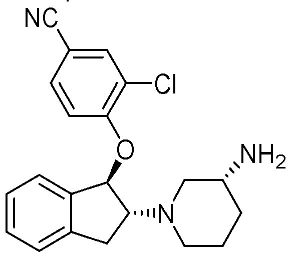 (SAR7334) ou un sel, un solvate ou un hydrate pharmaceutiquement acceptable de celui-ci ;
b) dans lequel l'inhibiteur de TRPC6 a la formule suivante (II) : 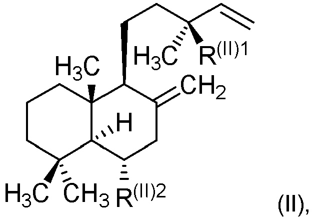 où:
R^{(II)1} et R^{(II)2} sont chacun choisis indépendamment dans le groupe constitué de -OH, 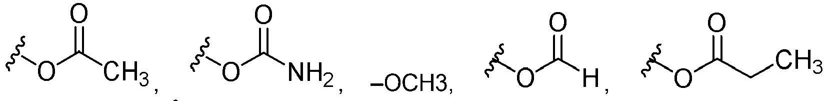 et 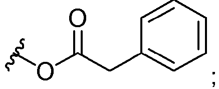
ou un sel, un solvate ou un hydrate pharmaceutiquement acceptable de ceux-ci ;
c) dans lequel l'inhibiteur de TRPC6 a la formule suivante (III) : 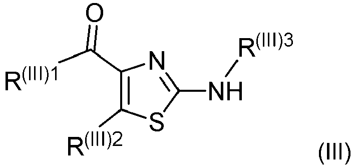 où:
R^{(III)1} est choisi indépendamment dans le groupe composé de 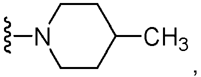 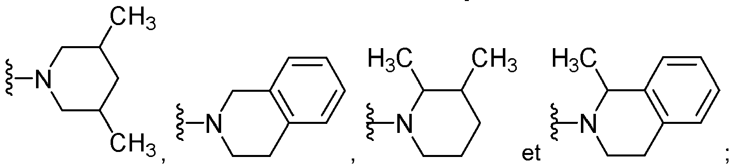
R^{(III)2} est choisi indépendamment dans le groupe constitué de -H, -CH₃ et d'halogènes (de préférence F, Cl, Br) ; et
R^{(III)3} est choisi indépendamment dans le groupe composé de 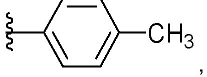 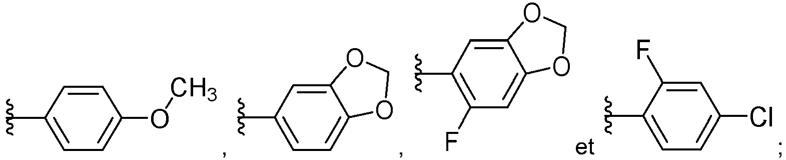
ou un sel, un solvate ou un hydrate pharmaceutiquement acceptable de ceux-ci ;
d) dans lequel l'inhibiteur de TRPC6 a la formule suivante (IV) : 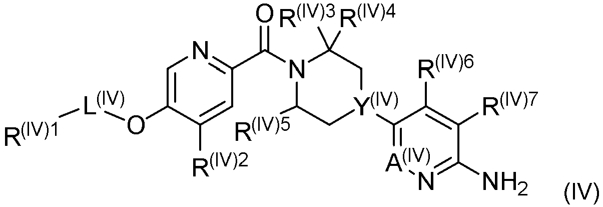 où:
L^{(IV)} est absent ou est du méthylène ou de l'éthylène ;
Y^{(IV)} est CH ou N ;
A^{(IV)} est CH ou N ;
R^{(IV)1} est choisi dans le groupe composé de :
Alkyle en C₁₋₆ éventuellement substitué par 1 à 3 groupes choisis indépendamment dans le groupe constitué du halo, du cycloalkyle en C₃₋₆ et de l'OC₃₋₆cycloalkyle ;
Phényle éventuellement substitué par 1 à 3 groupes choisis indépendamment dans le groupe constitué de CF₃, halo, C₃₋₆cycloalkyle, OC₃₋₆cycloalkyle, OC₁₋₆alkyle éventuellement substitué par un à trois halo ; et
C₃₋₆cycloalkyle éventuellement substitué par 1 à 3 groupes choisis indépendamment dans le groupe constitué par le halo et C₁₋₆alkyle éventuellement substitué par 1 à 3 halo ;
R^{(IV)2} est choisi dans le groupe constitué de H, C₁₋₆alkyle, OCF₃, C₃₋₆cycloalkyle, OC₁₋₆alkyle ; OC₃₋₆cycloalkyle ;
R^{(IV)3} est choisi dans le groupe constitué par H, C₁₋₆alkyle, C₃₋₆cycloalkyle, OC₃₋₆cycloalkyle; où chacun des alkyles en C₁₋₆, en C₃₋₆cycloalkyle et en OC₃₋₆cycloalkyle du R^{(IV)3} groupe peut être éventuellement substitué par un à trois groupes, chacun sélectionné indépendamment dans le groupe composé de halo, OH, OC₁₋₆alkyle, SC₁₋₆alkyl , N(C₁₋₆alkyle)₂; et dans lequel un à trois atomes de carbone de l'alkyle C₁₋₆ de l'alkyle R^{(IV)3} le groupe peut éventuellement être remplacé par une ou deux fractions choisies dans le groupe constitué de NH, (NC₁₋₆alkyle), O et S ;
R^{(IV)4} et R^{(IV)5} sont chacun choisis indépendamment dans le groupe constitué d'alkyle H ou C₁₋₆;
R^{(IV)3} et R^{(IV)4} peuvent, avec l'atome auquel ils sont attachés, se joindre pour former un cycle carbocyclyle à 3 à 9 chaînons qui, éventuellement, peut contenir un à trois hétéroatomes choisis dans le groupe constitué de N, O et S ; ou
R^{(IV)3} et R^{(IV)5} peuvent former ensemble un cycle bicyclique de 3 à 9 chaînons qui, éventuellement, peut contenir un à trois hétéroatomes choisis dans le groupe constitué de N, O et S ;
R^{(IV)6} est choisi dans le groupe composé de H, C₁₋₆alkyle, CN, CF₃, OCF₃, C₃₋₆cycloalkyle, OC₁₋₆alkyle et OC₃₋₆cycloalkyle ;
R^{(IV)7} est choisi dans le groupe constitué de H et d'alkyle OC₁₋₆ ;
ou un sel, un solvate ou un hydrate pharmaceutiquement acceptable de ceux-ci ; et/ou
e) dans lequel l'inhibiteur de TRPC6 a la formule suivante (V) : 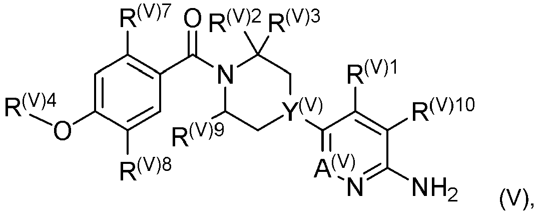 où:
Y^{V)} est CH ou N ;
A^{(V)} est CH ou N ;
R^{(V)1} est un H, C₁₋₃alkyle ou OC₁₋₃alkyle ;
R^{(V)2} est un H, un alkyle en C₁₋₃ ou un cycloalkyle en C₃₋₆, chacun des alkyles en C₁₋₃ ou en C₃₋₆cycloalkyle du groupe R² pouvant être substitué de manière facultative par OH, halo ou alkyle OC₁₋₃ ;
R^{(V)3} est un H ou C₁-₃alkyle;
R^{(V)2} et R^{(V)3} ainsi que le carbone auquel ils sont fixés peuvent éventuellement se joindre pour former un cycle carbocyclique de 3 à 6 chaînons ;
R^{(V)4} représente
alkyle en C₁₋₆ qui peut éventuellement être substitué par un à trois groupes choisis indépendamment dans le groupe constitué par le halo,
C₃₋₆cycloalkylméthyle et C₃₋₆cycloalkyléthyle, où le C₃₋₆cycloalkyle du C₃₋₆cycloalkylméthyle et du C₃₋₆cycloalkyléthyle peut éventuellement être substitué par un à trois groupes choisis indépendamment dans le groupe constitué par halo et méthyle,
1,2,3-thiadiazoylméthyle, thiazoylméthyle, isoxazolylméthyle ou
un groupe de formules 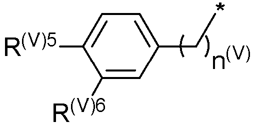
où n^{(V)} est égal à 0 ou 1 ;
R^{(V)5} est choisi dans le groupe constitué par H, halo, CF₃, OCF₃, CN, C₁₋₃alkyle, OC₁₋₃alkyle, C₃₋₆cycloalkyle ; dans lequel chacun des alkyle C₁₋₃ et OC₁₋₃alkyle des groupes R^{(V)5} peut être substitué de manière facultative par un à trois groupes choisis indépendamment chacun dans le groupe constitué de halo, oxo, NH₂, NH(C₁₋₃alkyle) et N(C₁₋₃alkyle)₂ ;
R^{(V)6} est choisi dans le groupe composé de H, halo, alkyle en C₁₋₃ et alkyle OC₁₋₃ ;
Où
R^{(V)5} et R^{(V)6} peuvent s'unir pour former un cycle carbocyclique à 5 ou 6 chaînons, dans lequel un ou deux atomes de carbone du cycle carbocyclique à 5 ou 6 chaînons peuvent être remplacés de manière facultative par un ou deux atomes d'oxygène ;
R^{(V)7} est choisi dans le groupe constitué de H, halo, alkyle en C₁₋₃ et alkyle OC₁₋₃, l'alkyle en C₁₋₃ du groupe R^{(V)7} pouvant être éventuellement substitué par un à trois substituants choisis dans le groupe constitué par le halo ;
R^{(V)8} est choisi dans le groupe constitué de H et de halo ;
R^{(V)9} est un H ou alkyle en C₁₋₃ ; dans lequel
R^{(V)2} et R^{V)9} peuvent se joindre pour former un anneau bicyclique ;
R^{(V)10} est un H ou C₁₋₃alkyle;
ou un sel, un solvate ou un hydrate pharmaceutiquement acceptable de ceux-ci.

7. Procédé de fabrication d'un dispositif médical apte à être inséré dans une lumière d'une structure anatomique d'un sujet selon l'une quelconque des revendications 1 à 6, dans lequel le procédé comprend la mise en contact d'une surface du dispositif médical avec une composition de revêtement comprenant un inhibiteur de TRPC6.

8. Inhibiteur de TRPC6 destiné au traitement ou à la prévention d'une maladie associée à l'hyperplasie néointimale, dans laquelle l'hyperplasie néointimale est associée à la migration de cellules musculaires lisses ;
dans lequel ledit inhibiteur de TRPC6 est administré par l'intermédiaire d'un dispositif médical, ledit dispositif médical comprenant des moyens d'administration de l'inhibiteur de TRPC6, et dans lequel le dispositif médical est un dispositif médical tel que défini dans l'une quelconque des revendications 1 à 6 ; et/ou
dans lequel l'inhibiteur de TRPC6 est administré par voie systémique.

9. Inhibiteur de TRPC6 selon la revendication 8, ladite utilisation comprenant l'inhibition de la migration des cellules musculaires lisses (CML).

10. Inhibiteur de TRPC6 pour l'utilisation de l'une quelconque des revendications 8 ou 9, dans lequel la maladie est une sténose et/ou une resténose, de préférence une sténose ou une resténose d'un vaisseau sanguin.

11. Procédé in vitro d'inhibition de la migration de cellules musculaires lisses, ledit procédé comprenant la mise en contact des cellules musculaires lisses avec un inhibiteur de TRPC6, et la détermination de la migration des cellules musculaires lisses par rapport à la migration de cellules musculaires lisses en l'absence ou avant le contact avec l'inhibiteur de TRPC6.

12. Un *in vitro* Kit d'essai comprenant :
a) les cellules musculaires lisses (CML) ;
b) une surface adaptée à la culture de SMC recouverte d'un inhibiteur de TRPC6.
